(19) Europäisches Patentamt
European Patent Office
Office européen des brevets

(11) EP 1 230 214 B1

(12) EUROPEAN PATENT SPECIFICATION

(45) Date of publication and mention of the grant of the patent:
**17.12.2008 Bulletin 2008/51**

(21) Application number: **00984944.9**

(22) Date of filing: **30.10.2000**

(51) Int Cl.:
***C07D 201/00*** *(2006.01)*

(86) International application number:
**PCT/EP2000/010670**

(87) International publication number:
**WO 2001/032614 (10.05.2001 Gazette 2001/19)**

(54) **NOVEL GENES TZAP7/A, TZAP7/B AND TZAP7 INVOLVED IN T CELL ACTIVATION AND USES THEREOF**

NEUE GENE TZAP7/A, TZAP7/B AND TZAP7 WELCHE IN DIE T ZELLEN AKTIVIERUNG INVOLVIERT SIND, UND DEREN VERWENDUNGEN

NOUVEAUX GENES TZAP7/A, TZAP7/B ET TZAP7 JOUANT UN ROLE DANS L'ACTIVATION DES LYMPHOCYTES T ET LEURS APPLICATIONS

(84) Designated Contracting States:
**AT BE CH CY DE DK ES FI FR GB GR IE IT LI LU MC NL PT SE**

(30) Priority: **01.11.1999 US 162675 P**
**25.02.2000 US 185016 P**

(43) Date of publication of application:
**14.08.2002 Bulletin 2002/33**

(73) Proprietors:
- **Utku, Nalan**
**10719 Berlin (DE)**
- **THE BRIGHAM AND WOMEN'S HOSPITAL, INC.**
**Boston, MA 02115 (US)**

(72) Inventors:
- **Utku Nalan**
**10719 Berlin (DE)**
- **Milford Edgar L**
**Dover , MA 02030 (US)**

(74) Representative: **Von Renesse, Dorothea et al**
**König Szynka Tilmann von Renesse**
**Patentanwälte Partnerschaft**
**Lohengrinstrasse 11**
**40549 Düsseldorf (DE)**

(56) References cited:
**WO-A-01/05970** **WO-A-99/35170**

- **DATABASE EMBL [Online] EMBL:AF069737, 2 October 1998 (1998-10-02) ROYET J.: "Notchless encodes a novel WD40-repeat protein that modulates Notch signalling activity" XP002167244**
- **DATABASE EMBL [Online] EMBL:HSAJ5257, 7 April 1998 (1998-04-07) STANCHI F.: "Homo sapiens partial mRNA for beta-transducin family protein" XP002167245**
- **ROYET ET AL: "Notchless encodes a novel WD40-repeat-containing protein that modulates Notch signalling activity" EMBO JOURNAL,GB, OXFORD UNIVERSITY PRESS, SURREY, vol. 17, no. 24, 15 December 1998 (1998-12-15), pages 7351-7360, XP002133042 ISSN: 0261-4189**
- **HEINEMANN THOMAS ET AL: "Genomic organization of the gene coding for TIRC7, a novel membrane protein essential for T cell activation." GENOMICS, vol. 57, no. 3, 1 May 1999 (1999-05-01), pages 398-406, XP000999606 ISSN: 0888-7543**
- **ROBEY E: "NOTCH IN VERTBRATES" CURRENT OPINION IN GENETICS & DEVELOPMENT,XX, CURRENT BIOLOGY LTD, vol. 7, no. 4, 1 August 1997 (1997-08-01), pages 551-557, XP002056593 ISSN: 0959-437X**
- **MARGOTTIN F: "H. sapiens beta transducin (b-TrCP)" GENBANK, XP002141481**
- **DATABASE EMBL [Online] EMBL:AK001320, 22 February 2000 (2000-02-22) ISOGAI T.: "Homo sapiens cDNA FLJ10458 fis,..." XP002167293**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 230 214 B1

SEQUENCE LISTING

<110> Utku, Nalan

<120> Novel genes TZap7/A, TZap7/B and TZap7 involved in T cell activation and uses thereof

<130> D 2397 US

<140>
<141>

<160> 6

<170> PatentIn Ver. 2.1

<210> 1
<211> 1928
<212> DNA
<213> Homo sapiens

<220>
<221> CDS
<222> (92)..(1543)

<400> 1

```
ggcacgagct cttccgcaca tgtgtctgct gtaaacacat gtctgtagtt ttttaagctc 60

ctgtctgaag taaggcgacc cttctgaata a atg gtg tgg atc gcg gac gag    112
                                   Met Val Trp Ile Ala Asp Glu
                                   1               5

gcg gtg gcg cgc gat gtg cag cgg ttg cta gtg cag ttc cag gat gag   160
Ala Val Ala Arg Asp Val Gln Arg Leu Leu Val Gln Phe Gln Asp Glu
        10                  15                  20

ggc ggg cag ctg ctg ggt tcc ccg ttc gac gtg ccc gtg gac atc acc   208
Gly Gly Gln Leu Leu Gly Ser Pro Phe Asp Val Pro Val Asp Ile Thr
    25                  30                  35

ccg gac agg ctg cag ctc gtt tgc aac gcg cta ctg gcc cag gag gat   256
Pro Asp Arg Leu Gln Leu Val Cys Asn Ala Leu Leu Ala Gln Glu Asp
40                  45                  50                  55

ccc ctg tca ctg gct ttc ttt gtc cac gat gct gag atc gtt tcc tca   304
Pro Leu Ser Leu Ala Phe Phe Val His Asp Ala Glu Ile Val Ser Ser
                60                  65                  70

ctc ggg aag acg ttg gag tcc cag gca gtg gag aca gag aag gtc cta   352
Leu Gly Lys Thr Leu Glu Ser Gln Ala Val Glu Thr Glu Lys Val Leu
            75                  80                  85

gac atc att tta ccc aca caa gct gtg ttc aaa gtt cgt gct gta aca   400
Asp Ile Ile Leu Pro Thr Gln Ala Val Phe Lys Val Arg Ala Val Thr
        90                  95                  100

cga tgt acc agc tca ttg gag gga cac acc gag gct gtt att tca gta   448
Arg Cys Thr Ser Ser Leu Glu Gly His Thr Glu Ala Val Ile Ser Val
    105                 110                 115
```

```
gcc ttc agc cca act gga aag tat ttg gca agt ggt tct ggg gac act     496
Ala Phe Ser Pro Thr Gly Lys Tyr Leu Ala Ser Gly Ser Gly Asp Thr
120                 125                 130                 135

aca gtc cgc ttt tgg gat ctc agc aca gaa act cca cat ttt aca tct     544
Thr Val Arg Phe Trp Asp Leu Ser Thr Glu Thr Pro His Phe Thr Ser
                140                 145                 150

aaa ggg cat aca cac tgg gtt ctc agt att gct tgg tct cca gat ggc     592
Lys Gly His Thr His Trp Val Leu Ser Ile Ala Trp Ser Pro Asp Gly
            155                 160                 165

aaa aaa ctt gcc tca gga tgt aaa aat agt cag atc ttc att tgg gac     640
Lys Lys Leu Ala Ser Gly Cys Lys Asn Ser Gln Ile Phe Ile Trp Asp
        170                 175                 180

cca agc aca ggg aag cag att ggc aaa cca tta aca ggg cac tca aag     688
Pro Ser Thr Gly Lys Gln Ile Gly Lys Pro Leu Thr Gly His Ser Lys
    185                 190                 195

tgg att aca tgg ctg tgt tgg gaa cct ctc cac ctg aac cca gag agc     736
Trp Ile Thr Trp Leu Cys Trp Glu Pro Leu His Leu Asn Pro Glu Ser
200                 205                 210                 215

cga tac cta gcc agt gcc tcc agc ggc cgc gtc gac cgg atc tgg gac     784
Arg Tyr Leu Ala Ser Ala Ser Ser Gly Arg Val Asp Arg Ile Trp Asp
                220                 225                 230

aca act gca ggc cgc tgt gag cgc atc ctc acc ggg cac acc cag tcg     832
Thr Thr Ala Gly Arg Cys Glu Arg Ile Leu Thr Gly His Thr Gln Ser
            235                 240                 245

gtc acc tgt ctc cgg tgg gga ggg gac ggg ctt ctc tac tct gcc tcc     880
Val Thr Cys Leu Arg Trp Gly Gly Asp Gly Leu Leu Tyr Ser Ala Ser
        250                 255                 260

cag gac cgc acc atc aaa gtc tgg aga gct cat gac ggt gtg ctg tgc     928
Gln Asp Arg Thr Ile Lys Val Trp Arg Ala His Asp Gly Val Leu Cys
    265                 270                 275

cgg act ctg caa ggc cac ggc cac tgg gtg aac acc atg gcc ctc agc     976
Arg Thr Leu Gln Gly His Gly His Trp Val Asn Thr Met Ala Leu Ser
280                 285                 290                 295

act gac tat gcc ctg cgc act ggg gcc ttt gaa cct gct gag gcc tca    1024
Thr Asp Tyr Ala Leu Arg Thr Gly Ala Phe Glu Pro Ala Glu Ala Ser
                300                 305                 310

gtt aat ccc caa gac ctc caa gga tcc ttg cag gag ttg aag gag agg    1072
Val Asn Pro Gln Asp Leu Gln Gly Ser Leu Gln Glu Leu Lys Glu Arg
            315                 320                 325

gct ctg agc cga tac aac ctc gtg cgg ggc cag ggt cca gag agg ctg    1120
Ala Leu Ser Arg Tyr Asn Leu Val Arg Gly Gln Gly Pro Glu Arg Leu
        330                 335                 340

gtg tct ggc tcc gac gac ttc acc tta ttc ctg tgg tcc cca gca gag    1168
Val Ser Gly Ser Asp Asp Phe Thr Leu Phe Leu Trp Ser Pro Ala Glu
    345                 350                 355
```

```
gac aaa aag cct ctc act cgg atg aca gga cac caa gct ctc atc aac    1216
Asp Lys Lys Pro Leu Thr Arg Met Thr Gly His Gln Ala Leu Ile Asn
360                 365                 370                 375

cag gtg ctc ttc tct cct gac tcc cgc atc gtg gct agt gcc tcc ttt    1264
Gln Val Leu Phe Ser Pro Asp Ser Arg Ile Val Ala Ser Ala Ser Phe
                380                 385                 390

gac aag tcc atc aag ctg tgg gat ggc agg acg ggc aag tac ctg gct    1312
Asp Lys Ser Ile Lys Leu Trp Asp Gly Arg Thr Gly Lys Tyr Leu Ala
            395                 400                 405

tcc cta cgc ggc cac gtg gct gcc gtg tac cag att gcg tgg tca gct    1360
Ser Leu Arg Gly His Val Ala Ala Val Tyr Gln Ile Ala Trp Ser Ala
        410                 415                 420

gac agt cgg ctc ctg gtc agc ggc agc agt gac agc aca ctg aag gtg    1408
Asp Ser Arg Leu Leu Val Ser Gly Ser Ser Asp Ser Thr Leu Lys Val
    425                 430                 435

tgg gat gtg aag gcc cag aag ctg gcc atg gac ctg ccc ggc cac gcg    1456
Trp Asp Val Lys Ala Gln Lys Leu Ala Met Asp Leu Pro Gly His Ala
440                 445                 450                 455

gat gag gta tat gct gtt gac tgg agt cca gat ggc cag aga gtg gca    1504
Asp Glu Val Tyr Ala Val Asp Trp Ser Pro Asp Gly Gln Arg Val Ala
                460                 465                 470

agt ggt ggg aag gac aaa tgc ctc cgg ata tgg agg aga tgagacggcc     1553
Ser Gly Gly Lys Asp Lys Cys Leu Arg Ile Trp Arg Arg
            475                 480

cgaagttctc tctgaccccc acctcgactc ggcctctgcc agctgccttc cctgccagag 1613

aacaaaggct gagatggcag tgcacacacc ctccccacca gtggggacct gagaatgcgt 1673

gtggcctgct gtcctcgata gaccggaatg gggttttccc acagatcccc gcctgtggca 1733

caccccagag ccagaaatcg aaggtcacag gaagttgtca ctgaacttgg cccgtgtctg 1793

ctactctgta ccttgctggt acagacaggg gtggtgggca gccaggctct atgagtgggc 1853

ccctagtgtc agctctgtac agggtcagat cccaggttct atgaccaaat aagtaactta 1913

aaaaaaaaaa aaaaa                                                  1928
```

<210> 2
<211> 484
<212> PRT
<213> Homo sapiens

<400> 2

```
Met Val Trp Ile Ala Asp Glu Ala Val Ala Arg Asp Val Gln Arg Leu
 1               5                  10                  15

Leu Val Gln Phe Gln Asp Glu Gly Gly Gln Leu Leu Gly Ser Pro Phe
            20                  25                  30

Asp Val Pro Val Asp Ile Thr Pro Asp Arg Leu Gln Leu Val Cys Asn
        35                  40                  45
```

Ala Leu Leu Ala Gln Glu Asp Pro Leu Ser Leu Ala Phe Phe Val His
50 55 60

Asp Ala Glu Ile Val Ser Ser Leu Gly Lys Thr Leu Glu Ser Gln Ala
65 70 75 80

Val Glu Thr Glu Lys Val Leu Asp Ile Ile Leu Pro Thr Gln Ala Val
85 90 95

Phe Lys Val Arg Ala Val Thr Arg Cys Thr Ser Ser Leu Glu Gly His
100 105 110

Thr Glu Ala Val Ile Ser Val Ala Phe Ser Pro Thr Gly Lys Tyr Leu
115 120 125

Ala Ser Gly Ser Gly Asp Thr Thr Val Arg Phe Trp Asp Leu Ser Thr
130 135 140

Glu Thr Pro His Phe Thr Ser Lys Gly His Thr His Trp Val Leu Ser
145 150 155 160

Ile Ala Trp Ser Pro Asp Gly Lys Lys Leu Ala Ser Gly Cys Lys Asn
165 170 175

Ser Gln Ile Phe Ile Trp Asp Pro Ser Thr Gly Lys Gln Ile Gly Lys
180 185 190

Pro Leu Thr Gly His Ser Lys Trp Ile Thr Trp Leu Cys Trp Glu Pro
195 200 205

Leu His Leu Asn Pro Glu Ser Arg Tyr Leu Ala Ser Ala Ser Ser Gly
210 215 220

Arg Val Asp Arg Ile Trp Asp Thr Thr Ala Gly Arg Cys Glu Arg Ile
225 230 235 240

Leu Thr Gly His Thr Gln Ser Val Thr Cys Leu Arg Trp Gly Gly Asp
245 250 255

Gly Leu Leu Tyr Ser Ala Ser Gln Asp Arg Thr Ile Lys Val Trp Arg
260 265 270

Ala His Asp Gly Val Leu Cys Arg Thr Leu Gln Gly His Gly His Trp
275 280 285

Val Asn Thr Met Ala Leu Ser Thr Asp Tyr Ala Leu Arg Thr Gly Ala
290 295 300

Phe Glu Pro Ala Glu Ala Ser Val Asn Pro Gln Asp Leu Gln Gly Ser
305 310 315 320

Leu Gln Glu Leu Lys Glu Arg Ala Leu Ser Arg Tyr Asn Leu Val Arg
325 330 335

Gly Gln Gly Pro Glu Arg Leu Val Ser Gly Ser Asp Asp Phe Thr Leu
340 345 350

Phe Leu Trp Ser Pro Ala Glu Asp Lys Lys Pro Leu Thr Arg Met Thr
355 360 365

Gly His Gln Ala Leu Ile Asn Gln Val Leu Phe Ser Pro Asp Ser Arg
370 375 380

```
Ile Val Ala Ser Ala Ser Phe Asp Lys Ser Ile Lys Leu Trp Asp Gly
385                 390                 395                 400

Arg Thr Gly Lys Tyr Leu Ala Ser Leu Arg Gly His Val Ala Ala Val
                405                 410                 415

Tyr Gln Ile Ala Trp Ser Ala Asp Ser Arg Leu Leu Val Ser Gly Ser
            420                 425                 430

Ser Asp Ser Thr Leu Lys Val Trp Asp Val Lys Ala Gln Lys Leu Ala
        435                 440                 445

Met Asp Leu Pro Gly His Ala Asp Glu Val Tyr Ala Val Asp Trp Ser
    450                 455                 460

Pro Asp Gly Gln Arg Val Ala Ser Gly Gly Lys Asp Lys Cys Leu Arg
465                 470                 475                 480

Ile Trp Arg Arg
```

<210> 3
<211> 1170
<212> DNA
<213> Homo sapiens

<220>
<221> CDS
<222> (3)..(785)

<400> 3

```
gc ggc cgc gtc gac cgg atc tgg gac aca act gca ggc cgc tgt gag     47
   Gly Arg Val Asp Arg Ile Trp Asp Thr Thr Ala Gly Arg Cys Glu
     1               5                  10                  15

cgc atc ctc acc ggg cac acc cag tcg gtc acc tgt ctc cgg tgg gga    95
Arg Ile Leu Thr Gly His Thr Gln Ser Val Thr Cys Leu Arg Trp Gly
                20                  25                  30

ggg gac ggg ctt ctc tac tct gcc tcc cag gac cgc acc atc aaa gtc   143
Gly Asp Gly Leu Leu Tyr Ser Ala Ser Gln Asp Arg Thr Ile Lys Val
            35                  40                  45

tgg aga gct cat gac ggt gtg ctg tgc cgg act ctg caa ggc cac ggc   191
Trp Arg Ala His Asp Gly Val Leu Cys Arg Thr Leu Gln Gly His Gly
        50                  55                  60

cac tgg gtg aac acc atg gcc ctc agc act gac tat gcc ctg cgc act   239
His Trp Val Asn Thr Met Ala Leu Ser Thr Asp Tyr Ala Leu Arg Thr
     65                  70                  75

ggg gcc ttt gaa cct gct gag gcc tca gtt aat ccc caa gac ctc caa   287
Gly Ala Phe Glu Pro Ala Glu Ala Ser Val Asn Pro Gln Asp Leu Gln
 80                  85                  90                  95

gga tcc ttg cag gag ttg aag gag agg gct ctg agc cga tac aac ctc   335
Gly Ser Leu Gln Glu Leu Lys Glu Arg Ala Leu Ser Arg Tyr Asn Leu
                100                 105                 110
```

```
gtg cgg ggc cag ggt cca gag agg ctg gtg tct ggc tcc gac gac ttc    383
Val Arg Gly Gln Gly Pro Glu Arg Leu Val Ser Gly Ser Asp Asp Phe
            115                 120                 125

acc tta ttc ctg tgg tcc cca gca gag gac aaa aag cct ctc act cgg    431
Thr Leu Phe Leu Trp Ser Pro Ala Glu Asp Lys Lys Pro Leu Thr Arg
        130                 135                 140

atg aca gga cac caa gct ctc atc aac cag gtg ctc ttc tct cct gac    479
Met Thr Gly His Gln Ala Leu Ile Asn Gln Val Leu Phe Ser Pro Asp
    145                 150                 155

tcc cgc atc gtg gct agt gcc tcc ttt gac aag tcc atc aag ctg tgg    527
Ser Arg Ile Val Ala Ser Ala Ser Phe Asp Lys Ser Ile Lys Leu Trp
160                 165                 170                 175

gat ggc agg acg ggc aag tac ctg gct tcc cta cgc ggc cac gtg gct    575
Asp Gly Arg Thr Gly Lys Tyr Leu Ala Ser Leu Arg Gly His Val Ala
                180                 185                 190

gcc gtg tac cag att gcg tgg tca gct gac agt cgg ctc ctg gtc agc    623
Ala Val Tyr Gln Ile Ala Trp Ser Ala Asp Ser Arg Leu Leu Val Ser
            195                 200                 205

ggc agc agt gac agc aca ctg aag gtg tgg gat gtg aag gcc cag aag    671
Gly Ser Ser Asp Ser Thr Leu Lys Val Trp Asp Val Lys Ala Gln Lys
        210                 215                 220

ctg gcc atg gac ctg ccc ggc cac gcg gat gag gta tat gct gtt gac    719
Leu Ala Met Asp Leu Pro Gly His Ala Asp Glu Val Tyr Ala Val Asp
    225                 230                 235

tgg agt cca gat ggc cag aga gtg gca agt ggt ggg aag gac aaa tgc    767
Trp Ser Pro Asp Gly Gln Arg Val Ala Ser Gly Gly Lys Asp Lys Cys
240                 245                 250                 255

ctc cgg ata tgg agg aga tgagacggcc cgaagttctc tctgaccccc           815
Leu Arg Ile Trp Arg Arg
                260

acctcgactc ggcctctgcc agctgccttc cctgccagag aacaaaggct gagatggcag 875

tgcacacacc ctccccacca gtggggacct gagaatgcgt gtggcctgct gtcctcgata 935

gaccggaatg ggttttccc acagatcccc gcctgtggca caccccagag ccagaaatcg 995

aaggtcacag gaagttgtca ctgaacttgg cccgtgtctg ctactctgta ccttgctggt 1055

acagacaggg gtggtgggca gccaggctct atgagtgggc cctagtgtc agctctgtac 1115

agggtcagat cccaggttct atgaccaaat aagtaactta aaaaaaaaa aaaaa      1170
```

<210> 4
<211> 261
<212> PRT
<213> Homo sapiens

<400> 4

```
Gly Arg Val Asp Arg Ile Trp Asp Thr Thr Ala Gly Arg Cys Glu Arg
1               5                   10                  15

Ile Leu Thr Gly His Thr Gln Ser Val Thr Cys Leu Arg Trp Gly Gly
            20                  25                  30

Asp Gly Leu Leu Tyr Ser Ala Ser Gln Asp Arg Thr Ile Lys Val Trp
        35                  40                  45

Arg Ala His Asp Gly Val Leu Cys Arg Thr Leu Gln Gly His Gly His
    50                  55                  60

Trp Val Asn Thr Met Ala Leu Ser Thr Asp Tyr Ala Leu Arg Thr Gly
65                  70                  75                  80

Ala Phe Glu Pro Ala Glu Ala Ser Val Asn Pro Gln Asp Leu Gln Gly
                85                  90                  95

Ser Leu Gln Glu Leu Lys Glu Arg Ala Leu Ser Arg Tyr Asn Leu Val
            100                 105                 110

Arg Gly Gln Gly Pro Glu Arg Leu Val Ser Gly Ser Asp Asp Phe Thr
        115                 120                 125

Leu Phe Leu Trp Ser Pro Ala Glu Asp Lys Lys Pro Leu Thr Arg Met
    130                 135                 140

Thr Gly His Gln Ala Leu Ile Asn Gln Val Leu Phe Ser Pro Asp Ser
145                 150                 155                 160

Arg Ile Val Ala Ser Ala Ser Phe Asp Lys Ser Ile Lys Leu Trp Asp
                165                 170                 175

Gly Arg Thr Gly Lys Tyr Leu Ala Ser Leu Arg Gly His Val Ala Ala
            180                 185                 190

Val Tyr Gln Ile Ala Trp Ser Ala Asp Ser Arg Leu Leu Val Ser Gly
        195                 200                 205

Ser Ser Asp Ser Thr Leu Lys Val Trp Asp Val Lys Ala Gln Lys Leu
    210                 215                 220

Ala Met Asp Leu Pro Gly His Ala Asp Glu Val Tyr Ala Val Asp Trp
225                 230                 235                 240

Ser Pro Asp Gly Gln Arg Val Ala Ser Gly Gly Lys Asp Lys Cys Leu
                245                 250                 255

Arg Ile Trp Arg Arg
            260
```

<210> 5
<211> 1859
<212> DNA
<213> Homo sapiens

<220>

<221> CDS
<222> (20)..(1474)

<400> 5

```
cgcccgggca ggtcgcagg atg gcg gca gca gtg gcg gac gag gcg gtg gcg  52
                     Met Ala Ala Ala Val Ala Asp Glu Ala Val Ala
                     1               5                   10
```

```
cgc gat gtg cag cgg ttg cta gtg cag ttc cag gat gag ggc ggg cag     100
Arg Asp Val Gln Arg Leu Leu Val Gln Phe Gln Asp Glu Gly Gly Gln
                15                  20                  25

ctg ctg ggt tcc ccg ttc gac gtg ccc gtg gac atc acc ccg gac agg     148
Leu Leu Gly Ser Pro Phe Asp Val Pro Val Asp Ile Thr Pro Asp Arg
            30                  35                  40

ctg cag ctc gtg tgc aac gcg cta ctg gcc cag gag gat ccc ctg cca     196
Leu Gln Leu Val Cys Asn Ala Leu Leu Ala Gln Glu Asp Pro Leu Pro
        45                  50                  55

ctg gct ttc ttt gtc cac gat gct gag atc gtc tcc tca ctg ggg aag     244
Leu Ala Phe Phe Val His Asp Ala Glu Ile Val Ser Ser Leu Gly Lys
    60                  65                  70                  75

acg ttg gag tcc cag gca gtg gag aca gag aag gtc cta gac atc atc     292
Thr Leu Glu Ser Gln Ala Val Glu Thr Glu Lys Val Leu Asp Ile Ile
                80                  85                  90

tac cag cca cag gct atc ttc aga gtc cgg gct gtg act cgc tgc acc     340
Tyr Gln Pro Gln Ala Ile Phe Arg Val Arg Ala Val Thr Arg Cys Thr
                95                  100                 105

agc tcc ttg gag ggt cac agt gag gca gtc att tct gtg gcc ttc agc     388
Ser Ser Leu Glu Gly His Ser Glu Ala Val Ile Ser Val Ala Phe Ser
            110                 115                 120

cct acg gga aag tac ctg gcc agt ggc tct gga gac acc acc gtg cgc     436
Pro Thr Gly Lys Tyr Leu Ala Ser Gly Ser Gly Asp Thr Thr Val Arg
        125                 130                 135

ttc tgg gat ctc agc aca gag aca cca cat ttc aca tgc aag gga cac     484
Phe Trp Asp Leu Ser Thr Glu Thr Pro His Phe Thr Cys Lys Gly His
140                 145                 150                 155

aga cac tgg gtc ctt agt ata tcc tgg tct cca gat ggc aag aag ctg     532
Arg His Trp Val Leu Ser Ile Ser Trp Ser Pro Asp Gly Lys Lys Leu
                160                 165                 170

gcc tca ggc tgc aag aat ggc cag att ctc ctc tgg gac cca agc aca     580
Ala Ser Gly Cys Lys Asn Gly Gln Ile Leu Leu Trp Asp Pro Ser Thr
            175                 180                 185

ggg aag cag gtg ggc agg acc ctc gct ggc cac agc aag tgg atc aca     628
Gly Lys Gln Val Gly Arg Thr Leu Ala Gly His Ser Lys Trp Ile Thr
        190                 195                 200

ggc ctg agc tgg gag ccc ctc cat gcg aac cct gag tgc cgc tat gtg     676
Gly Leu Ser Trp Glu Pro Leu His Ala Asn Pro Glu Cys Arg Tyr Val
    205                 210                 215

gcc agc agc tcc aag gat ggc agt gtg cgg atc tgg gac aca act gca     724
Ala Ser Ser Ser Lys Asp Gly Ser Val Arg Ile Trp Asp Thr Thr Ala
220                 225                 230                 235

ggc cgc tgt gag cgc atc ctc acc ggg cac acc cag tcg gtc acc tgt     772
Gly Arg Cys Glu Arg Ile Leu Thr Gly His Thr Gln Ser Val Thr Cys
                240                 245                 250
```

```
ctc cgg tgg gga ggg gac ggg ctt ctc tac tct gcc tcc cag gac cgc    820
Leu Arg Trp Gly Gly Asp Gly Leu Leu Tyr Ser Ala Ser Gln Asp Arg
            255                 260                 265

acc atc aaa gtc tgg aga gct cat gac ggt gtg ctg tgc cgg act ctg    868
Thr Ile Lys Val Trp Arg Ala His Asp Gly Val Leu Cys Arg Thr Leu
        270                 275                 280

caa ggc cac ggc cac tgg gtg aac acc atg gcc ctc agc act gac tat    916
Gln Gly His Gly His Trp Val Asn Thr Met Ala Leu Ser Thr Asp Tyr
    285                 290                 295

gcc ctg cgc act ggg gcc ttt gaa cct gct gag gcc tca gtt aat ccc    964
Ala Leu Arg Thr Gly Ala Phe Glu Pro Ala Glu Ala Ser Val Asn Pro
300                 305                 310                 315

caa gac ctc caa gga tcc ttg cag gag ttg aag gag agg gct ctg agc    1012
Gln Asp Leu Gln Gly Ser Leu Gln Glu Leu Lys Glu Arg Ala Leu Ser
                320                 325                 330

cga tac aac ctc gtg cgg ggc cag ggt cca gag agg ctg gtg tct ggc    1060
Arg Tyr Asn Leu Val Arg Gly Gln Gly Pro Glu Arg Leu Val Ser Gly
            335                 340                 345

tcc gac gac ttc acc tta ttc ctg tgg tcc cca gca gag gac aaa aag    1108
Ser Asp Asp Phe Thr Leu Phe Leu Trp Ser Pro Ala Glu Asp Lys Lys
        350                 355                 360

cct ctc act cgg atg aca gga cac caa gct ctc atc aac cag gtg ntc    1156
Pro Leu Thr Arg Met Thr Gly His Gln Ala Leu Ile Asn Gln Val Xaa
    365                 370                 375

ttc tct cct gac tcc cgc atc gtg gct agt gcc tcc ttt gac aag tcc    1204
Phe Ser Pro Asp Ser Arg Ile Val Ala Ser Ala Ser Phe Asp Lys Ser
380                 385                 390                 395

atc aag ctg tgg gat ggc agg acg ggc aag tac ctg gct tcc cta cgc    1252
Ile Lys Leu Trp Asp Gly Arg Thr Gly Lys Tyr Leu Ala Ser Leu Arg
                400                 405                 410

ggc cac gtg gct gcc gtg tac cag att gcg tgg tca gct gac agt cgg    1300
Gly His Val Ala Ala Val Tyr Gln Ile Ala Trp Ser Ala Asp Ser Arg
            415                 420                 425

ctc ctg gtc agc ggc agc agt gac agc aca ctg aag gtg tgg gat gtg    1348
Leu Leu Val Ser Gly Ser Ser Asp Ser Thr Leu Lys Val Trp Asp Val
        430                 435                 440

aag gcc cag aag ctg gcc atg gac ctg ccc ggc cac gcg gat gag gta    1396
Lys Ala Gln Lys Leu Ala Met Asp Leu Pro Gly His Ala Asp Glu Val
    445                 450                 455

tat gct gtt gac tgg agt cca gat ggc cag aga gtg gca agt ggt ggg    1444
Tyr Ala Val Asp Trp Ser Pro Asp Gly Gln Arg Val Ala Ser Gly Gly
460                 465                 470                 475

aag gac aaa tgc ctc cgg ata tgg agg aga tgagacggcc cgaagttctc      1494
Lys Asp Lys Cys Leu Arg Ile Trp Arg Arg
                480                 485

tctgaccccc acctcgactc ggcctctgcc agctgccttc cctgccagag aacaaaggct 1554
```

gagatggcag tgcacacacc ctccccacca gtggggacct gagaatgcgt gtggcctgct 1614

gtcctcgata gaccggaatg gggttttccc acagatcccc gcctgtggca caccccagag 1674

ccagaaatcg taggtcacag gaagttgtca ctgaacttgg cccgtgtctg ctactctgta 1734

ccttgctggt acagacaggg gtggtgggca gccaggctct atgagtgggc ccctagtgtc 1794

agctctgtac agggtcagat cccaggttct atgaccaaat aagtaactta aaaaaaaaaa 1854

aaaaa 1859

n=a, c, g or t

<210> 6
<211> 485
<212> PRT
<213> Homo sapiens

<400> 6

```
Met Ala Ala Ala Val Ala Asp Glu Ala Val Ala Arg Asp Val Gln Arg
1               5                   10                  15

Leu Leu Val Gln Phe Gln Asp Glu Gly Gly Gln Leu Leu Gly Ser Pro
            20                  25                  30

Phe Asp Val Pro Val Asp Ile Thr Pro Asp Arg Leu Gln Leu Val Cys
        35                  40                  45

Asn Ala Leu Leu Ala Gln Glu Asp Pro Leu Pro Leu Ala Phe Phe Val
    50                  55                  60

His Asp Ala Glu Ile Val Ser Ser Leu Gly Lys Thr Leu Glu Ser Gln
65                  70                  75                  80

Ala Val Glu Thr Glu Lys Val Leu Asp Ile Ile Tyr Gln Pro Gln Ala
                85                  90                  95

Ile Phe Arg Val Arg Ala Val Thr Arg Cys Thr Ser Ser Leu Glu Gly
            100                 105                 110

His Ser Glu Ala Val Ile Ser Val Ala Phe Ser Pro Thr Gly Lys Tyr
        115                 120                 125

Leu Ala Ser Gly Ser Gly Asp Thr Thr Val Arg Phe Trp Asp Leu Ser
    130                 135                 140

Thr Glu Thr Pro His Phe Thr Cys Lys Gly His Arg His Trp Val Leu
145                 150                 155                 160

Ser Ile Ser Trp Ser Pro Asp Gly Lys Lys Leu Ala Ser Gly Cys Lys
                165                 170                 175

Asn Gly Gln Ile Leu Leu Trp Asp Pro Ser Thr Gly Lys Gln Val Gly
            180                 185                 190

Arg Thr Leu Ala Gly His Ser Lys Trp Ile Thr Gly Leu Ser Trp Glu
        195                 200                 205

Pro Leu His Ala Asn Pro Glu Cys Arg Tyr Val Ala Ser Ser Ser Lys
    210                 215                 220
```

```
Asp Gly Ser Val Arg Ile Trp Asp Thr Thr Ala Gly Arg Cys Glu Arg
225                 230                 235                 240

Ile Leu Thr Gly His Thr Gln Ser Val Thr Cys Leu Arg Trp Gly Gly
                245                 250                 255

Asp Gly Leu Leu Tyr Ser Ala Ser Gln Asp Arg Thr Ile Lys Val Trp
            260                 265                 270

Arg Ala His Asp Gly Val Leu Cys Arg Thr Leu Gln Gly His Gly His
        275                 280                 285

Trp Val Asn Thr Met Ala Leu Ser Thr Asp Tyr Ala Leu Arg Thr Gly
    290                 295                 300

Ala Phe Glu Pro Ala Glu Ala Ser Val Asn Pro Gln Asp Leu Gln Gly
305                 310                 315                 320

Ser Leu Gln Glu Leu Lys Glu Arg Ala Leu Ser Arg Tyr Asn Leu Val
                325                 330                 335

Arg Gly Gln Gly Pro Glu Arg Leu Val Ser Gly Ser Asp Asp Phe Thr
            340                 345                 350

Leu Phe Leu Trp Ser Pro Ala Glu Asp Lys Lys Pro Leu Thr Arg Met
        355                 360                 365

Thr Gly His Gln Ala Leu Ile Asn Gln Val Xaa Phe Ser Pro Asp Ser
    370                 375                 380

Arg Ile Val Ala Ser Ala Ser Phe Asp Lys Ser Ile Lys Leu Trp Asp
385                 390                 395                 400

Gly Arg Thr Gly Lys Tyr Leu Ala Ser Leu Arg Gly His Val Ala Ala
                405                 410                 415

Val Tyr Gln Ile Ala Trp Ser Ala Asp Ser Arg Leu Leu Val Ser Gly
            420                 425                 430

Ser Ser Asp Ser Thr Leu Lys Val Trp Asp Val Lys Ala Gln Lys Leu
        435                 440                 445

Ala Met Asp Leu Pro Gly His Ala Asp Glu Val Tyr Ala Val Asp Trp
    450                 455                 460

Ser Pro Asp Gly Gln Arg Val Ala Ser Gly Gly Lys Asp Lys Cys Leu
465                 470                 475                 480

Arg Ile Trp Arg Arg
                485
```

## Description

### FIELD OF THE INVENTION

The present invention pertains generally to TZap kinase 7 cDNAs TZap7/A, TZap7/B and TZap7 encoding novel immune response modulating proteins as well as peptides and polypeptides derived therefrom and antibodies recognizing said (poly)peptides. In a first aspect, the present invention relates to TZap7/A, TZap7/B and TZap7 cDNAs and their encoded proteins. In a further aspect, the present invention relates to polynucleotides derived from said TZap7/A, TZap7/B and TZap7 cDNAs encoding a peptide or polypeptide being capable of modulating immune responses. Furthermore, the present invention relates to vectors comprising such polynucleotides and host cells transformed therewith as well as their use in the production of the above-defined peptides or polypeptides. In addition, the present invention relates to the (poly)peptide encoded by said polynucleotides or obtainable by the method of the invention. In another important aspect the present invention relates to antibodies against said TZap7/A, TZap7/B and TZap7 proteins. Furthermore, the present invention relates to the use of antisense polynucleotides and vectors containing the same for the suppression of growth of leukocytes, in particular lymphocytes and monocytes. The present invention additionally relates to pharmaceutical and diagnostic compositions comprising the aforementioned polynucleotides, vectors, proteins, or antibodies.

Furthermore, the present invention relates to methods and uses for modulating immune responses through the novel TZap7/A, TZap7/B and TZap7 proteins as well as to pharmaceutical compositions comprising agents which act on the TZap7/A, TZap7/B and TZap7 proteins or their ligand. Also, the invention relates to the use of the before-described polynucleotide, vector, protein or antibody for the preparation of pharmaceutical compositions for use in organ transplantation, for the treatment of autoimmune, allergic or infectious diseases, or for treatment of tumors. Furthermore, the present invention relates to methods for modulating (antigen-specific) T, B, NK cell or monocyte (un)responsiveness.

### BACKGROUND OF THE INVENTION

T cell activation is accompanied with sequential changes in the expression of various genes over several days and involves multiple signaling pathways [1]. Stimulation of T cells is initiated by the interaction of antigen-specific T cell receptors (TCR) with MHC bound antigenic peptides presented on the surface of antigen presenting cells (APC), but full proliferative T cell response requires additional costimulatory signals which are provided by the interaction of proteins expressed on the surface of T cells and APC [2,3,4,5]. In addition, a number of cytokines as well as other proteins are known to augment T cell activation, although many of them appear not to be essential for the basic proliferative T cell response [3,6]. Moreover, a growing body of evidence indicates that the microtubule cytoskeleton of lymphocytes plays a major role in T cell activation. Stimulation of T cells was demonstrated to result in molecular rearrangement in the actin cytoskeleton leading to re-localization and concentration of signaling molecules in restricted areas of the cell membrane close to the bound APC [7,8,9].

Although considerable information on T cell activation has been gathered in recent years, the complex molecular mechanisms of stimulation and signaling pathways are not completely understood. Since T cell activation provides the central event in various types of inflammation as well as in autoimmune disease and graft rejection, knowledge about the distinct steps and molecules involved in the stimulation process is of considerable biomedical importance, as they might provide targets for therapeutic modulation of the immune response. Therapeutic prevention of T cell activation in organ transplantation and autoimmune diseases presently relies on panimmunosuppressive drugs interfering with downstream intracellular events. Specific modulation of the T cell response remains a longstanding goal in immunological research.

### SUMMARY OF THE INVENTION

The present invention relates to polynucleotides encoding a novel immune response modulating protein. Furthermore, the present invention relates to peptides and polypeptides derived therefrom as well as to antibodies. More particularly, the present invention relates to applications in the medical field that directly arise from the polynucleotides, protein, peptides, (poly)peptides and antibodies of the invention. Additionally, the present invention relates to a novel method for testing modulators of the immune response. The pharmaceutical compositions, methods and uses of the invention are useful therapeutically in situations where it is desirable to modulate (antigen-specific) immune responses, e.g., to induce and maintain (antigen-specific) T cell or B-cell unresponsiveness or restore (antigen-specific) B or T cell responsiveness. For example, it may be necessary to induce or maintain T cell unresponsiveness in a subject who has received an organ or bone marrow transplant to prevent graft rejection by inhibiting stimulation through the TZap7/A, TZap7/B and/or TZap7 protein. In addition, T cell unresponsiveness can be maintained by blocking TZap7/A, TZap7/B and/or TZap7 stimulation in a subject who has an autoimmune disease to alleviate symptoms of the autoimmune disease.

In these cases, a TZap7/A, TZap7/B and/or TZap7 inhibitory agent is administered to the subject in an amount and over a period of time sufficient to maintain T cell unresponsiveness. Alternatively, T cell unresponsiveness can be reversed in a subject bearing a tumor to stimulate a tumor specific NK and T cell response or in a subject receiving a vaccine to enhance the efficacy of the vaccine. For example, a cell (e.g., a tumor cell) can be modified to express a TZap7/A, TZap7/B and/or TZap7 ligand or a TZap7/A, TZap7/B and/or TZap7 stimulatory agent can be administered to the subject bearing a tumor or who has had a tumor surgically removed to prevent recurrence of the tumor.

## BRIEF DESCRIPTION OF DRAWINGS

This disclosure may best be understood in conjunction with the accompanying drawings, incorporated herein by references, which show:

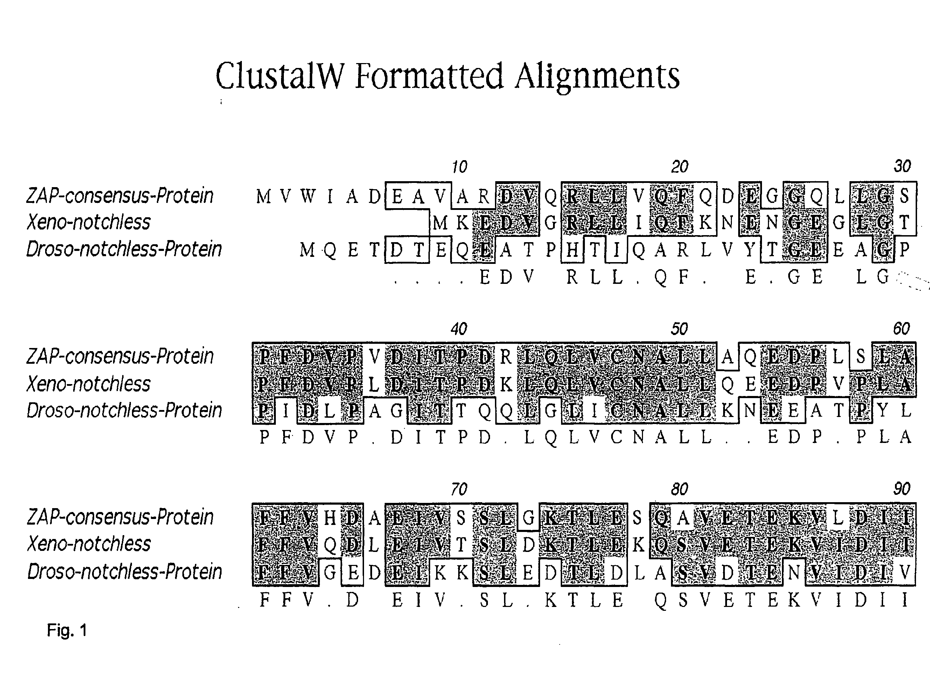

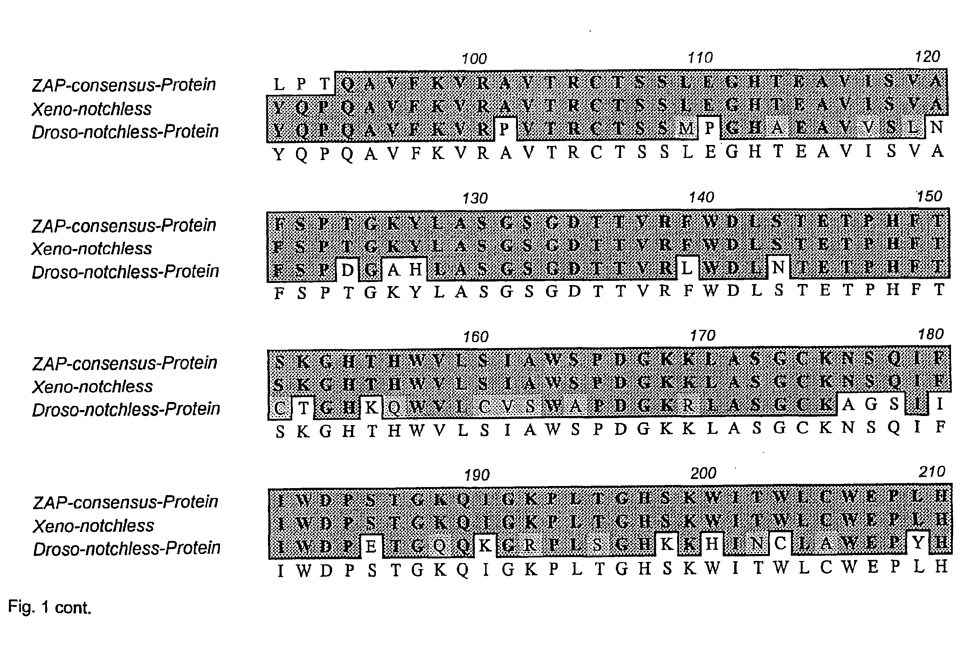

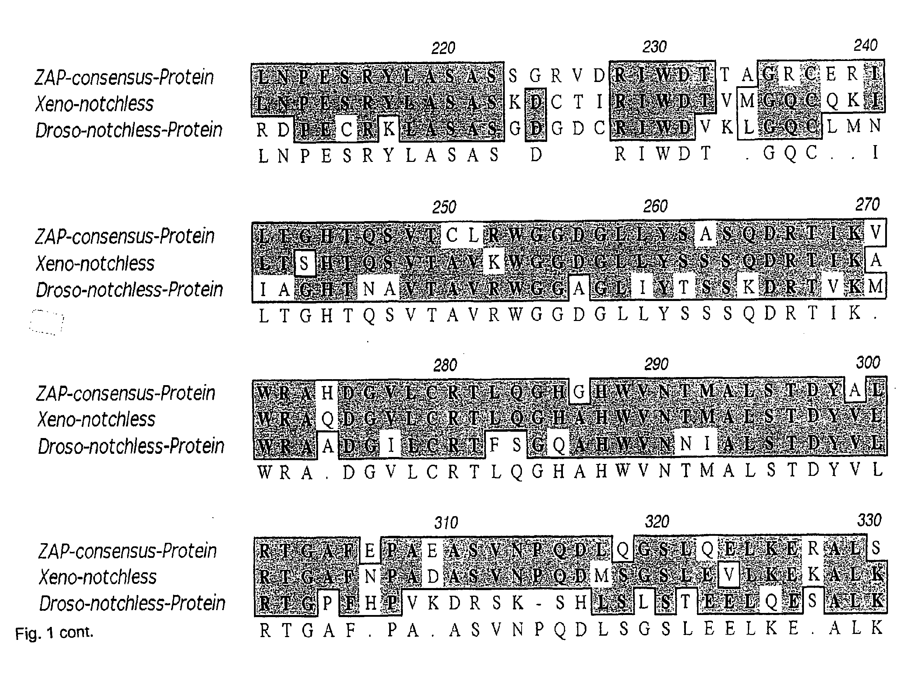

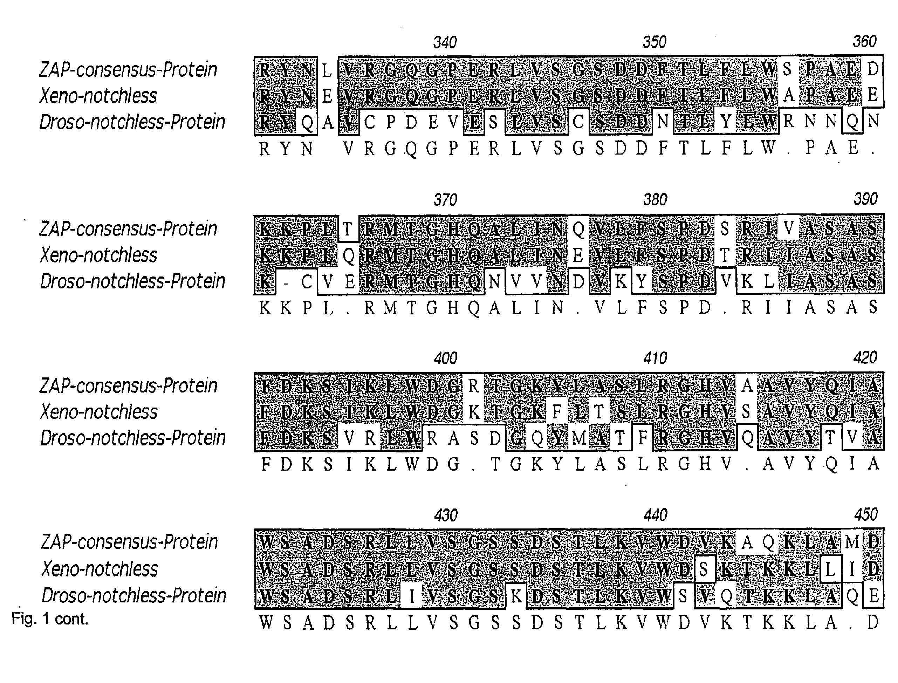

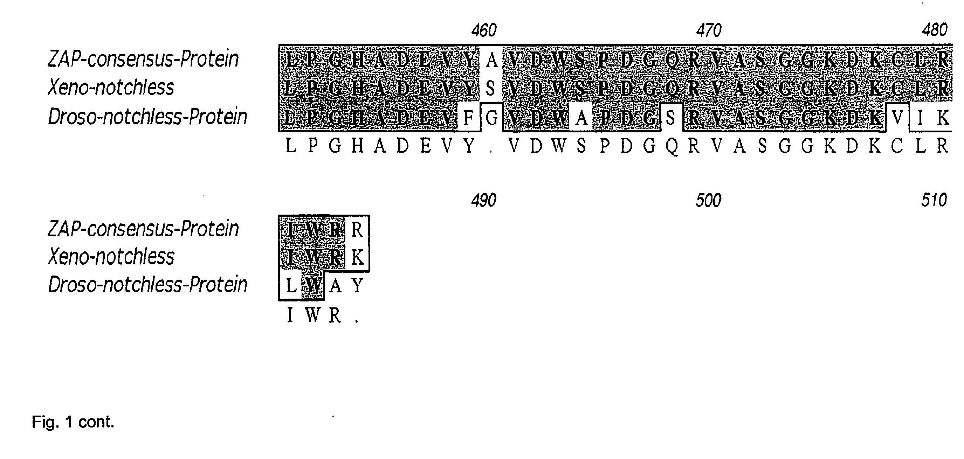

**Figure 1:** Human TZap B (Zap-consensus) amino acid sequence shares a high degree of identity with notchless protein in two different species (Xenopus and Drosophila) as shown in boxes.

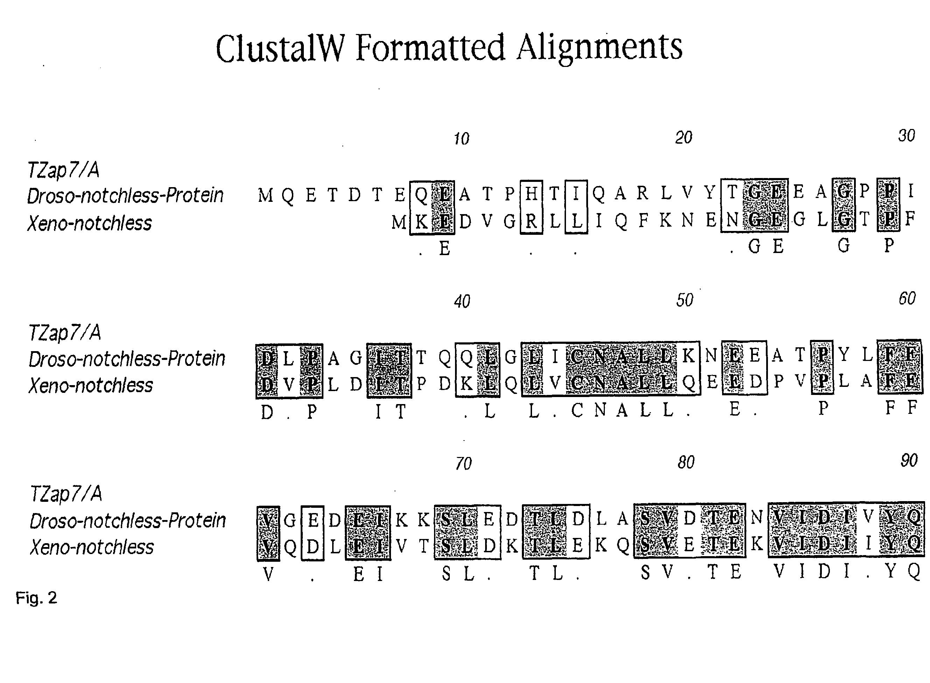

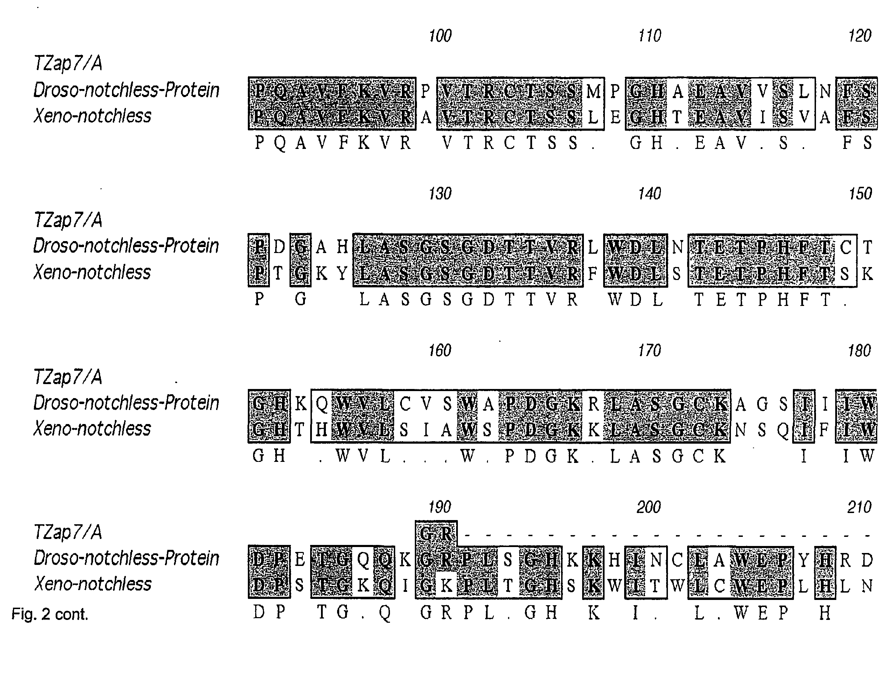

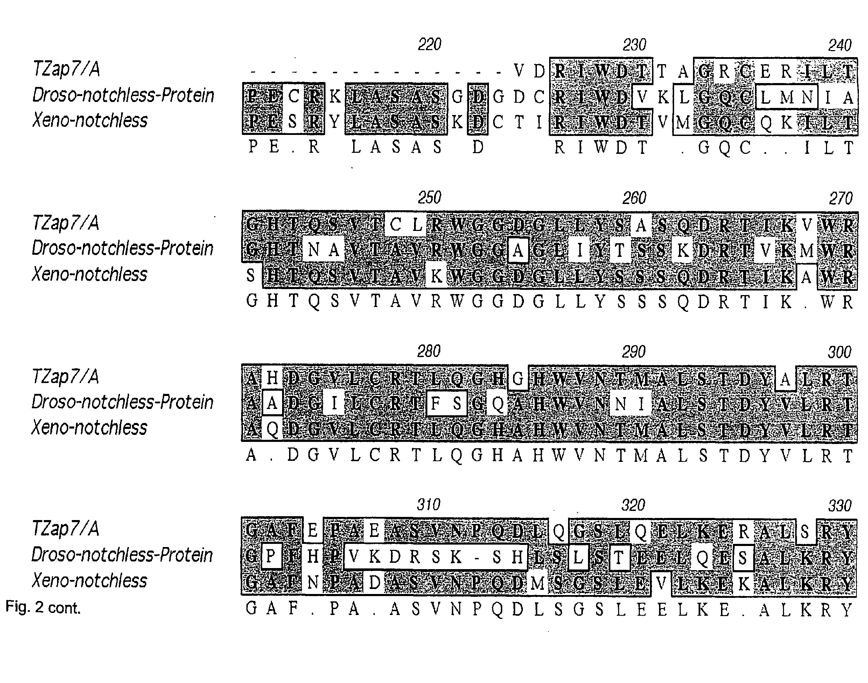

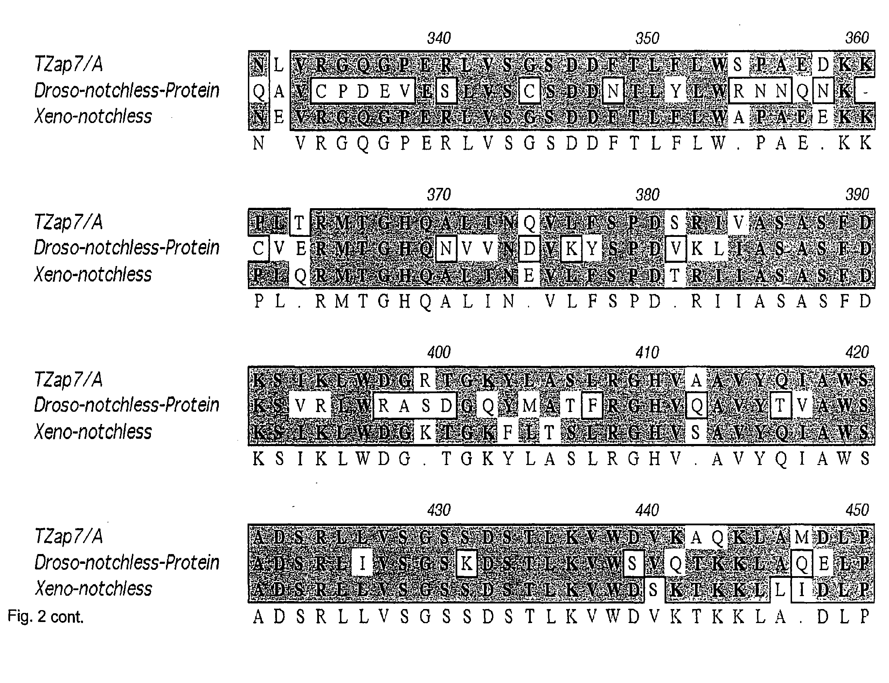

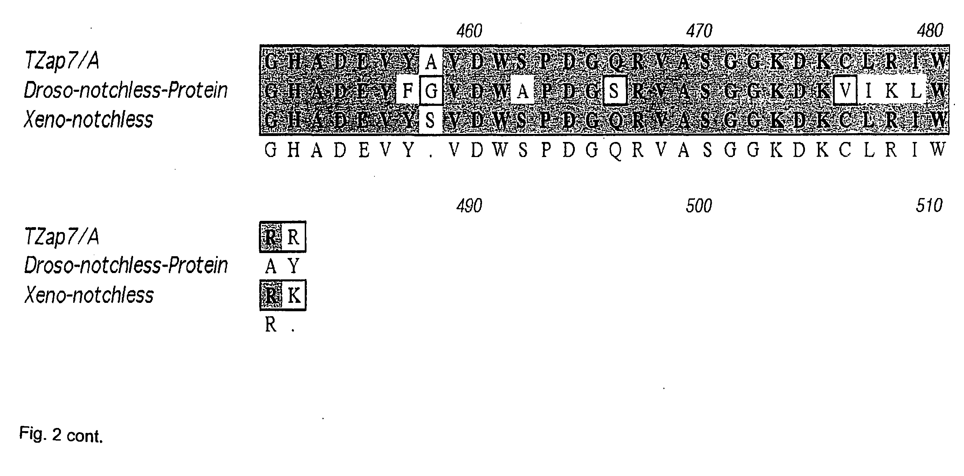

**Figure 2:** Human TZap A (Tzap7/A) amino acid sequence shows high degree of identity with notchless proteins in Xenopus and Drosophila.

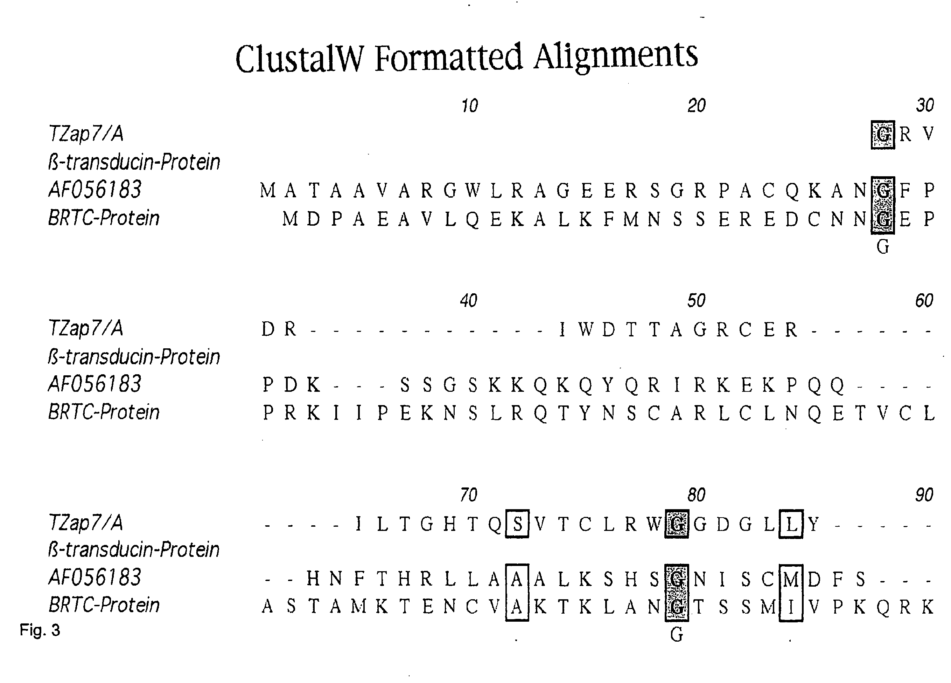

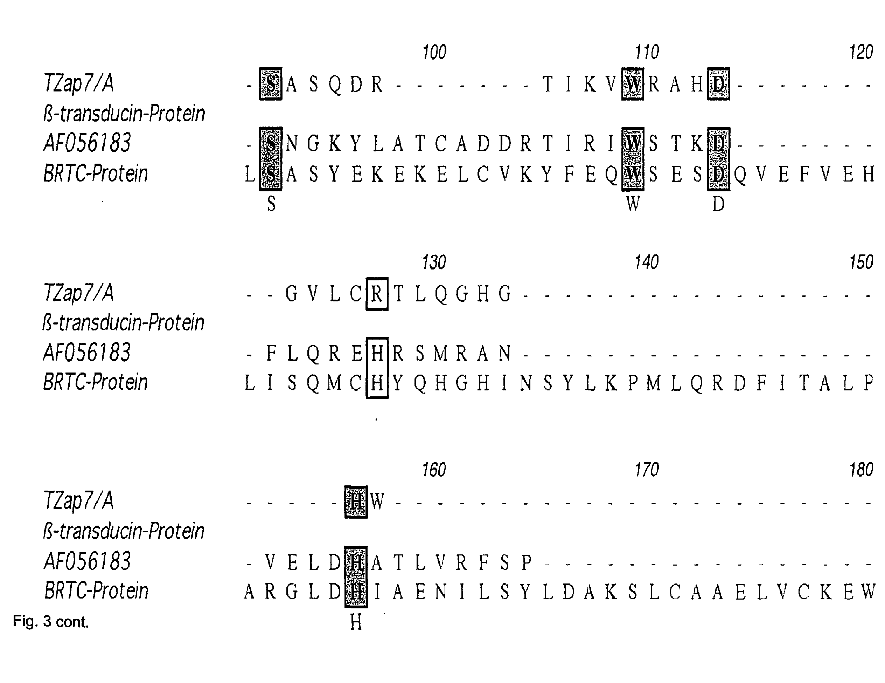

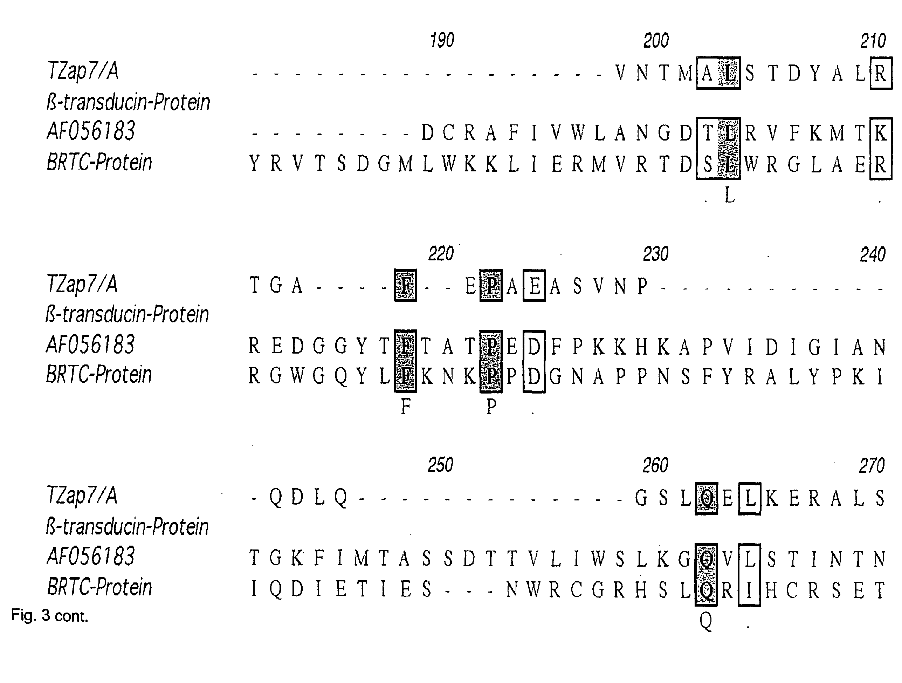

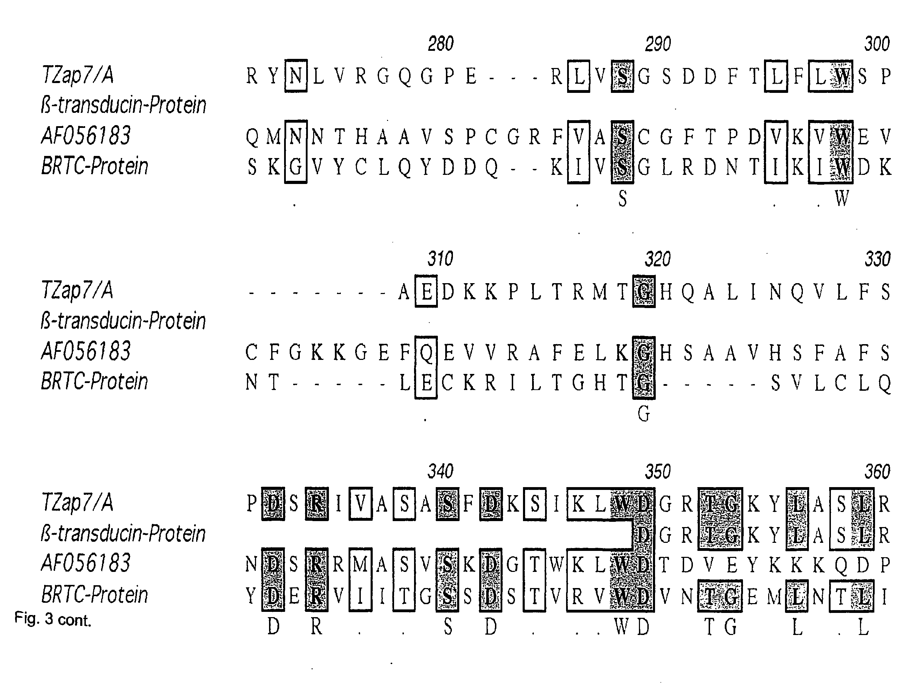

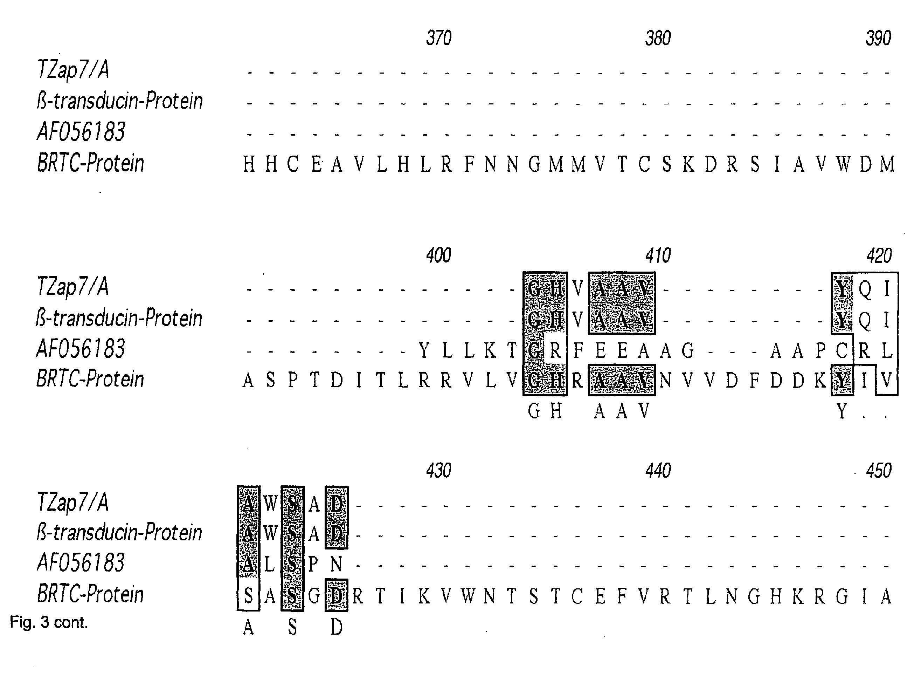

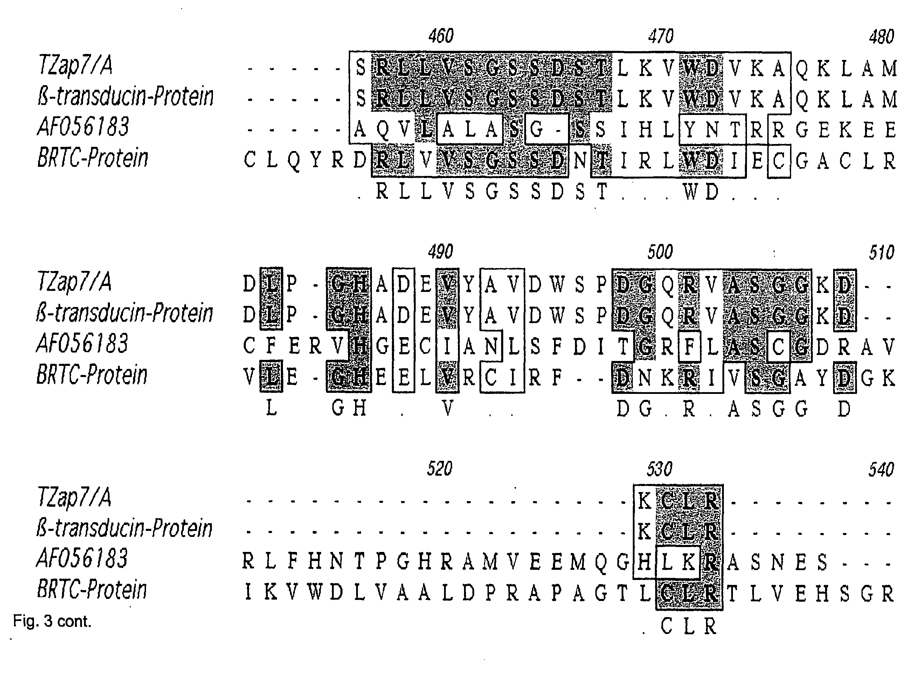

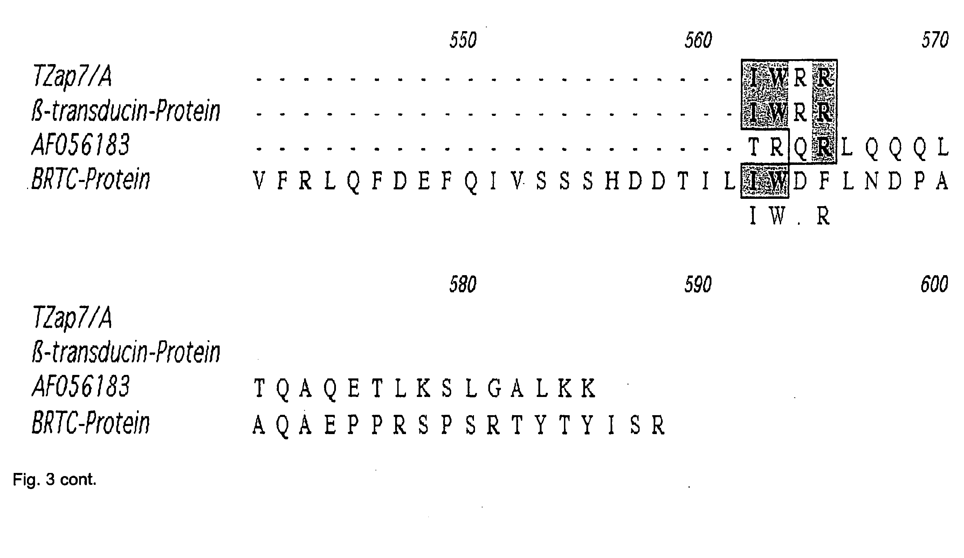

**Figure 3:** The amino acid sequence comparison between human TZap7 amino acid sequence and betatransducin proteins exhibits a number of motif-homology stretches to betatransducin protein within the 3' region (boxes).

**Figure 4:** Human TZap7 amino acid sequence shows high degree of identity with notchless proteins in Xenopus and Drosphila.

## DETAILED DESCRIPTION OF THE PRESENT INVENTION

In view of the need of therapeutic means for the diagnosis and treatment of diseases related to immune responses of the human body, the technical problem of the invention is to provide means and methods for the early detection and modulation of immune cell responses which are particularly useful in organ transplantation and autoimmune diseases. The solution to this technical problem is achieved by providing the embodiments characterized in the claims, namely novel immune response modulating proteins encoded by cell immune response cDNAs designated TZap7/A, TZap7/B and/or TZap7 are described which exhibit a central role in T cell activation. Thus, targeting of TZap7/A, TZap7/B and/or TZap7 protein and its encoding gene provides a novel therapeutic approach for modulation of the immune response. Accordingly, the invention relates to a polynucleotide encoding a protein consisting of a DNA sequence selected from the group consisting of:

(i) DNA sequences comprising a nucleotide sequence encoding the amino acid sequence depicted in SEQ ID NO. 2, 4 or 6;
(ii) DNA sequences comprising the nucleotide sequence depicted in SEQ ID NO. 1, 3 or 5;
(iii) DNA sequences the complementary strand of which hybridizes with and which is at least 90% identical to the polynucleotide as defined in any one of (i) to (iii); and
(iv) DNA sequences the nucleotide of which is degenerate to the nucleotide sequence of a DNA sequence of any one of (i) to (iii).

The terms "TZap7/A, TZap7/B and/or TZap7 protein", and "TZap" are used interchangeably herein and in accordance with the present invention, denote a protein involved in the modulation of immune responses, e.g. modulating activation and differentiation of T cells. Studies which had been carried out within the scope of the present invention revealed that antisense polynucleotides directed to the mRNA encoding TZap protein are able to efficiently activate Notch, a class of proteins involved in signal transduction further explained in Example 1.

The term "leukocytes" generally denotes all kinds of white blood cells and preferably refers to monocytes and lymphocytes (B, T and NK cells), either in combination or individually. Thus, it should be understood that the term leukocyte may also be used herein so as to refer to individual species of leukocytes such as T cells only.

The term "biologically active fragment thereof" refers to peptides and polypeptides that are derived from said TZap7/A, TZap7/B and/or TZap7 protein and that are capable of effecting substantially the same or similar activity or at least one of said activities of TZap7/A (29 kDA), TZap7/B (53 kDA) and/or TZap7 (53 kDA).

In accordance with the present invention, novel genes induced in the early stage of T cell activation have been identified by examining mRNA expression in alloactivated human lymphocytes. Differential display-reverse transcription PCR analysis revealed a 230 bp cDNA fragment which was upregulated 24 h after allostimulation of a human T cell line and three corresponding cDNAs designated TZap7/A, TZap7/B and TZap7, respectively, have been cloned; see Example 1.

Expression studies with TZap7/A on mRNA of tissues from the human immune system revealed two bands of 1200 nt and 4000 nt, respectively. No differences in the expression level of human Nle in spleen, lymph Node, thymus, peripheral blood leukocytes, bone marrow and fetal liver could be detected. Most of these expression domains correspond to regions where Notch signaling has been implicated in cell fate specifications. Overexpression of notchless in the AKR1010 DP thymoma cell line leads to an activation of Notch resulting in a high level expression of TCR. Control cells do not exhibit detectable amounts of TCR on their surface.

Application of antisense RNA of TZap7/A to bone marrow and thymus of mice, resulted in an activation of Notch. In thymus this activation leads to a slight increase of CD8 T cells over CD4 T cells. In bone marrow, an increase of thymic-independent T cells was demonstrated, although this increase was not accompanied by a persistent block in B cell maturation, as it was shown for Notch overexpression in the bone marrow (Pui JC, Allman D, Xu L, DeRocco S, Karnell FG, Bakkour S, Lee JY, Kadesch T, Hardy RR, Aster JC, Pear WS (1999): Notch1 expression in early lymphopoiesis influences B versus T lineage determination. Immunity, 11, 299-308).

TZap7/A, TZap7/B and TZap7 share homology with two different family of proteins which are involved in directing the immune response in different ways.

Beta-transducin is one of the three subunits of the guanine nucleotide-binding proteins (G proteins) which act as intermediaries in the transduction of signals generated by transmembrane receptors (Neer EJ, Schmidt CJ, Nambudripad R, Smith TF (1994): The ancient regulatory-protein family of WD-repeat proteins. Nature, 371, 297-300 and Neer EJ and Smith TF (1996) G protein heterodimers: new structures propel new questions. Cell, 84, 175-178).

In higher eukaryotes G-beta exists as a small multigene family of highly conserved proteins. They consist of eight tandem repeats of about 40 residues, each containing a central Trp-Asp motif which is called also WD-40 repeat. Beta-transducin repeat containing protein (HSBTRCP) has been shown to interact with HIV-1 Vpu which connects CD4 to the ER degradation pathway (Smith TF, Gaitatzes CG, Saxena K, Neer EJ. (1999) The WD repeat: a common architecture for diverse functions. TIBS, 24, 181-185).

On one side, TZap7/A, TZap7/B and TZap7 share high percentage of identity on the nucleotide level within the 3'-region with beta-transducin family proteins supporting the hypothesis to be functionally involved in mediating the immune reaction during the course of response to an antigen. On the other side the same region is homologue to the notchless proteins in different species.

All these proteins have in common that they share WD-repeat motifs and are directly involved in early signalling cascade upon cell activation. Based on the homologies, it can be concluded that modulation of TZap by using antisense, small molecule, antibody as well as overexpression of the TZap proteins might lead to selected modulation of immune response and can serve as a novel target to treat diseases involving undesired immune reaction.

From the above it is evident that the nucleotide sequences depicted in SEQ ID No. 1, 3 and 5 encode novel immune response modulating proteins. By the provision of this nucleotide sequence it is now possible to isolate identical or similar polynucleotides which code for proteins with the biological activity of TZap7/A, TZap7/B and TZap7 from other species or organisms. Well-established approaches for the identification and isolation of such related sequences are, for example, the isolation from genomic or cDNA libraries using the complete or part of the disclosed sequence as a probe or the amplification of corresponding polynucleotides by polymerase chain reaction using specific primers.

Thus, the invention also relates to polynucleotides which hybridize to the above-described polynucleotides and differ at one or more positions in comparison to these as long as they encode a TZap7/A, TZap7/B or TZap7 protein as defined above. Such molecules comprise those which are changed, for example, by deletion(s), insertion(s), alteration (s) or any other modification known in the art in comparison to the above described polynucleotides either alone or in combination. Methods for introducing such modifications in the polynucleotides of the invention are well-known to the person skilled in the art; see, e.g., Sambrook et al. (Molecular cloning; A Laboratory Manual, Second Edition, Cold Spring Harbor Laboratory Press, Cold Spring Harbor NY (1989)). The invention also relates to polynucleotides the nucleotide sequence of which differs from the nucleotide sequence of any of the above-described polynucleotides due to the degeneracy of the genetic code.

With respect to the DNA sequences characterized under (iv) above, the term "hybridizing" in this context is understood as referring to conventional hybridization conditions, preferably such as hybridization in 50%formamide/6xSSC/0.1 %SDS/100μg/ml ssDNA, in which temperatures for hybridization are above 37°C and temperatures for washing in 0.1xSSC/0.1%SDS are above 55°C. Most preferably, the term "hybridizing" refers to stringent hybridization conditions, for example such as described in Sambrook, supra.

Nucleic acid hybridization will be affected by such conditions as salt concentration, temperature, solvents, the base composition of the hybridizing species, length of the complementary regions, and the number of nucleotide base mismatches between the hybridizing nucleic acids, as will be readily appreciated by those skilled in the art. "Stringent hybridization conditions" and "stringent wash conditions" in the context of nucleic acid hybridization experiments depend upon a number of different physical parameters. The most important parameters include temperature of hybridization, base composition of the nucleic acids, salt concentration and length of the nucleic acid. One having ordinary skill in the art knows how to vary these parameters to achieve a particular stringency of hybridization. In general, "stringent hybridization" is performed at about 25°C below the thermal melting point (T m) for the specific DNA hybrid under a particular set of conditions. "Stringent washing" is performed at temperatures about 5°C lower than the Tm for the specific DNA hybrid under a particular set of conditions. The Tm is the temperature at which 50% of the target sequence hybridizes to a perfectly matched probe. See Sambrook et al., page 9.51, hereby incorporated by reference.

The Tm for a particular DNA-DNA hybrid can be estimated by the formula:

Tm = 81.5°C + 16.6 (log10[Na+ ]) + 0.41 (fraction G + C) - 0.63 (% formamide) - (600/l) where l is the length of the hybrid in base pairs.

The Tm for a particular RNA-RNA hybrid can be estimated by the formula:

Tm = 79.8°C + 18.5 (log10[Na+ ]) + 0.58 (fraction G + C) + 11.8 (fraction G + C)2 - 0.35 (% formamide) - (820/l).

The Tm for a particular RNA-DNA hybrid can be estimated by the formula:

Tm = 79.8°C + 18.5(log10[Na+ ]) + 0.58 (fraction G + C) + 11.8 (fraction G + C)2 - 0.50 (% formamide) - (820/l).

In general, the Tm decreases by 1-1.5°C for each 1 % of mismatch between two nucleic acid sequences. Thus, one having ordinary skill in the art can alter hybridization and/or washing conditions to obtain sequences that have higher or lower degrees of sequence identity to the target nucleic acid. For instance, to obtain hybridizing nucleic acids that contain up to 10% mismatch from the target nucleic acid sequence, 10-15°C would be subtracted from the calculated Tm of a perfectly matched hybrid, and then the hybridization and washing temperatures adjusted accordingly. Probe sequences may also hybridize specifically to duplex DNA under certain conditions to form triplex or other higher order DNA complexes. The preparation of such probes and suitable hybridization conditions are well known in the art.

An example of stringent hybridization conditions for hybridization of complementary nucleic acid sequences having more than 100 complementary residues on a filter in a Southern or Northern blot or for screening a library is 50% formamide/6X SSC at 42°C for at least ten hours. Another example of stringent hybridization conditions is 6X SSC at 68°C for at least ten hours. An example of low stringency hybridization conditions for hybridization of complementary nucleic acid sequences having more than 100 complementary residues on a filter in a Southern or northern blot or for screening a library is 6X SSC at 42°C for at least ten hours. Hybridization conditions to identify nucleic acid sequences that are similar but not identical can be identified by experimentally changing the hybridization temperature from 68°C to 42°C while keeping the salt concentration constant (6X SSC), or keeping the hybridization temperature and salt concentration constant (e.g. 42°C and 6X SSC) and varying the formamide concentration from 50% to 0%. Hybridization buffers may also include blocking agents to lower background. These agents are well-known in the art. See Sambrook et al., pages 8.46 and 9.46-9.58, herein incorporated by reference.

Wash conditions also can be altered to change stringency conditions. An example of stringent wash conditions is a 0.2x SSC wash at 65°C for 15 minutes (see Sambrook et al., for SSC buffer). Often the high stringency wash is preceded by a low stringency wash to remove excess probe. An exemplary medium stringency wash for duplex DNA of more than 100 base pairs is 1x SSC at 45°C for 15 minutes. An exemplary low stringency wash for such a duplex is 4x SSC at 40°C for 15 minutes. In general, signal-to-noise ratio of 2x or higher than that observed for an unrelated probe in the particular hybridization assay indicates detection of a specific hybridization.

The term "percent sequence identity" or "identical" in the context of nucleic acid sequences refers to the residues in the two sequences which are the same when aligned for maximum correspondence. The length of sequence identity comparison may be over a stretch of at least about nine nucleotides, usually at least about 20 nucleotides, more usually at least about 24 nucleotides, typically at least about 28 nucleotides, more typically at least about 32 nucleotides, and preferably at least about 36 or more nucleotides. There are a number of different algorithms known in the art which can be used to measure nucleotide sequence identity. For instance, polynucleotide sequences can be compared using NCBI BLASTx and BLASTn software. Alternatively, Fasta, a program in GCG Version 6.1. Fasta provides alignments and percent sequence identity of the regions of the best overlap between the query and search sequences (Pearson, 1990, herein incorporated by reference). For instance, percent sequence identity between nucleic acid sequences can be determined using Fasta with its default parameters (a word size of 6 and the NOPAMfactor for the scoring matrix) as provided in GCG Version 6.1, herein incorporated by reference.

Particularly preferred are polynucleotides which share 70%, preferably at least 85%, more preferably 90-95%, and most preferably 96-99% sequence identity with one of the above-mentioned polynucleotides and have the same biological activity. Such polynucleotides also comprise those which are altered, for example by nucleotide deletion(s), insertion(s), substitution(s), addition(s), and/or recombination(s) and/or any other modification(s) known in the art either alone or in combination in comparison to the above-described polynucleotides. Methods for introducing such modifications in the nucleotide sequence of the polynucleotide of the invention are well known to the person skilled in the art. Thus, the present invention encompasses any polynucleotide that can be derived from the above-described polynucleotides by way of genetic engineering and that encode upon expression a TZap7/A, TZap7/B and/or TZap7 protein or a biologically active fragment thereof.

It is also immediately evident to the person skilled in the art that regulatory sequences may be added to the polynucleotide of the invention. For example, promoters, transcriptional enhancers and/or sequences which allow for induced expression of the polynucleotide of the invention may be employed. A suitable inducible system is for example tetracycline-regulated gene expression as described, e.g., by Gossen and Bujard (Proc. Natl. Acad. Sci. USA 89 (1992), 5547-5551) and Gossen et al. (Trends Biotech. 12 (1994), 58-62).

In a further embodiment, the invention relates to nucleic acid molecules of at least 15 nucleotides in length hybridizing specifically with a polynucleotide as described above or with a complementary strand thereof. Specific hybridization occurs preferably under stringent conditions and implies no or very little cross-hybridization with nucleotide sequences encoding no or substantially different proteins. Such nucleic acid molecules may be used as probes and/or for the control of gene expression. Nucleic acid probe technology is well known to those skilled in the art who will readily appreciate that such probes may vary in length. Preferred are nucleic acid probes of 17 to 35 nucleotides in length. Of course, it may also be appropriate to use nucleic acids of up to 100 and more nucleotides in length. The nucleic acid probes of the invention are useful for various applications. On the one hand, they may be used as PCR primers for amplification of polynucleotides according to the invention. Another application is the use as a hybridization probe to identify polynucleotides hybridizing to the polynucleotides of the invention by homology screening of genomic DNA libraries. Nucleic acid molecules according to this preferred embodiment of the invention which are complementary to a polynucleotide as described above may also be used for repression of expression of a gene comprising such a polynucleotide, for example due to an antisense or triple helix effect or for the construction of appropriate ribozymes (see, e.g., EP-A1 0 291 533, EP-A1 0 321 201, EP-A2 0 360 257) which specifically cleave the (pre)-mRNA of a gene comprising a polynucleotide of the invention. Selection of appropriate target sites and corresponding ribozymes can be done as described for example in Steinecke, Ribozymes, Methods in Cell Biology 50, Galbraith et al. eds Academic Press, Inc. (1995), 449-460. Standard methods relating to antisense technology have also been described (Melani, Cancer Res. 51 (1991), 2897-2901). Furthermore, the person skilled in the art is well aware that it is also possible to label such a nucleic acid probe with an appropriate marker for specific applications, such as for the detection of the presence of a polynucleotide of the invention in a sample derived from an organism.

The above described nucleic acid molecules may either be DNA or RNA or a hybrid thereof. Furthermore, said nucleic acid molecule may contain, for example, thioester bonds and/or nucleotide analogues, commonly used in oligonucleotide anti-sense approaches. Said modifications may be useful for the stabilization of the nucleic acid molecule against endo- and/or exonucleases in the cell. Said nucleic acid molecules may be transcribed by an appropriate vector containing a chimeric gene which allows for the transcription of said nucleic acid molecule in the cell. Such nucleic acid molecules may further contain ribozyme sequences as described above.

In this respect, it is also to be understood that the polynucleotide of the invention can be used for "gene targeting" and/or "gene replacement", for restoring a mutant gene or for creating a mutant gene via homologous recombination; see for example Mouellic, Proc. Natl. Acad. Sci. USA, 87 (1990), 4712-4716; Joyner, Gene Targeting, A Practical Approach, Oxford University Press.

In a preferred embodiment said nucleic acid molecules are labeled. Methods for the detection of nucleic acids are well known in the art, e.g., Southern and northern blotting, PCR or primer extension. In another preferred embodiment said nucleic acid molecules may be used for the suppression of TZap7/A, TZap7/B and/or TZap7 expression.

The polynucleotide of the invention encoding the above described TZap7/A, TZap7/B and/or TZap7 protein or biologically active fragments thereof may be, e.g., DNA, cDNA, RNA or synthetically produced DNA or RNA or a recombinantly produced chimeric nucleic acid molecule comprising any of those polynucleotides either alone or in combination. Preferably said polynucleotide is part of a vector. Such vectors may comprise further genes such as marker genes which allow for the selection of said vector in a suitable host cell and under suitable conditions. Preferably, the polynucleotide of the invention is operatively linked to expression control sequences allowing expression in prokaryotic or eukaryotic cells. Expression of said polynucleotide comprises transcription of the polynucleotide into a translatable mRNA. Regulatory elements ensuring expression in eukaryotic cells, preferably mammalian cells, are well known to those skilled in the art. They usually comprise regulatory sequences ensuring initiation of transcription and optionally poly-A signals ensuring termination of transcription and stabilization of the transcript. Additional regulatory elements may include transcriptional as well as translational enhancers, and/or naturally-associated or heterologous promoter regions. Possible regulatory elements permitting expression in prokaryotic host cells comprise, e.g., the $P_L$, lac, trp or tac promoter in E. coli, and examples for regulatory elements permitting expression in eukaryotic host cells are the AOX1 or GAL1 promoter in yeast or the CMV-, SV40- , RSV-promoter (Rous sarcoma virus), CMV-enhancer, SV40-enhancer or a globin intron in mammalian and other animal cells. Beside elements which are responsible for the initiation of transcription, such regulatory elements may also comprise transcription termination signals, such as the SV40-poly-A site or the tk-poly-A site, downstream of the polynucleotide. Furthermore, depending on the expression system used leader sequences capable of directing the polypeptide to a cellular compartment or secreting it into the medium may be added to the coding sequence of the polynucleotide of the invention and are well known in the art. The leader sequence(s) is (are) assembled in appropriate phase with translation, initiation and termination sequences, and preferably, a leader sequence capable of directing secretion of translated protein, or a portion thereof, into the periplasmic space or extracellular medium. Optionally, the heterologous sequence can encode a fusion protein including a C- or N-terminal identification peptide imparting desired characteristics, e.g., stabilization or simplified purification of expressed recombinant product. In this context, suitable expression vectors are known in the art such as Okayama-Berg cDNA expression vector pcDV1 (Pharmacia), pCDM8, pRc/CMV, pcDNA1, pcDNA3 (In-vitrogene), or pSPORT1 (GIBCO BRL).

Preferably, the expression control sequences will be eukaryotic promoter systems in vectors capable of transforming or transfecting eukaryotic host cells, but control sequences for prokaryotic hosts may also be used. Once the vector has been incorporated into the appropriate host, the host is maintained under conditions suitable for high level expression of the nucleotide sequences, and, as desired, the collection and purification of the protein of the invention may follow; see, e.g., the appended examples. In one preferred embodiment of the present invention antisense constructs are made based on the polynucleotide of the invention and combined with an appropriate expression control sequence.

In accordance with the above, the present invention relates to vectors, particularly plasmids, cosmids, viruses and bacteriophages used conventionally in genetic engineering that comprise a polynucleotide of the invention. Methods which are well known to those skilled in the art can be used to construct recombinant vectors; see, for example, the techniques described in Sambrook, Molecular Cloning A Laboratory Manual, Cold Spring Harbor Laboratory (1989) N.Y. and Ausubel, Current Protocols in Molecular Biology, Green Publishing Associates and Wiley Interscience, N.Y. (1989). Alternatively, the polynucleotides and vectors of the invention can be reconstituted into liposomes for delivery to target cells. The vectors containing the polynucleotides of the invention can be transferred into the host cell by well-known methods, which vary depending on the type of cellular host. For example, calcium chloride transfection is commonly utilized for prokaryotic cells, whereas calcium phosphate treatment or electroporation may be used for other cellular hosts; see Sambrook, supra.

In a still further embodiment, the present invention relates to a cell containing the polynucleotide or vector described above. Preferably, said cell is a eukaryotic, most preferably a mammalian cell if therapeutic uses of the protein are envisaged. Of course, yeast and less preferred prokaryotic, e.g., bacterial cells may serve as well, in particular if the produced protein is used as a diagnostic means. The polynucleotide or vector of the invention, which is present in the host cell, may either be integrated into the genome of the host cell or it may be maintained extrachromosomally.

The term "prokaryotic" is meant to include all bacteria which can be transformed or transfected with a DNA or RNA molecules for the expression of a protein of the invention. Prokaryotic hosts may include gram negative as well as gram positive bacteria such as, for example, E. coli, S. typhimurium, Serratia marcescens and Bacillus subtilis. The term "eukaryotic" is meant to include yeast, higher plant, insect and preferably mammalian cells. Depending upon the host employed in a recombinant production procedure, the protein encoded by the polynucleotide of the present invention may be glycosylated or may be non-glycosylated. TZap7/A, TZap7/B and TZap7 proteins of the invention may also include an initial methionine amino acid residue. A polynucleotide of the invention can be used to transform or transfect the host using any of the techniques commonly known to those of ordinary skill in the art. Furthermore, methods for preparing fused, operably linked genes and expressing them in, e.g., mammalian cells and bacteria are well-known in the art (Sambrook, Molecular Cloning: A Laboratory Manual, Cold Spring Harbor Laboratory, Cold Spring Harbor, NY, 1989). The TZap7/A, TZap7/B and TZap7 proteins of the invention can be produced by recombinant DNA technology in eukaryotic or prokaryotic hosts. In general, expression vectors containing promoter sequences which facilitate the efficient transcription of the inserted polynucleotide are used in connection with the host. The expression vector typically contains an origin of replication, a promoter, and a terminator, as well as specific genes which are capable of providing phenotypic selection of the transformed cells. Furthermore, transgenic animals, preferably mammals, comprising cells of the invention may be used for the large scale production of the TZap7/A, TZap7/B and TZap7 proteins of the invention.

Alternatively, an animal, preferably mammalian, cell naturally having a polynucleotide of the invention present in its genome can be used and modified such that said cell expresses the endogenous gene corresponding to the polynucleotide of the invention under the control of an heterologous promoter. The introduction of the heterologous promoter which does not naturally control the expression of the polynucleotide of the invention can be done according to standard methods, see supra. Suitable promoters include those mentioned hereinbefore.

Thus, in a further embodiment, the present invention relates to a method for the production of a protein comprising:

(a) culturing a host of the invention under conditions allowing for the expression of the protein; or
(b) in vitro translation of the polynucleotide of the invention; and recovering the protein produced in (a) or (b).

The transformed hosts can be grown in fermentors and cultured according to techniques known in the art to achieve optimal cell growth. The TZap protein of the invention can then be isolated from the growth medium, cellular lysates, or cellular membrane fractions. Once expressed, the protein of the present invention can be purified according to standard procedures of the art, including ammonium sulfate precipitation, affinity columns, column chromatography, gel electrophoresis and the like; see, Scopes, "Protein Purification", Springer-Verlag, N.Y. (1982). Substantially pure proteins of at least about 90 to 95% homogeneity are preferred, and 98 to 99% or more homogeneity are most preferred, for pharmaceutical uses. Once purified, partially or to homogeneity as desired, the proteins may then be used therapeutically (including extracorporeally) or in developing and performing assay procedures.

Hence, in a still further embodiment, the present invention relates to a protein encoded by the polynucleotide of the invention or produced by a method of as described above. It will be apparent to those skilled in the art that the protein of the invention can be further coupled to other moieties as described above for, e.g., drug targeting and imaging applications. Such coupling may be conducted chemically after expression of the protein to site of attachment or the coupling product may be engineered into the protein of the invention at the DNA level. The DNAs are then expressed in a suitable host system, and the expressed proteins are collected and renatured, if necessary. Furthermore, the provision of the TZap7/A, TZap7/B and TZap7 proteins of the present invention enables the production of TZap specific antibodies. In this respect, hybridoma technology enables production of cell lines secreting antibody to essentially any desired substance that produces an immune response. RNA encoding the light and heavy chains of the immunoglobulin can then be obtained from the cytoplasm of the hybridoma. The 5' end portion of the RNA can be used to prepare cDNA to be inserted into an expression vector. The DNA encoding the antibody or its immunoglobulin chains can subsequently be expressed in cells, preferably mammalian cells.

Depending on the host cell, renaturation techniques may be required to attain proper conformation of the antibody. If necessary, point substitutions seeking to optimize binding may be made in the DNA using conventional cassette mutagenesis or other protein engineering methodology such as is disclosed herein.

Thus, the present invention also relates to an antibody specifically recognizing the peptide or polypeptide of the invention.

In a still further embodiment, the present invention relates to a cell that has been modified to express a TZap protein or an antibody of the invention. This embodiment may be well suited for, e.g., restoring B and/or T cell responsiveness to an antigen, in particular if the antibody of the invention capable of stimulating T cell proliferation is expressed in a form suitable to be presented on the cell surface.

Moreover, the present invention relates to pharmaceutical compositions comprising any one of the above described polynucleotides, vectors, cells, proteins and/or antibodies and/or a ligand capable of binding to the TZap7/A, TZap7/B and/or TZap7 protein of the invention.

The antibody or ligand comprised in the pharmaceutical composition of the invention preferably has a specificity at least substantially identical to the binding specificity of the natural ligand of the TZap7/A, TZap7/B and/or TZap7 protein of the invention. Such an antibody or ligand can have a binding affinity of at least $10^5M^{-1}$, preferably higher than $10^7M^{-1}$ if leukocyte stimulation is envisaged and advantageously up to $10^{10}M^{-1}$ in case leukocyte suppression should be mediated.

In a preferred embodiment of the invention, said antibody is a monoclonal antibody, a polyclonal antibody, a single chain antibody, humanized antibody, or fragment thereof that specifically binds said peptide or polypeptide also including bispecific antibody, synthetic antibody, antibody fragment, such as Fab, Fv or scFv fragments etc., or a chemically modified derivative of any of these. Monoclonal antibodies can be prepared, for example, by the techniques as originally described in Köhler and Milstein, Nature 256 (1975), 495, and Galfré, Meth. Enzymol. 73 (1981), 3, which comprise the fusion of mouse myeloma cells to spleen cells derived from immunized mammals with modifications developed by the art. Furthermore, antibodies or fragments thereof to the aforementioned peptides can be obtained by using methods which are described, e.g., in Harlow and Lane "Antibodies, A Laboratory Manual", CSH Press, Cold Spring Harbor, 1988. When derivatives of said antibodies are obtained by the phage display technique, surface plasmon resonance as employed in the BIAcore system can be used to increase the efficiency of phage antibodies which bind to an epitope of the peptide or polypeptide of the invention (Schier, Human Antibodies Hybridomas 7 (1996), 97-105; Malmborg, J. Immunol. Methods 183 (1995), 7-13). The production of chimeric antibodies is described, for example, in WO89/09622. Methods for the production of humanized antibodies are described in, e.g., EP-A1 0 239 400 and WO90/07861. A further source of antibodies to be utilized in accordance with the present invention are so-called xenogenic antibodies. The general principle for the production of xenogenic antibodies such as human antibodies in mice is described in, e.g., WO 91/10741, WO 94/02602, WO 96/34096 and WO 96/33735. Antibodies to be employed in accordance with the invention or their corresponding immunoglobulin chain(s) can be further modified using conventional techniques known in the art, for example, by using amino acid deletion(s), insertion(s), substitution(s), addition(s), and/or recombination(s) and/or any other modification(s) known in the art either alone or in combination. Methods for introducing such modifications in the DNA sequence underlying the amino acid sequence of an immunoglobulin chain are well known to the person skilled in the art; see, e.g., Sambrook, Molecular Cloning A Laboratory Manual, Cold Spring Harbor Laboratory (1989) N.Y.

The pharmaceutical composition of the present invention may further comprise a pharmaceutically acceptable carrier. Examples of suitable pharmaceutical carriers are well known in the art and include phosphate buffered saline solutions, water, emulsions, such as oil/water emulsions, various types of wetting agents, sterile solutions etc. Compositions comprising such carriers can be formulated by well-known conventional methods. These pharmaceutical compositions can be administered to the subject at a suitable dose. Administration of the suitable compositions may be effected by different ways, e.g., by intravenous, intraperitoneal, subcutaneous, intramuscular, topical or intradermal administration. The dosage regimen will be determined by the attending physician and clinical factors. As is well known in the medical arts, dosages for any one patient depends upon many factors, including the patient's size, body surface area, age, the particular compound to be administered, sex, time and route of administration, general health, and other drugs being administered concurrently. A typical dose can be, for example, in the range of 0.001 to 1000 μg (or of nucleic acid for expression or for inhibition of expression in this range); however, doses below or above this exemplary range are envisioned, especially considering the aforementioned factors. Generally, the regimen as a regular administration of the pharmaceutical composition should be in the range of 1 μg to 10 mg units per day. If the regimen is a continuous infusion, it should also be in the range of 1 μg to 10 mg units per kilogram of body weight per minute, respectively. Progress can be monitored by periodic assessment. Dosages will vary but a preferred dosage for intravenous administration of DNA is from approximately $10^6$ to $10^{12}$ copies of the DNA molecule. The compositions of the invention may be administered locally or systemically. Administration will generally be parenterally, e.g., intravenously; DNA may also be administered directly to the target site, e.g., by biolistic delivery to an internal or external target site or by catheter to a site in an artery. Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Preservatives and other additives may also be present such as, for example, antimicrobials, anti-oxidants, chelating agents, and inert gases and the like. Furthermore, the pharmaceutical composition of the invention may comprise further agents such as T cell costimulatory molecules or cytokines known in the art, or their inhibitors or activators depending on the intended use of the pharmaceutical composition.

Furthermore, it is envisaged by the present invention that the various polynucleotides and vectors encoding the above described peptides or polypeptides are administered either alone or in any combination using standard vectors and/or gene delivery systems, and optionally together with a pharmaceutically acceptable carrier or excipient. For example, the polynucleotide of the invention can be used alone or as part of a vector to express the (poly)peptide of the invention in cells, for, e.g., gene therapy or diagnostics of diseases related to disorders of the immune system. The polynucleotides or vectors of the invention are introduced into the cells which in turn produce the (poly)peptide. Subsequent to administration, said polynucleotides or vectors may be stably integrated into the genome of the subject. On the other hand, viral vectors may be used which are specific for certain cells or tissues and persist in said cells. Suitable pharmaceutical carriers and excipients are well known in the art. The pharmaceutical compositions prepared according to the invention can be used for the prevention or treatment or delaying of different kinds of diseases, which are related to lymphocyte and/or monocyte related immunodeficiencies and malignancies.

In another embodiment the present invention relates to a diagnostic composition comprising any one of the above described proteins, antibodies, (poly)peptides, polynucleotides, vectors or cells, and optionally suitable means for detection. The (poly)peptides and antibodies described above are, for example, suited for use in immunoassays in which they can be utilized in liquid phase or bound to a solid phase carrier. Examples of immunoassays which can utilize said (poly)peptides are competitive and non-competitive immunoassays in either a direct or indirect format. Examples of such immunoassays are the radioimmunoassay (RIA), the sandwich (immunometric assay) and the Western blot assay. The (poly)peptides and antibodies can be bound to many different carriers and used to isolate cells specifically bound to said polypeptides. Examples of well-known carriers include glass, polystyrene, polyvinyl chloride, polypropylene, polyethylene, polycarbonate, dextran, nylon, amyloses, natural and modified celluloses, polyacrylamides, agaroses, and magnetite. The nature of the carrier can be either soluble or insoluble for the purposes of the invention.

There are many different labels and methods of labeling known to those of ordinary skill in the art. Examples of the types of labels that can be used in the present invention include enzymes, radioisotopes, colloidal metals, fluorescent compounds, chemiluminescent compounds, and bioluminescent compounds.

Said diagnostic compositions may also be used for methods for detecting expression of a polynucleotide of the invention by detecting the presence of mRNA coding for a TZap protein which comprises obtaining mRNA from a cell and contacting the mRNA so obtained with a probe comprising a nucleic acid molecule of at least 15 nucleotides capable of specifically hybridizing with a polynucleotide of the invention under suitable hybridizing conditions (see also supra), detecting the presence of mRNA hybridized to the probe, and thereby detecting the expression of the TZap protein by the cell.

Furthermore, the invention comprises methods of detecting the presence of a TZap protein in a sample, for example, a cell sample, which comprises obtaining a cell sample from a subject, contacting said sample with one of the aforementioned antibodies under conditions permitting binding of the antibody to the TZap protein, and detecting the presence of the antibody so bound, for example, using immunoassay techniques such as radioimmunoassay or enzyme-immunoassay. Furthermore, one skilled in the art may specifically detect and distinguish polypeptides which are functional TZap proteins from mutated forms which have lost or altered their immunomodulating activity by using an antibody which either specifically recognizes a (poly)peptide which has TZap7/A, TZap7/B and/or TZap7 activity but does not recognize an inactive form thereof or which specifically recognizes an inactive form but not the corresponding polypeptide having TZap7/A, TZap7/B and/or TZap7 activity. The antibodies of the present invention may also be used in affinity chromatography for purifying the TZap proteins or above described (poly)peptides and isolating them from various sources.

The invention also encompasses a method for diagnosing in a subject a predisposition to a disorder associated with the expression of a TZap7/A, TZap7/B and/or TZap7 allele which comprises isolating DNA from victims of the disorder associated with the under-or over-expression of a TZap7/A, TZap7/B and/or TZap7 protein; digesting the isolated DNA with at least one restriction enzyme; electrophoretically separating the resulting DNA fragments on a sizing gel; contacting the resulting gel with a nucleic acid probe as described above capable of specifically hybridizing to DNA encoding a TZap protein and labeled with a detectable marker; detecting labeled bands on the gel which have hybridized to the labeled probe to create a band pattern specific to the DNA of victims of the disorder associated with the expression of a TZap protein; preparing the subject's DNA according to the above-mentioned steps to produce detectable labeled bands on a gel; and comparing the band pattern specific to the DNA of victims of the disorder associated with the expression of a TZap protein and the subject's DNA to determine whether the patterns are the same or different and to diagnose thereby predisposition to the disorder if the patterns are the same.
The detectable markers of the present invention may be labeled with commonly employed radioactive labels, such as, for example, $^{32}P$ and $^{35}S$, although other labels such as biotin or mercury as well as those described above may be employed as well.

Various methods well-known to the person skilled in the art may be used to label the detectable markers. For example, DNA sequences and RNA sequences may be labeled with $^{32}P$ or $^{35}S$ using the random primer method. Once a suitable detectable marker has been obtained, various methods well-known to the person skilled in the art may be employed for contacting the detectable marker with the sample of interest. For example, DNA-DNA, RNA-RNA and DNA-RNA hybridizations may be performed using standard procedures. Various methods for the detection of nucleic acids are well-known in the art, e.g., Southern and northern blotting, PCR, primer extension and the like. Furthermore, the mRNA, cRNA, cDNA or genomic DNA obtained from the subject may be sequenced to identify mutations which may be characteristic fingerprints of TZap mutations in disorders associated with the expression of TZap or mutated versions thereof. The present invention further comprises methods, wherein such a fingerprint may be generated by RFLPs of DNA or RNA obtained from the subject, optionally the DNA or RNA may be amplified prior to analysis, the methods of which are well known in the art. RNA fingerprints may be performed by, for example, digesting an RNA sample obtained from the subject with a suitable RNA-Enzyme, for example RNase $T_1$, RNase $T_2$ or the like or a ribozyme and, for example, electrophoretically separating and detecting the RNA fragments on PAGE as described above or in the appended examples.

Advantageously, the pharmaceutical composition of the invention is intended for use in organ transplantation, for the treatment of autoimmune, allergic or infectious diseases, or for the treatment of tumors. An example for the use of the pharmaceutical composition of the invention for improving allograft or xenograft tolerance is described with respect to administration of an LFA-3 and CD2 binding protein, respectively, in WO93/06852.

In another embodiment, the present invention relates to a pharmaceutical composition comprising an agent which modulates immune response through the TZap7/A, TZap7/B and/or TZap7 protein of the invention, and optionally a pharmaceutically acceptable carrier. As is immediately evident to the person skilled in the art, the provision of the novel TZap7/A, TZap7/B and TZap7 proteins of the invention opens up the way of alternative approaches for modulating immune responses and treating corresponding diseases. The agent that, for example, stimulates the proliferation and/or differentiation of leukocytes through the TZap7/A, TZap7/B and/or TZap7 protein is expected to markedly enhance the proliferation of, e.g., (activated) T cells and thus is capable of augmenting the immune response. Examples for this type of "Vaccine" is described, e.g., in WO91/11194 and in the literature, e.g., referred to above. The agents to be employed in accordance with the present invention usually specifically bind to TZap7/A, TZap7/B and/or TZap7 protein in order to exert their effect. Such agents can be identified in accordance with a method of the invention described below. Such agents also comprise promoters which can be inserted in front of the coding region of the TZap protein encoding gene, e.g., via gene transfer and homologous recombination in the 5' untranslated region of the gene, see also supra. Such promoter may be regulated and thus permit the controlled expression of the TZap protein in certain cells.

In a further aspect the present invention relates to a method for identifying a binding partner to a protein comprising:

(a) contacting a protein of the invention with a compound to be screened; and
(b) determining whether the compound affects an activity of the polypeptide.

TZap7/A, TZap7/B and TZap7 polypeptides may be used to screen for molecules that bind to TZap7/A, TZap7/B and/or TZap7 or for molecules to which TZap7/A, TZap7/B and/or TZap7 binds. The binding of TZap7/A, TZap7/B and/or TZap7 and the molecule may activate (agonist), increase, inhibit (antagonist), or decrease activity of the TZap7/A, TZap7/B and/or TZap7 or the molecule bound. Examples of such molecules include antibodies, oligonucleotides, proteins (e.g., receptors), or small molecules.

Preferably, the molecule is closely related to the natural ligand of TZap, e.g., a fragment of the ligand, or a natural substrate, a ligand, a structural or functional mimetic; see, e.g., Coligan, Current Protocols in Immunology 1 (2) (1991); Chapter 5. Similarly, the molecule can be closely related to the natural receptor to which TZap7/A, TZap7/B and/or TZap7 binds, or at least, a fragment of the receptor capable of being bound by TZap7/A, TZap7/B and/or TZap7 (e.g., active site). In either case, the molecule can be rationally designed using known techniques; see also infra.

Preferably, the screening for these molecules involves producing appropriate cells which express TZap, either as a secreted protein or on the cell membrane. Preferred cells include cells from mammals, yeast, Drosophila, or E. coli. Cells expressing TZap (or cell membrane containing the expressed polypeptide) are then preferably contacted with a test compound potentially containing the molecule to observe binding, stimulation, or inhibition of activity of either TZap or the molecule.

The assay may simply test binding of a candidate compound to TZap, wherein binding is detected by a label, or in an assay involving competition with a labeled competitor. Further, the assay may test whether the candidate compound results in a signal generated by binding to TZap.

Alternatively, the assay can be carried out using cell-free preparations, polypeptide/molecule affixed to a solid support, chemical libraries, or natural product mixtures. The assay may also simply comprise the steps of mixing a candidate compound with a solution containing TZap7/A, TZap7/B and/or TZap7, measuring TZap/molecule activity or binding, and comparing the TZap/molecule activity or binding to a standard.
Preferably, an ELISA assay can measure TZap level or activity in a sample (e.g., biological sample) using a monoclonal or polyclonal antibody. The antibody can measure TZap level or activity by either binding, directly or indirectly, to TZap or by competing with TZap for a substrate.

All of these above assays can be used as diagnostic or prognostic markers. The molecules discovered using these assays can be used to treat disease or to bring about a particular result in a patient (e.g., increase of immune response) by activating or inhibiting the TZap/molecule. Moreover, the assays can discover agents which may inhibit or enhance the production of TZap from suitably manipulated cells or tissues.

Therefore, the invention includes a method of identifying compounds which bind to the protein of the invention comprising the steps of:

(a) incubating a candidate binding compound with the protein of the invention, and
(b) determining if binding has occurred.

Moreover, the invention includes a method of identifying modulators of immune responses comprising the steps of:

(a) incubating a candidate compound with the protein of the invention;
(b) assaying a biological activity as described above, and
(c) determining if a biological activity of the protein of the invention has been altered.

As mentioned hereinbefore, the polynucleotides and polypeptides of the present invention provide a basis for the development of mimetic compounds that may be modulators of TZap or their encoding genes. It will be appreciated that the present invention also provides cell based screening methods that allow a high-throughput-screening (HTS) of compounds that may be candidates for such modulators.

Furthermore, the invention relates to a method for identifying leukocyte activating or co-stimulating compounds or for identifying inhibitors of leukocyte activation and stimulation comprising

(a) culturing T cells in the presence of the protein of the invention, (poly)peptide, antibody, or cell described above and, optionally, in the presence of a component capable of providing a detectable signal in response to leukocyte activation, with a compound to be screened under conditions permitting interaction of the compound with the protein of the invention, (poly)peptide, antibody or cell(s); and
(b) detecting the presence or absence of a signal generated from the interaction of the compound with the cells.

The term "compound" in the method of the invention includes a single substance or a plurality of substances which may or may not be identical.
Said compound(s) may be comprised in, for example, samples, e.g., cell extracts from, e.g., plants, animals or microorganisms. Furthermore, said compounds may be known in the art but hitherto not known to be capable of inhibiting proliferation of leukocytes or not known to be useful as an immune response costimulatory or modulating factor, respectively. The plurality of compounds may be, e.g., added to a simple in vitro, to the culture medium or injected into the cell.
If a sample containing (a) compound(s) is identified in the method of the invention, then it is either possible to isolate the compound from the original sample identified as containing the compound in question, or one can further subdivide the original sample, for example, if it consists of a plurality of different compounds, so as to reduce the number of different substances per sample and repeat the method with the subdivisions of the original sample. It can then be determined whether said sample or compound displays the desired properties by methods known in the art such as described herein and in the appended examples. Depending on the complexity of the samples, the steps described above can be performed several times, preferably until the sample identified according to the method of the invention only comprises a limited number of or only one substance(s). Preferably said sample comprises substances of similar chemical and/or physical properties, and most preferably said substances are identical. The methods of the present invention can be easily performed and designed by the person skilled in the art, for example in accordance with other cell based assays described in the prior art (see, e.g., EP-A-0 403 506) or by using and modifying the methods as described in the appended examples.
Furthermore, the person skilled in the art will readily recognize which further compounds and/or cells may be used in order to perform the methods of the invention, for example, B cells, interleukins, or enzymes, if necessary, that, e.g., convert a certain compound into the precursor which in turn stimulates or suppresses lymphocyte or monocyte activation or that provide for (co)stimulatory signals. Such adaptation of the method of the invention is well within the skill of the person skilled in the art and can be performed without undue experimentation.
Compounds which can be used in accordance with the method of the present invention include peptides, proteins, nucleic acids including cDNA expression libraries, antibodies, small organic compounds, ligands, peptidomimetics, PNAs and the like. Said compounds can also be functional derivatives or analogues of known B or T cell activators or inhibitors. Methods for the preparation of chemical derivatives and analogues are well known to those skilled in the art and are described in, for example, Beilstein, Handbook of Organic Chemistry, Springer edition New York Inc., 175 Fifth Avenue, New York, N.Y. 10010 U.S.A. and Organic Synthesis, Wiley, New York, USA. Furthermore, said derivatives and analogues can be tested for their effects according to methods known in the art or as described, for example, in the appended examples.
Furthermore, peptidomimetics and/or computer aided design of appropriate activators or inhibitors of T cell activation can be used, for example, according to the methods described below. Appropriate computer programs can be used for the identification of interactive sites of a putative inhibitor and the TZap protein by computer assistant searches for complementary structural motifs (Fassina, Immunomethods 5 (1994), 114-120). Further appropriate computer systems for the computer aided design of protein and peptides are described in the prior art, for example, in Berry, Biochem. Soc. Trans. 22 (1994), 1033-1036; Wodak, Ann. N. Y. Acad. Sci. 501 (1987), 1-13; Pabo, Biochemistry 25 (1986), 5987-5991. The results obtained from the above-described computer analysis can be used in combination with the method of the invention for, e.g., optimizing known leukocyte activators or inhibitors.
Appropriate peptidomimetics can also be identified by the synthesis of peptidomimetic combinatorial libraries through successive chemical modification and testing the resulting compounds, e.g., according to the methods described herein and in the appended examples. Methods for the generation and use of peptidomimetic combinatorial libraries are described in the prior art, for example in Ostresh, Methods in Enzymology 267 (1996), 220-234 and Dorner, Bioorg. Med. Chem. 4 (1996), 709-715. Furthermore, the three-dimensional and/or crystallographic structure of inhibitors or activators of leukocyte stimulation can be used for the design of peptidomimetic inhibitors or activators of leukocyte activation to be tested in the method of the invention (Rose, Biochemistry 35 (1996), 12933-12944; Rutenber, Bioorg. Med. Chem. 4 (1996), 1545-1558). Furthermore, ligands of Notch proteins described in Example 1 can be used as the starting material for any one of the above described methods.

In summary, the present invention provides methods for identifying compounds which are capable of modulating immune responses. Accordingly compounds identified in accordance with the method of the present invention to be inhibitors and activators, respectively, of immune response are also within the scope of the present invention.

Compounds found to enhance leukocyte proliferation may be used in the treatment of cancer or infections and related diseases. In addition, it may also be possible to specifically inhibit viral diseases, thereby preventing viral infection or viral spread. Compounds identified as suppressors of leukocyte proliferation can be used, e.g., for treating skin conditions (see, e.g., WO93/06866) or in allogenic or xenogenic cell or organ transplantation in order to avoid graft rejection; see also supra.

The compounds identified or obtained according to the method of the present invention are thus expected to be very useful in diagnostic and in particular for therapeutic applications.

Hence, in a further embodiment the invention relates to a method for the production of a pharmaceutical composition comprising formulating and optionally synthesizing the compound identified in step (b) or (c) of the above described methods of the invention in a pharmaceutically acceptable form. Hence, the present invention generally relates to a method of making a therapeutic agent comprising synthesizing the proteins, (poly)peptides, polynucleotides, vectors, antibodies or compounds according to the invention in an amount sufficient to provide said agent in a therapeutically effective amount to the patient. Methods for synthesizing these agents are well known in the art and are described, e.g. above.

The therapeutically useful compounds identified according to the method of the invention may be administered to a patient by any appropriate method for the particular compound, e.g., orally, intravenously, parenterally, transdermally, transmucosally, or by surgery or implantation (e.g., with the compound being in the form of a solid or semi-solid biologically compatible and resorbable matrix) at or near the site where the effect of the compound is desired. Therapeutic doses are determined to be appropriate by one skilled in the art, see also supra.

Such useful compounds can be, for example, transacting factors which bind to the TZap protein of the invention. Identification of transacting factors can be carried out using standard methods in the art (see, e.g., Sambrook, supra, and Ausubel, supra). To determine whether a protein binds to the TZap7/A, TZap7/B and/or TZap7 protein of the invention, standard native gel-shift analyses can be carried out. In order to identify a transacting factor which binds to the TZap7/A, TZap7/B and/or TZap7 of the invention, the polypeptides and peptides of the invention can be used as an affinity reagent in standard protein purification methods, or as a probe for screening an expression library. Once the transacting factor is identified, modulation of its binding to the TZap protein of the invention can be pursued, beginning with, for example, screening for inhibitors against the binding of the transacting factor to the TZap protein of the present invention. Activation or repression of TZap specific genes could then be achieved in subjects by applying the transacting factor (or its inhibitor) or the gene encoding it, e.g., in a vector described in the embodiments hereinbefore. In addition, if the active form of the transacting factor is a dimer, dominant-negative mutants of the transacting factor could be made in order to inhibit its activity. Furthermore, upon identification of the transacting factor, further components in the pathway leading to activation (e.g. signal transduction) or repression of a gene encoding the TZap protein of the present invention can then be identified. Modulation of the activities of these components can then be pursued, in order to develop additional drugs and methods for modulating the expression or activity of the TZap protein of the present invention.

Beside the above described possibilities to use the polynucleotides according to the invention for gene therapy and their use to identify homologous molecules, the described polynucleotides may also be used for several other applications, for example, for the identification of nucleic acid molecules which encode proteins which interact with the TZap7/A, TZap7/B and/or TZap7 protein described above. This can be achieved by assays well known in the art, for example, as described in Scofield (Science 274 (1996), 2063-2065) by use of the so-called yeast "two-hybrid system". In this system the (poly)peptide encoded by the polynucleotides according to the invention or a smaller part thereof is linked to the DNA-binding domain of the GAL4 transcription factor. A yeast strain expressing this fusion protein and comprising a lacZ reporter gene driven by an appropriate promoter, which is recognized by the GAL4 transcription factor, is transformed with a library of cDNAs which will express animal, preferably mammal proteins or peptides thereof fused to an activation domain. Thus, if a peptide encoded by one of the cDNAs is able to interact with the fusion protein comprising a (poly)peptide of the invention, the complex is able to direct expression of the reporter gene. In this way the polynucleotide according to the invention and the encoded peptide can be used to identify peptides and proteins interacting with TZap proteins.

Other methods for identifying compounds which interact with the TZap protein according to the invention or nucleic acid molecules encoding such molecules are, for example, the in vitro screening with the phage display system as well as filter binding assays or "real time" measuring of interaction using, for example, the BIAcore apparatus (Pharmacia); see references cited supra.

Furthermore, the present invention relates to the use of the polynucleotide, the nucleic acid molecule, the vectors, peptides, polypeptides, antibodies and cells of the invention as well as compounds identified in accordance with a method of the invention described hereinabove for the preparation of a composition for diagnosing and/or the treatment of diseases involving T cell activation and associated with Th1 and Th2 immune response, for the treatment of acute and chronic rejection of allo-and xeno organ transplants and bone marrow transplantation, for the treatment of rheumatoid arthritis, lupus erythramatodes, multiple sclerosis, encephalitis, vasculitis, diabetes mellitus, pancreatitis, gastritis, thyroiditis, for the treatment of maligne disorders of T, B or NK cells, for the treatment of asthma, lepramatosis, Helicobacter pylori associated gastritis or for the treatment of skin tumors, adrenal tumors or lung tumors, wound healing, growth disorders, inflammatory and/or infectious diseases.

The polynucleotides, vectors, cells, proteins, (poly)peptides, antibodies, inhibitors, activators, pharmaceutical and diagnostic compositions, uses and methods of the invention can be used for the treatment of all kinds of diseases hitherto unknown as being related to or dependent on the modulation of TZap. The pharmaceutical compositions, methods and uses of the present invention may be desirably employed in humans, although animal treatment is also encompassed by the methods and uses described herein.

Further methods and uses that can be employed in accordance with the present invention are described in WO99/11782 the disclosure of which is hereby specifically incorporated. These and other embodiments are disclosed and encompassed by the description and Examples of the present invention. Further literature concerning any one of the antibodies, methods, uses and compounds to be employed in accordance with the present invention may be retrieved from public libraries and databases, using for example electronic devices. For example the public database "Medline" may be utilized which is available on the Internet, for example under http://www.ncbi.nlm.nih.gov/PubMed/medline.html. Further databases and addresses, such as http://www.ncbi.nlm.nih.gov/, http://www.infobiogen.fr/, http://www.fmi.ch/biologjr/research_tools.html, http://www.tigr.org/, are known to the person skilled in the art and can also be obtained using, e.g., http://www.lycos.com. An overview of patent information in biotechnology and a survey of relevant sources of patent information useful for retrospective searching and for current awareness is given in Berks, TIBTECH 12 (1994), 352-364.

Throughout this specification and claims, the word "comprise," or variations such as "comprises" or "comprising," will be understood to imply the inclusion of a stated integer or group of integers but not the exclusion of any other integer or group of integers.

A better understanding of the present invention and of its many advantages will be had from the following examples, given by way of illustration.

## EXAMPLES

### Isolation of cDNAs from alloactivated human T cell line encoding novel proteins which share homology with Notchless proteins in *Drosophila* and *Xenopus* as well as with proteins from the β-transducin family in human cells

To identify genes induced during the early phase of T cell activation in response to alloantigens, differential display analysis of mRNA expression was performed using an allostimulated human T cell line. Activation of human T cells was performed as follows. In accordance with the ethical standards as formulated in the Helsinki Declaration of 1975 peripheral blood was obtained from healthy volunteers. Lymphocytes (PBL) were isolated using standard Ficoll centrifugation and resuspended in RPMI containing 10% fetal calf serum. Responder PBL were stimulated with equal numbers of irradiated (3000 rad, 13 min) stimulator lymphocytes. To establish an alloactivated human T cell line, cells were co-cultured for 24 h in tissue flasks at an initial concentration of $10^6$ cells/ml. Responding human T cells were restimulated three times with the irradiated stimulator lymphocytes from the same volunteer at 10 day intervals. The third restimulation was carried out with 5 x $10^6$ cells/ml irradiated stimulator cells, and total RNA was isolated immediately and 24 h after co-culturing. The RNA expression pattern was analyzed immediately and 24 h after the third allostimulation. Differential display-reverse transcription PCR (DDRT-PCR) analysis was then performed. Total RNA was isolated from lymphocytes immediately and 24 h after co-culturing using RNAzol B (Tel-Test) and differential display was performed as described previously [10]. Briefly, 2 μg of total RNA was used for reverse transcription with an oligo d(T) primer (25 nM) and 200 U MMLV reverse transcriptase (Gibco BRL). 50 ng of the resulting cDNA was used for subsequent PCR with primers 5'-GATGCCACCATGG-3' (SEQ ID NO: 7) and 5'-TGCGTCTGGTTCT-3' (SEQ ID NO: 8) artificial DNA. PCR was performed in a 20 μl reaction containing 1.25 mM MgCl2, 50 mM KCl, 10 mM Tris HCl, pH 8.3, 20 nM dNTP, 2.5 nM of each primer, 5 μCi 35S[dATP], and 0.3 U Taq polymerase (Promega). PCR products were separated by electrophoresis in a 6% polyacrylamid-urea gel (Sequagel, National Diagnostics) and subjected to autoradiography.

Using the primer pair given above, differential display RT-PCR revealed a single 230 bp cDNA fragment, which was significantly increased 24 h after stimulation.

To obtain a full length cDNA, this fragment was used to screen a human leukocyte cDNA in λ Trip1 Ex. The differentially expressed cDNA fragment was excised from the filter, eluted in 0.5 M ammonium acetate/1 mM EDTA, pH 8.3, and ethanol precipitated. The cDNA product was reamplified, electrophoresed in a 2% agarose gel and purified using Gene Clean kit (Quiagen). The recovered cDNA product was blunt-ended with Klenow enzyme (Gibco BRL) following standard protocols [11] and ligated into pBluescript $SK^+$ vector. After labeling with $\alpha^{32}P$[dCTP] (800 mCi/mmol, Amersham Inc.) using the random priming method [12], the cDNA fragment isolated from DDRT-PCR was used as a probe to screen a human leukocyte cDNA in λ Trip1 Ex. Hybridization was carried out for 24 h at 42 C in 40% formamide, 10% Dextran sulfate, 4 x SSC (1 x SSC consists of 150 mM NaCl, 15 mM sodium citrate, pH 7.0), 0.8 x Denhardt's solution (1 x Denhardt's solution contains 0.02% polyvinylpyrrolidone, 0.02% Ficoll, 0.02% bovine serum albumin), 0.5% sodium dodecyl sulfate (SDS), and 20 μg/ml salmon sperm DNA. The filters were washed twice for 20 min with 2 x SSC/ 10% SDS at room temperature, and for 30 min with 0.2 x SSC/10% SDS at 65 C, followed by autoradiography. From 2 x $10^6$ recombinants, two positive clones were plaque purified, subcloned into pBluescript $SK^+$ vector. Complete cDNA inserts were sequenced according to the method of Sanger [13], using a primer walking strategy starting from primers flanking the multiple cloning site of the plasmid. Sequence analysis was performed using Geneworks software system. For homology searches NCBI BLASTx and BLASTn software were used. Three clones, TZap7/A, TZap7/B and TZap7 were isolated and sequenced. Sequence analysis of TZap7/B revealed a 1928 bp cDNA (SEQ ID NO: 1) with an open reading frame of 1455 Nt, predicting a protein length of 485 amino acids (SEQ ID NO: 2).

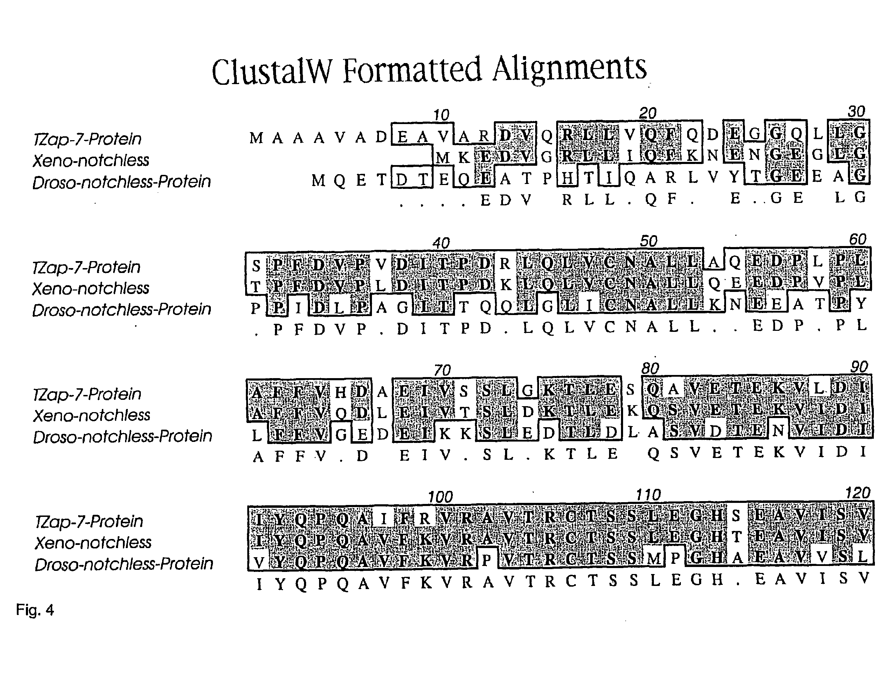

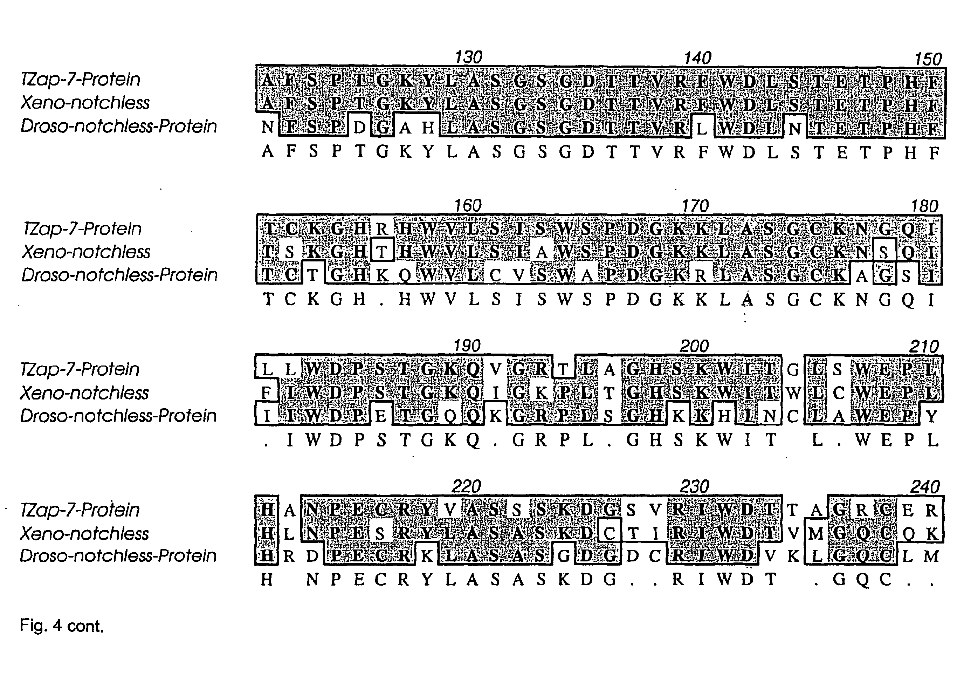

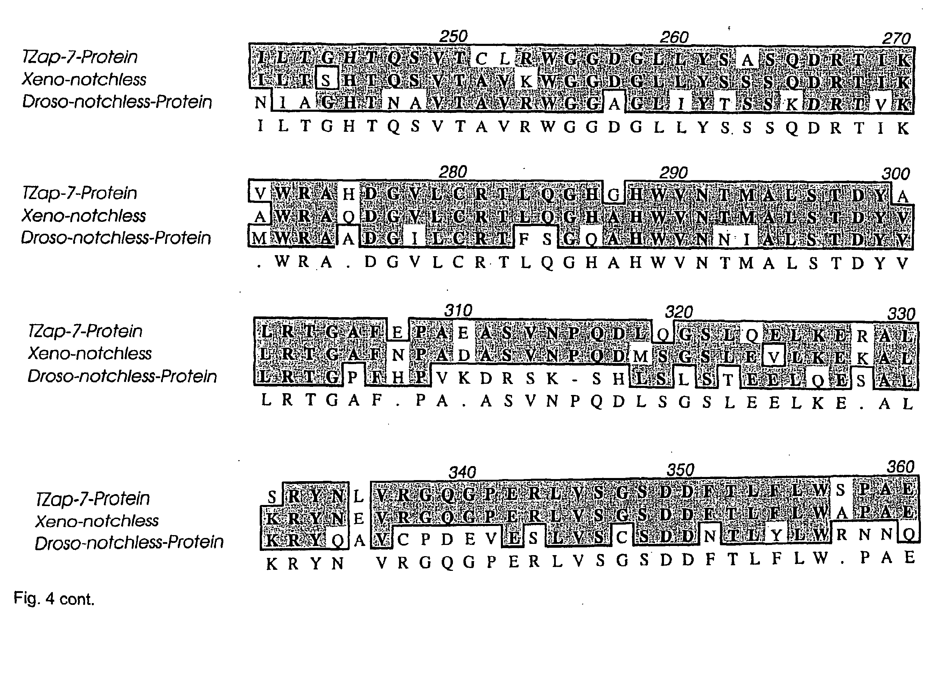

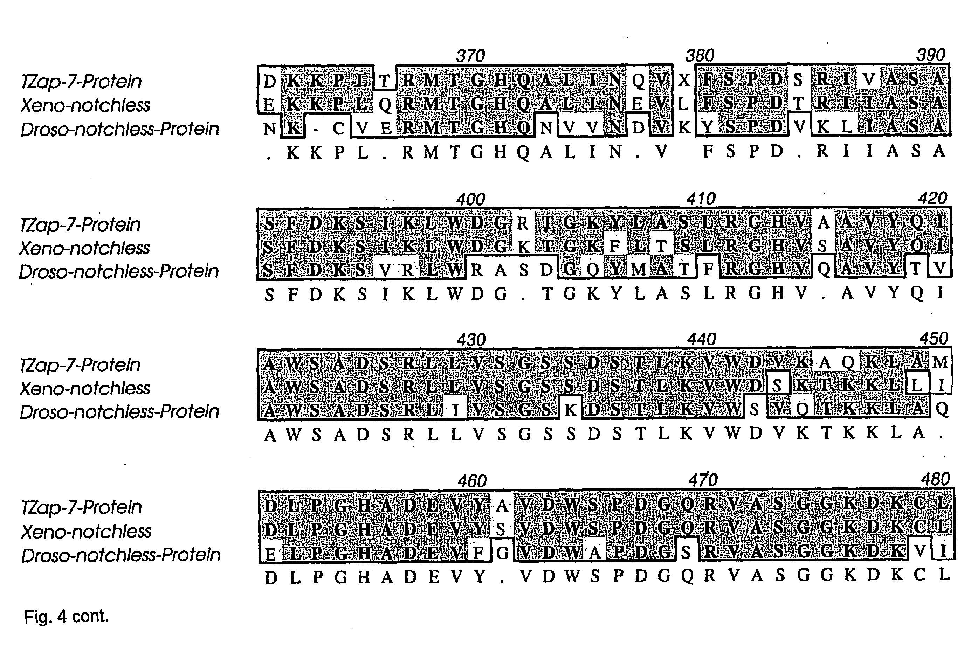

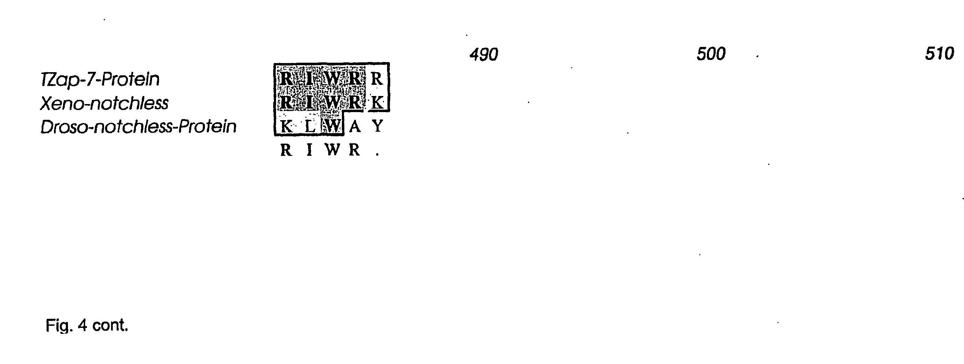

The putative translation product of TZap7/B shows extensive homology to Notchless proteins of *Drosophila* and *Xenopus.* Homologies are as high as 83% amino acids being identical between TZap and *Xenopus* Nle and 55% between TZap and *Drosophila* Nle. Sequencing of the second clone, designated TZap7/A, revealed a cDNA of 1170 bp (SEQ ID NO: 3) with an open reading frame of 786 Nt, predicting a protein length of 262 amino acids (SEQ ID NO: 4). The putative translation product of this clone also shows significant homologies to the Notchless proteins of *Drosophila* and *Xenopus.* Sequence identities ranging from 31% between *Drosophila* and TZap7/A and 45% between *Xenopus* and the human Nle. Furthermore, a third cDNA clone was obtained designated TZap7 with cDNA of 1,859 base pairs in length (SEQ ID NO: 5) encoding a protein of 458 amino acids in length (SEQ ID NO: 6). The amino acid sequence of TZap7 shows homology with notchless proteins similar to TZap7/B; see Figure 4. The relationship of the novel TZap proteins and Notch, Notchless and transducin proteins will be discussed below.

<u>Notch-signalling in differentiation</u>

During development, a cell's fate is determined by a type of signaling known as lateral inhibition or lateral specification (Artavanis-Tsakonas, S., Matsuno, K. and Fortini, M.E. (1995) Notch Signalling. Science, 268, 225-232). Such signals are transmitted between cells in direct contact with each other and direct the differentiation of distinct cell types emerging from a group of cells that have otherwise equivalent potential. This evolutionarily conserved pathway is mediated by the transmembrane receptor protein encoded by the *Notch* gene of *Drosophila* and its vertebrate homologues, as well as by related proteins that are encoded by the *lin-12* and *glp-1* genes of *C. elegans* (Artavanis-Tsakonas, S., Matsuno, K. and Fortini, M.E. (1995) Notch Signalling. Science, 268, 225-232).

One of the best understood examples of Notch participation in local intercellular communication is the decision between so-called AC and VU cell fates in the nematode *C. elegans* (Greenwald. I. and Rubin, G.M. (1992) Making a difference: the role of cell-cell interactions in establishing separate identities for equivalent cells. Cell, 68, 271-281).

Each of two cells that are in direct contact with each other has the capacity to become an anchor cell (AC), but stochastically only one adopts the AC fate while the other adopts the ventral uterine precursor (VU) fate. It is thought that when one cell chooses its fate, it sends a signal to its neighbor, inhibiting the neighbor from choosing the same fate and directing it to assume an alternative fate. Lateral signaling or notch signaling appears to guide the development of numerous structures in invertebrates such as the proper formation of the nervous system, the mesoderm and the germ line in embryonic *Drosophila* as well as the larval Malpighian tubules, adult sensory bristles and eye structures of the fly (reviewed in Artavanis-Tsakonas et al., 1995).

The role of lateral inhibition is best studied in invertebrates but recent studies indicate an important role of notch signaling in numerous cell fate decisions in mammals.

<u>Role of *Notch* family members in mammalian cell fate specification</u>

The role of *Notch* family members in mammals was explored both through gene overexpression and gene inactivation. In mice and man, each of which possess at least four different Notch proteins, chromosomal rearrangments that affect *Notch* genes are associated with certain neoplasias. (Ellisen, L.W., Bird, J., West, D.C., Soreng, A.L., Reynolds, T.C., Smith, S.D. and Sklar, J. (1991): Tan-1, the human homolog of the Drosophila notch gene, is broken by chromosomal translocations in T lymphoblastic neoplasms. Cell, 66, 649-661). The vertebrate *Notch* genes are expressed throughout developing tissues at embryonic stages and in proliferative cell layers of mature tissues. Mice defective for one of their *Notch* genes die before 11.5 days of gestation with extensive regions of cell death (Swiatek, P.J., Lindsell, C.E., del Amo, F.F., Weinmaster, G. and Gridley, T. (1994): Notch1 is essential for postimplantation development in mice. Genes & Development, 6, 707-719). These findings imply an essential function of Notch for the development in mammals.

That Notch is involved in mammalian cell fate determination was suggested by constitutive activation of Notch1 in immature thymocytes that resulted in a biased CD4 versus CD8 lineage decision in favor of CD8 T cells as well as in T cell receptor expression that was skewed toward α/β TCRs (Robey, E., Chang, D., Itano, A., Cado, D., Alexander, H.,

Lans, D., Weinmaster, G. and Salmon, P. (1996): An activated form of notch influences the choice between CD4 and CD8 T cell lineage. Cell, 87, 483-4.92). And even earlier in T cell lineage, it was shown that constitutive expression of Notch1 results in a emergence of a population of thymic-independent T cells in the bone marrow, concurrent with an early and persistent block in B cell maturation (Pui JC, Allman D, Xu L, DeRocco S, Karnell FG, Bakkour S, Lee JY, Kadesch T, Hardy RR, Aster JC, Pear WS (1999): Notch1 expression in early lymphopoiesis influences B versus T lineage determination. Immunity, 11, 299-308). These findings suggest that Notch1 plays an obligatory and selective role not only in T cell lineage induction but also in several cell fate decisions in T cell maturation.

Molecular structure of notch protein family members

Notch proteins are 300 kD single-pass transmembrane receptors. The large extracellular domain contains 34-36 tandem EGF-like repeats and three cystein-rich Notch/Lin-12 (NL) repeats. Six tandem ankyrin or Cdc10 repeats, a glutamin-rich domain (opa), and a PEST sequence are found within the intracellular domain (Wharton KA, Johansen KM, Xu T, Artavanis-Tsakonas S (1985): Nucleotide sequence from the neurogenic locus notch implies a gene product that shares homology with proteins containing EGF-like repeats. Cell, 43, 567581). The intracellular domain is divided in the subdomains ICN1 and ICN2 with ICN1 containing the Cdc10 repeats (Pear, W.S., Aster, J.C., Hasserjian, R.B., Soffer, B., Sklar and Baltimore, D. (1996): Exclusive development of T cell neoplasms in mice transplanted with bone marrow expressing activated Notch alleles. Journal of Experimental Medicine, 183, 2283-2291).

Notch ligands and signaling pathways

Genetic and molecular interaction studies resulted in the identification of a number of proteins that participate in transmitting and regulating Notch signal. In *Drosophila*, the two single -pass transmembrane proteins, Delta and Serrate (Delta and Jagged in vertebrates), have been identified as partially redundant extracellular Notch ligands (Artavanis-Tsakonas, S. Rand, M.D. and Lake, R.J. (1999): Notch signaling: cell fate control and signal integration in development. Science, 284, 770-776). The extracellular domain of these ligands, expressed on the surface of one cell, are thought to interact with the extracellular domain of the Notch protein expressed on a adjacent cell, resulting in the activation of the intra cellular notch domain. As a result of activation the intracellular domain is cleaved by Presenilin, a multiple transmembrane protein, (Struhl and Greenwald, 1999) and bind to different transcription factors such as Suppressor of Hairless ( Su(H)) in *Drosophila* (CBF1/RJBκ in mammals). Upon binding the Notch/Su(H) complex is translocated to the nucleus and upregulate the expression of the genes of the Enhancer of split Locus, which encode nuclear basic helix-loop-helix (bHLH) proteins (Bailey, A.M. and Posakony, J.W. (1995): Suppressor of hairless directly activates transcription of Enhancer of split complex genes in response to notch receptor activity. Genes & Development, 9, 2609-2622). The bHLH proteins, in turn, affect the regulation of downstream target genes, for instance, the genes of the Achaete-Scute complex in *Drosophila,* which contains proneuronal genes that encode proteins involved in the segregation of neuronal and epidermal lineages.

Beside of CBF1 and RJBκ several other proteins reported to interact with the intracellular notch domain are e. g. Bcl3, a member of the IκB family (Jehn BM, Bielke W, Pear WS, Osborne BA (1999): Protective effects of notch-1 on TCR-induced apoptosis. J Immunol, 162, 635-638), Nur77, a protein involved in lymphoid development, Deltex, a cytoplasmic protein with putative SRC homology binding domains and Notchless, a WD 40-repeat containing protein (Royet, J., Bouwmeester, T. and Cohen, S.M. (1998): Notchless encodes a novel WD40-repeat-containing protein that modulates Notch signaling activity. The EMBO Journal, 17, 7351-7360).

Notchless modulates Notch signaling activity

Several proteins have been identified as modifiers of the activity of Notch-family receptors. Deltex binds to the CDC10 repeats and positively regulates notch activity (Matsuno K, Eastman D, Mitsiades T, Quinn AM, Carcanciu ML, Ordentlich P, Kadesch T, Artavanis-Tsakonas S (1995) Human deltex is a conserved regulator of Notch signalling. Nature Genetics, 19, 74-78), Numb, Disheveled and Sel-10 binding reduce Notch activity. A novel protein, which reduces Notch activity by binding the intracellular ICN2 domain, is Notchless a novel WD40 repeat containing protein (Royet, J., Bouwmeester, T. and Cohen, S.M. (1998): Notchless encodes a novel WD40-repeat-containing protein that modulates Notch signaling activity. The EMBO Journal, 17, 7351-7360).

Notchless (Nle) was first identified in a genetic screen for modifiers of *Drosophila* notch activity. Removing one copy of Notchless in Deltex mutant flies restores the deltex mutant wing to normal. This result suggests that Deltex and Notchless act in opposite directions as modifiers of notch activity in *Drosophila* wing development. Binding of Nle to the cytoplasmatic domain of Notch was confirmed by GST pull-down and immunoprecipitation assays. Experiments using the yeast two-hybrid system that Nle binds to the ICN2 domain of notch, but not to ICN1. This suggests that Nle is likely to oppose Deltex function indirectly through an opposing activity on Notch, and not by direct competition for binding.

The function of Notchless appears to be to reduce Notch activity. Reduction or removal of Nle expression increase Notch activity, but overexpression of Nle also leads to increased Notch activity in *Drosophila* and *Xenopus.* It is thought that Nle functions as a modulator to keep Notch activity levels in balance. Nle mutants show increases Notch activity but are viable even as homozygotes, indicating that the level of overactivation is not so severe as to be lethal. In this regard Nle functions like Deltex, which modulates the level of Notch activity, but which is not absolutely required for Notch to function.

Using degenerated PCR Primer directed against the N-terminal domain of mouse and human ESTs a *Xenopus* cDNA was isolated. Both predicted Notchless proteins has novel highly conserved N-terminal domains followed by nine WD40 repeats. The WD40 repeat is found in a wide variety of proteins of diverse function and is thought to be a protein interaction domain (Neer EJ, Schmidt CJ, Nambudripad R, Smith TF (1994): The ancient regulatory-protein family of WD-repeat proteins. Nature, 371, 297-300). Typically WD40 proteins contain seven repeats. Structure analysis of β-Transducin suggests that these form a propeller-like structure and that seven repeats can pack to make a flat cylinder (Neer EJ and Smith TF (1996) G protein heterodimers: new structures propel new questions. Cell, 84, 175-178). Notchless is unusual in that it appears to contain nine WD40 repeats.

**References**

[1] G.R. Crabtree, Contingent genetic regulatory events in T lymphocyte activation, Science 243 (1989) 355-361.

[2] C.H. June, Signal transduction in T cells, Curr. Opin. Immunol. 3 (1991) 287-293.

[3] R. H. Schwartz, Costimulation of T lymphocytes: the role of CD28, CTLA-4, and B7/BB1 in Interleukin-2 production and immunotherapy, Cell 71 (1992) 1065-1068.

[4] J. Banchereau, F. Bazan, D. Blanchard, F. Briere, J. Galizzi, C. van Kooten, Y. Liu, F. Rousset, S. Seeland, The CD40 antigen and its ligand, Annu. Rev. Immunol. 12 (1994) 881-922.

[5] D.J. Lenschow, T. Walunas, J. Bluestone, CD28/B7 system of T cell costimulation, Annu. Rev. Immunol. 14 (1996) 233-258.

[6] P. Linsley, J. Ledbetter, The role of the CD28 receptor during T cell responses to antigen, Annu. Rev. Immunol. 11 (1993) 191-212.

[7] A. Kupfer, S.L. Swain, S.J. Singer, The specific direct interaction of helper T cells and antigen-presenting B cells. II. Reorientation of the microtubule organizing center and reorganization of the membrane-associated cytoskeleton inside the bound helper T cells, J. Exp. Med. 165 (1987) 1565-1580.

[8] M.V. Parsey, G.K. Lewis, Actin polymerization and pseudopod reorganization accompany anti-CD3-induced growth arrest in Jurkat T cells, J. Immunol. 151 (1993) 1881-1893.

[9] N. Selliah, W.H. Brooks, T.L. Roszman, Proteolytic cleavage of -actinin by calpain in T cells stimulated with anti-CD3 monoclonal antibody, J. Immunol. 156 (1996) 3215-3221.

[10] R. Kojima, J. Randall, B.M. Brenner, S.R. Gullans, Osmotic stress protein 94 (Osp94): A new member of the Hsp110/SSE gene subfamily, J. Biol. Chem. 271 (1996) 12327-12332.

[11] J. Sambrook, E.F. Fritsch, T. Maniatis, Molecular Cloning: A Laboratory Manual, Cold Spring Harbour, New York, 1989.

[12] A.P. Feinberg, B. Vogelstein, A technique for radiolabeling DNA restriction endonuclease fragments to high specific activity, Anal. Biochem. 132 (1983) 6-13.

[13] F. Sanger, S. Nicklen, A.R. Coulson, DNA sequencing with chain-terminating inhibitors, Proc. Natl. Acad. Sci. USA 74 (1977) 5463-5467.

[14] M. Ashburner, P. Thompson, J. Roote, P.F. Lasko, Y. Grau, M. El Messal, S. Roth, P. Simpson, The genetics of a small autosomal region of Drosophila melanogaster containing the structural gene for alcohol dehydrogenase. Characterization of the region around the snail and cactus loci, Genetics 126 (1990) 679-694.

[15] S. Roth, F.S. Neuman-Silberberg, G. Barcelo, T. Schüpbach, Cornichon and the EGF receptor signaling process are necessary for both anterior-posterior and dorsal-ventral pattern formation in Drosophila, Cell 81 (1995) 967-978.

[16] R. Lehmann, Establishment of embryonic Drosophila oogenesis, Dev. Biol. 6 (1995) 25-38.

[17] F.S. Neuman-Silberberg, T. Schüpbach, The Drosophila dorsoventral patterning gene gurken produces a dorsally localized RNA and encodes a TGF-like protein, Cell 75 (1993) 165-174.

[18] J.V. Price, R.J. Clifford, T. Schüpbach, The maternal ventralizing locus torpedo is allelic to faint little ball, an embryogenic lethal, and encodes the Drosophila EGF receptor homolog, Cell 56 (1998) 1085-1092.

[19] J. Schlessinger, B. Geiger, Epidermal growth factor induces redistribution of actin and alpha-actinin in human epidermal carcinoma cells, Exp. Cell. Res. 134 (1981) 273-279.

[20] J. Singer, Intercellular communication and cell-cell adhesion, Science 255 (1992) 1671-1677.

[21] R. Pardi, L. Inverardi, C. Rugarli, J.R. Bender, Antigen-receptor complex stimulation triggers protein kinase C-dependent CD11a/CD18-cytoskeleton association in T lymphocytes, J. Cell. Biol. 116 (1992) 1211-1220.

[22] Pearson (1990). Methods in Enzymology, 183, pp. 63-98.

## Claims

1. A polynucleotide encoding a protein consisting of a DNA sequence selected from the group consisting of

    (i) DNA sequences comprising a nucleotide sequence encoding the amino acid sequence depicted in SEQ ID NO: 2, 4 or 6;

(ii) DNA sequences comprising the nucleotide sequence depicted in SEQ ID NO: 1, 3 or 5;
(iii) DNA sequences the complementary strand of which hybridizes with and which is at least 90% identical to the polynucleotide as defined in (i) or (ii);
(iv) DNA sequences the nucleotide of which is degenerate to the nucleotide sequence of a DNA sequence of any one of (i) to (iii); and
(v) DNA sequences comprising a nucleotide sequence encoding a consensus sequence of the amino acid sequences depicted in SEQ ID NOs: 2, 4 and 6.

**2.** Use of a nucleic acid molecule of at least 15 nucleotides in length hybridizing specifically at stringent conditions for at least 10 hours in 50%formamide/6xSSC/0.1%SDS at 42°C or in 6xSSC at 68°C with the polynucleotide of claim 1 or the complementary strand thereof for the detection and/or amplification of a polynucleotide of claim 1 or for repression or targeting of a gene comprising a polynucleotide of claim 1.

**3.** A vector comprising the polynucleotide of claim 1.

**4.** The vector of claim 3, wherein said polynucleotide is operably linked to regulatory sequences allowing for the transcription and optionally expression of said polynucleotide.

**5.** A host cell comprising a polynucleotide of claim 1 or the vector of claim 3 or 4.

**6.** A method for the production of a protein comprising:

(a) culturing the host of claim 5 under conditions allowing for the expression of the protein; or
(b) *in vitro* translation of the polynucleotide of claim 1;

and recovering the protein produced in (a) or (b).

**7.** A protein encoded by the nucleic molecule of claim 1 or produced by the method of claim 6.

**8.** An antibody specifically recognizing the protein of claim 7.

**9.** A cell that has been modified to express the protein of claim 7 or the antibody of claim 8.

**10.** A pharmaceutical composition comprising the polynucleotide of claim 1, the nucleic acid molecule as defined in claim 2, a vector of claim 3 or 4, the cell of claim 5, the protein of claim 7, the antibody of claim 8 or an antisense construct capable of inhibiting the expression of a polynucleotide of claim 1, and optionally a pharmaceutically acceptable carrier.

**11.** The pharmaceutical composition of claim 10 for use in cell or organ transplantation, for the treatment of autoimmune, allergic or infectious diseases, or for the treatment of tumors.

**12.** A diagnostic composition comprising a polynucleotide of claim 1, the nucleic acid molecule as defined in claim 2, the vector of claim 3 or 4, the cell of claim 5 or 9, the protein of claim 7, or the antibody of claim 8; and optionally, at least one component which is labeled.

**13.** An *in vitro* method of diagnosing a pathological condition or a susceptibility to a pathological condition in a subject related to a disorder in the immune system comprising:

(a) determining the presence or absence of a mutation in the polynucleotide of claim 1 in a sample; and
(b) diagnosing a pathological condition or a susceptibility to a pathological condition based on the presence or absence of said mutation.

**14.** A method of diagnosing a pathological condition or a susceptibility to a pathological condition in a subject related to a disorder in the immune system comprising:

(a) determining the presence or amount of expression of the protein of claim 7 in a biological sample; and
(b) diagnosing a pathological condition or a susceptibility to a pathological condition based on the presence or amount of expression of the protein.

**15.** A method for identifying a binding partner to a protein comprising:

(a) contacting a protein of claim 7 with a compound to be screened; and
(b) determining whether the compound affects an activity of the protein.

**16.** A method for identifying leukocyte activating or co-stimulating compounds or for identifying inhibitors of leukocyte activation and stimulation comprising:

(a) culturing lymphocytes or monocytes in the presence of the protein of claim 7, the antibody of claim 8, the cell of claim 5 or 9 and, optionally, in the presence of a component capable of providing a detectable signal in response to leukocyte proliferation, with a compound to be screened under conditions permitting interaction of the compound with the (poly)peptide, antibody or cell(s); and
(b) detecting the presence or absence of a signal generated from the interaction of the compound with the cells.

**17.** A method of identifying modulators of immune responses comprising the steps of:

(a) incubating a candidate compound with the protein of claim 7;
(b) assaying a biological activity as described above; and
(c) determining if a biological activity of the protein of claim 7 has been altered.

**18.** A method for determining the status of an immune response comprising analyzing the presence of the polynucleotide of claim 1 or the protein of claim 7.

**19.** Use of the polynucleotide of claim 1, the nucleic acid molecule as defined in claim 2, the vector of claim 3 or 4, the protein of claim 7, the antibody of claim 8, the cell of claim 5 or 9 or the compound identified according to the method of any one of claims 15 to 17 for the preparation of a composition for diagnosing or the treatment of acute and chronic diseases, involving T cell activation and Th1 and Th2 immune response, for the treatment of acute and chronic rejection of allo and xeno organ transplants and bone marrow transplantation, for the treatment of rheumatoid arthritis, lupus erythematodes, multiple sclerosis, encephalitis, vasculitis, diabetes mellitus, pancreatitis, gastritis, thyroiditis, for the treatment of malign disorders of T, B or NK cells, for the treatment of asthma, lepromatosis, *Helicobacter pylori* associated gastritis or for the treatment of skin tumors, adrenal tumors or lung tumors, wound healing, growth disorders, inflammatory and/or infectious diseases.

**20.** Use of a polynucleotide of claim 1, a nucleic acid molecule as defined in claim 2 or the antibody of claim 8 for the *in vitro* detection of leukocyte activation.

**21.** Use of claim 20, wherein said leukocyte is a B cell, T cell, NK cell and/or monocyte.

**22.** Use of a polynucleotide of claim 1 or of a protein encoded by said polynucleotide in *in vitro* screening for identifying leukocyte activating or costimulating compounds or for identifying inhibitors of leukocyte activation and stimulation.

## Patentansprüche

**1.** Polynukleotid, codierend für ein Protein, bestehend aus einer DNA-Sequenz, ausgewählt aus der Gruppe:

(i) DNA-Sequenzen, umfassend eine für die in SEQ ID NO: 2, 4 oder 6 dargestellte Aminosäuresequenz codierende Nukleotidsequenz;
(ii) DNA-Sequenzen, umfassend die in SEQ ID NO: 1, 3 oder 5 dargestellte Nukleotidsequenz;
(iii) DNA-Sequenzen, deren komplementärer Strang mit dem Polynukleotid mit der unter (i) oder (ii) angegebenen Bedeutung hybridisiert und wenigstens 90% identisch damit ist;
(iv) DNA-Sequenzen, deren Nukleotid gegenüber der Nukleotidsequenz einer DNA-Sequenz eines der Punkte (i) bis (iii) degeneriert ist; und
(v) DNA-Sequenzen, umfassend eine für eine Konsensussequenz der in SEQ ID NO: 2, 4 und 6 dargestellten Aminosäuresequenzen codierende Nukleotidsequenz.

**2.** Verwendung eines Nukleinsäuremoleküls mit einer Länge von wenigstens 15 Nukleotiden spezifisch hybridisierend bei stringenten Bedingungen für mindestens 10 Stunden in 50% Formamid/6x SSC/0,1% SDS bei 42 °C oder in 6x SSC bei 68 °C mit dem Polynukleotid nach Anspruch 1 oder dessen komplementären Strang zum Nachweis und/oder zur Amplifikation eines Polynukleotids nach Anspruch 1 oder zur Repression oder targeting eines ein Polynukleotid nach Anspruch 1 umfassenden Gens.

**3.** Vektor, umfassend das Polynukleotid nach Anspruch 1.

**4.** Vektor nach Anspruch 3, bei dem das Polynukleotid in operativer Verknüpfung mit Regulationssequenzen steht, die die Transkription und gegebenenfalls Expression des Polynukleotids gestatten.

**5.** Wirtszelle, umfassend das Polynukleotid nach Anspruch 1 oder den Vektor nach Anspruch 3 oder 4.

**6.** Verfahren zur Produktion eines Proteins, bei dem man

(a) den Wirt nach Anspruch 5 unter Bedingungen, die die Expression des Proteins gestatten, kultiviert oder
(b) das Polynukleotid nach Anspruch 1 in vitro translatiert und das unter (a) oder (b) produzierte Protein gewinnt.

**7.** Protein, codiert durch das Nukleinmolekül nach Anspruch 1 oder produziert mit dem Verfahren nach Anspruch 6.

**8.** Antikörper, spezifisch erkennend das Protein nach Anspruch 7.

**9.** Zelle, die zur Expression des Proteins nach Anspruch 7 oder des Antikörpers nach Anspruch 8 modifiziert wurde.

**10.** Pharmazeutische Zusammensetzung, umfassend das Polynukleotid nach Anspruch 1, das Nukleinsäuremolekül mit der in Anspruch 2 angegebenen Bedeutung, einen Vektor nach Anspruch 3 oder 4, die Zelle nach Anspruch 5, das Protein nach Anspruch 7, den Antikörper nach Anspruch 8 oder ein Antisense-Konstrukt, das zur Hemmung der Expression eines Polynukleotids nach Anspruch 1 fähig ist, sowie gegebenenfalls einen pharmazeutisch unbedenklichen Trägerstoff.

**11.** Pharmazeutische Zusammensetzung nach Anspruch 10 zur Verwendung bei der Zell- oder Organtransplantation, zur Behandlung von Autoimmun-, Allergie- oder Infektionskrankheiten oder zur Behandlung von Tumoren.

**12.** Diagnostische Zusammensetzung, umfassend ein Polynukleotid nach Anspruch 1, das Nukleinsäuremolekül mit der in Anspruch 2 angegebenen Bedeutung, den Vektor nach Anspruch 3 oder 4, die Zelle nach Anspruch 5 oder 9, das Protein nach Anspruch 7 oder den Antikörper nach Anspruch 8; sowie gegebenenfalls wenigstens eine Komponente, die markiert ist.

**13.** In-vitro-Verfahren zur Diagnose eines Krankheitszustands oder einer Anfälligkeit für einen Krankheitszustand in einem Individuum in Verbindung mit einer Störung im Immunsystem, wobei man in dem Verfahren

(a) das Vorliegen oder Fehlen einer Mutation im Polynukleotid nach Anspruch 1 in einer Probe bestimmt und
(b) einen Krankheitszustand oder eine Anfälligkeit für einen Krankheitszustand auf der Grundlage des Vorliegens bzw. Fehlens der Mutation diagnostiziert.

**14.** Verfahren zur Diagnose eines Krankheitszustands oder einer Anfälligkeit für einen Krankheitszustand in einem Individuum in Verbindung mit einer Störung im Immunsystem, wobei man in dem Verfahren

(a) das Vorliegen von oder die Menge an Expression des Proteins nach Anspruch 7 in einer biologischen Probe bestimmt und
(b) einen Krankheitszustand oder eine Anfälligkeit für einen Krankheitszustand auf der Grundlage des Vorliegens von bzw. der Menge an Expression des Proteins diagnostiziert.

**15.** Verfahren zur Identifizierung eines Bindungspartners zu einem Protein, bei dem man

(a) ein Protein nach Anspruch 7 mit einer einem Screening zu unterziehenden Verbindung in Kontakt bringt und
(b) bestimmt, ob sich die Verbindung auf eine Aktivität des Protein auswirkt.

**16.** Verfahren zur Identifizierung Leukozyten aktivierender oder costimulierender Verbindungen oder zur Identifizierung von Inhibitoren der Leukozytenaktivierung bzw. -stimulierung, wobei man in dem Verfahren

(a) Lymphozyten oder Monozyten in Gegenwart des Proteins nach Anspruch 7, des Antikörpers nach Anspruch 8, der Zelle nach Anspruch 5 oder 9 und gegebenenfalls in Gegenwart einer Komponente, die ein nachweisbares Signal als Antwort auf Leukozytenproliferation liefern kann, mit einer einem Screening zu unterziehenden Verbindung unter Bedingungen kultiviert, die die Wechselwirkung der Verbindung mit dem (Poly-)Peptid, dem Antikörper oder der Zelle bzw. den Zellen gestatten, und
(b) das Vorliegen oder Fehlen eines aus der Wechselwirkung der Verbindung mit den Zellen erzeugten Signals nachweist.

**17.** Verfahren zur Identifizierung von Modulatoren von Immunantworten, wobei man in den Verfahrensschritten

(a) eine Kandidatenverbindung mit dem Protein nach Anspruch 7 inkubiert,
(b) eine biologische Aktivität wie oben beschrieben testet und
(c) bestimmt, ob eine biologische Aktivität des Proteins nach Anspruch 7 verändert wurde.

**18.** Verfahren zur Bestimmung des Status einer Immunantwort, bei dem man das Vorliegen des Polynukleotids nach Anspruch 1 oder des Proteins nach Anspruch 7 analysiert.

**19.** Verwendung des Polynukleotids nach Anspruch 1, des Nukleinsäuremoleküls mit der in Anspruch 2 angegebenen Bedeutung, des Vektors nach Anspruch 3 oder 4, des Proteins nach Anspruch 7, des Antikörpers nach Anspruch 8, der Zelle nach Anspruch 5 oder 9 oder der gemäß dem Verfahren nach einem der Ansprüche 15 bis 17 identifizierten Verbindung zur Herstellung einer Zusammensetzung zur Diagnose oder Behandlung akuter und chronischer Krankheiten mit Aktivierung von T-Zellen und Th1- und Th2-Immunantwort, zur Behandlung akuter und chronischer Abstoßung von Allo- und Xenoorgantransplantaten und Knochenmarkstransplantation, zur Behandlung rheumatoider Arthritis, Lupus erythematodes, multipler Sklerose, Enzephalitis, Vaskulitis, Diabetes mellitus, Pankreatitis, Gastritis, Thyreoiditis, zur Behandlung bösartiger Erkrankungen von T-, B- oder NK-Zellen, zur Behandlung von Asthma, Lepromatose, mit *Heliobacter pylori* assoziierter Gastritis oder zur Behandlung von Hauttumoren, Nebennierentumoren oder Lungentumoren, Wundheilung, Wachstumsstörungen, Entzündungs- und/oder Infektionskrankheiten.

**20.** Verwendung eines Polynukleotids nach Anspruch 1, eines Nukleinsäuremoleküls mit der in Anspruch 2 angegebenen Bedeutung oder des Antikörpers nach Anspruch 8 zum In-vitro-Nachweis der Leukozytenaktivierung.

**21.** Verwendung nach Anspruch 20, wobei es sich bei dem Leukozyten um eine B-Zelle, T-Zelle, NK-Zelle und/oder einen Monozyten handelt.

**22.** Verwendung eines Polynukleotids nach Anspruch 1 oder eines von dem Polynukleotid codierten Proteins beim In-vitro-Screening zur Identifizierung Leukozyten aktivierender oder costimulierender Verbindungen oder zur Identifizierung von Inhibitoren der Leukozytenaktivierung bzw. -stimulierung

## Revendications

**1.** Polynucléotide codant pour une protéine constituée par une séquence d'ADN choisie dans le groupe constitué par:

(i) des séquences d'ADN comprenant une séquence nucléotidique codant pour la séquence d'acides aminés représentée dans SEQ ID NO : 2, 4 ou 6 ;
(ii) des séquences d'ADN comprenant la séquence nucléotidique représentée dans SEQ ID NO : 1, 3 ou 5 ;
(iii) des séquences d'ADN dont le brin complémentaire s'hybride au et présente une identité d'au moins 90 % avec le polynucléotide tel que défini dans le point (i) ou (ii) ;
(iv) des séquences d'ADN dont le nucléotide est dégénéré en la séquence nucléotidique d'une séquence d'ADN selon l'un quelconque des points (i) à (iii) ; et
(v) des séquences d'ADN comprenant une séquence nucléotidique codant pour une séquence consensus des séquences d'acides aminés représentées dans SEQ ID NO : 2, 4 et 6.

**2.** Utilisation d'une molécule d'acide nucléique d'une longueur d'au moins 15 nucléotides s'hybridant spécifiquement dans des conditions stringentes pendant au moins 10 heures dans du formamide à 50 %/SSC 6 x/SDS à 0,1 % à 42 °C ou dans du SSC 6 x à 68 °C avec le polynucléotide selon la revendication 1 ou le brin complémentaire de celui-ci pour la détection et/ou l'amplification d'un polynucléotide selon la revendication 1 ou pour la répression ou le ciblage d'un gène comprenant un polynucléotide selon la revendication 1.

**3.** Vecteur comprenant le polynucléotide selon la revendication 1.

**4.** Vecteur selon la revendication 3, dans lequel ledit polynucléotide est lié de manière fonctionnelle à des séquences régulatrices pour la transcription et facultativement l'expression dudit polynucléotide.

**5.** Cellule hôte comprenant un polynucléotide selon la revendication 1 ou le vecteur selon la revendication 3 ou 4.

**6.** Procédé de production d'une protéine, qui comprend:

(a) la culture de l'hôte selon la revendication 5 dans des conditions permettant l'expression de la protéine ; ou
(b) la traduction *in vitro* du polynucléotide selon la revendication 1 ; et la récupération de la protéine produite dans (a) ou (b).

**7.** Protéine codée par une molécule d'acide nucléique selon la revendication 1 ou produite par le procédé selon la revendication 6.

**8.** Anticorps reconnaissant spécifiquement la protéine selon la revendication 7.

**9.** Cellule qui a été modifiée pour exprimer la protéine selon la revendication 7 ou l'anticorps selon la revendication 8.

**10.** Composition pharmaceutique comprenant le polynucléotide selon la revendication 1, la molécule d'acide nucléique telle que définie dans la revendication 2, un vecteur selon la revendication 3 ou 4, la cellule selon la revendication 5, la protéine selon la revendication 7, l'anticorps selon la revendication 8 ou une construction antisens capable d'inhiber l'expression d'un polynucléotide selon la revendication 1, et facultativement un véhicule pharmaceutiquement acceptable.

**11.** Composition pharmaceutique selon la revendication 10, destinée à être utilisée dans une transplantation cellulaire ou d'organe, pour le traitement de maladies autoimmunes, allergiques ou infectieuses, ou pour le traitement de tumeurs.

**12.** Composition diagnostique comprenant un polynucléotide selon la revendication 1, la molécule d'acide nucléique telle que définie dans la revendication 2, le vecteur selon la revendication 3 ou 4, la cellule selon la revendication 5 ou 9, la protéine selon la revendication 7, ou l'anticorps selon la revendication 8 ; et facultativement, au moins un composant qui est marqué.

**13.** Procédé *in vitro* de diagnostic d'une condition pathologique ou d'une prédisposition à une condition pathologique apparentée à un trouble du système immunitaire chez un sujet, qui comprend:

(a) la détermination de la présence ou de l'absence d'une mutation dans le polynucléotide selon la revendication 1 dans un échantillon ; et
(b) le diagnostic d'une condition pathologique ou d'une prédisposition à une condition pathologique basé sur la présence ou l'absence de ladite mutation.

**14.** Procédé de diagnostic d'une condition pathologique ou d'une prédisposition à une condition pathologique apparentée à un trouble du système immunitaire chez un sujet, qui comprend:

(a) la détermination de la présence ou de la quantité d'expression de la protéine selon la revendication 7 dans un échantillon biologique ; et
(b) le diagnostic d'une condition pathologique ou d'une prédisposition à une condition pathologique basé sur la présence ou la quantité d'expression de la protéine.

**15.** Procédé d'identification d'un partenaire de liaison à une protéine, qui comprend:

(a) la mise en contact d'une protéine selon la revendication 7 avec un composé à cribler ; et
(b) la détermination du fait que le composé affecte ou non une activité de la protéine.

**16.** Procédé d'identification de composés activant ou co-stimulant les leucocytes ou d'identification d'inhibiteurs de l'activation et de la stimulation des leucocytes, qui comprend:

(a) la culture de lymphocytes ou de monocytes en présence de la protéine selon la revendication 7, de l'anticorps selon la revendication 8, de la cellule selon la revendication 5 ou 9 et, éventuellement, en présence d'un composant capable de fournir un signal détectable en réponse à la prolifération des leucocytes, avec un composé à cribler dans des conditions permettant l'interaction du composé avec le (poly)peptide, l'anticorps ou la (les) cellule(s) ; et
(b) la détection de la présence ou de l'absence d'un signal généré par l'interaction du composé avec les cellules.

**17.** Procédé d'identification de modulateurs des réponses immunitaires, qui comprend les étapes consistant à:

(a) faire incuber un composé candidat avec la protéine selon la revendication 7 ;
(b) tester une activité biologique telle que décrite ci-dessus ; et
(c) déterminer si une activité biologique de la protéine selon la revendication 7 a été modifiée.

**18.** Procédé de détermination du statut d'une réponse immunitaire qui comprend l'analyse de la présence du polynucléotide selon la revendication 1 ou de la protéine selon la revendication 7.

**19.** Utilisation du polynucléotide selon la revendication 1, de la molécule d'acide nucléique telle que définie dans la revendication 2, du vecteur selon la revendication 3 ou 4, de la protéine selon la revendication 7, de l'anticorps selon la revendication 8, de la cellule selon la revendication 5 ou 9 ou du composé identifié par le procédé selon l'une quelconque des revendications 15 à 17 pour la préparation d'une composition destinée au diagnostic ou au traitement de maladies aiguës et chroniques, impliquant l'activation des cellules T et la réponse immunitaire Th1 et Th2, pour le traitement d'un rejet aigu et chronique de transplantations allogéniques et xénogéniques d'organes et de la transplantation de moelle osseuse, pour le traitement de la polyarthrite rhumatoïde, du lupus érythémateux, de la sclérose en plaques, d'une encéphalite, d'une vasculite, du diabète sucré, d'une pancréatite, d'une gastrite, d'une thyroïdite, pour le traitement de troubles malins des cellules T, B ou NK, pour le traitement de l'asthme, de la lèpre lépromateuse, d'une gastrite associée à *Helicobacter pylori* ou pour le traitement de tumeurs cutanées, de tumeurs des surrénales ou de tumeurs des poumons, de la cicatrisation de blessures, de troubles de la croissance, de maladies inflammatoires et/ou infectieuses.

**20.** Utilisation d'un polynucléotide selon la revendication 1, d'une molécule d'acide nucléique telle que définie dans la revendication 2 ou de l'anticorps selon la revendication 8, pour la détection *in vitro* de l'activation des leucocytes.

**21.** Utilisation selon la revendication 20, dans laquelle ledit leucocyte est une cellule B, une cellule T, une cellule NK et/ou un monocyte.

**22.** Utilisation d'un polynucléotide selon la revendication 1 ou d'une protéine codée par ledit polynucléotide dans un criblage *in vitro* pour l'identification de composés activant ou co-stimulant les leucocytes ou pour l'identification d'inhibiteurs de l'activation et de la stimulation des leucocytes.

# ClustalW Formatted Alignments

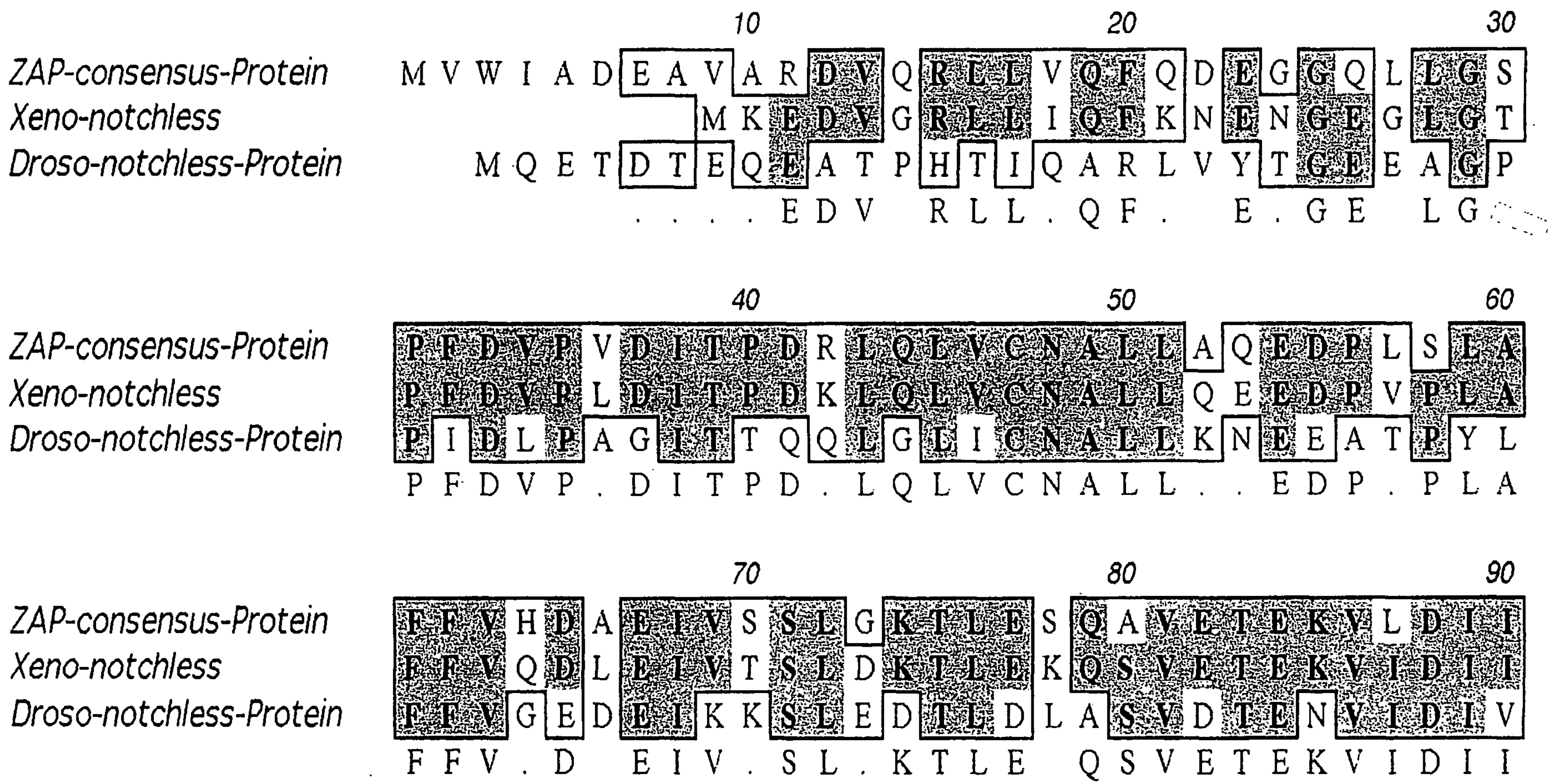


Fig. 1

| | 100 / 110 / 120 |
|---|---|
| ZAP-consensus-Protein | L P T Q A V F K V R A V T R C T S S L E G H T E A V I S V A |
| Xeno-notchless | Y Q P Q A V F K V R A V T R C T S S L E G H T E A V I S V A |
| Droso-notchless-Protein | Y Q P Q A V F K V R P V T R C T S S M P G H A E A V V S L N |
| | Y Q P Q A V F K V R A V T R C T S S L E G H T E A V I S V A |

| | 130 / 140 / 150 |
|---|---|
| ZAP-consensus-Protein | F S P T G K Y L A S G S G D T T V R F W D L S T E T P H F T |
| Xeno-notchless | F S P T G K Y L A S G S G D T T V R F W D L S T E T P H F T |
| Droso-notchless-Protein | F S P D G A H L A S G S G D T T V R L W D L N T E T P H F T |
| | F S P T G K Y L A S G S G D T T V R F W D L S T E T P H F T |

| | 160 / 170 / 180 |
|---|---|
| ZAP-consensus-Protein | S K G H T H W V L S I A W S P D G K K L A S G C K N S Q I F |
| Xeno-notchless | S K G H T H W V L S I A W S P D G K K L A S G C K N S Q I F |
| Droso-notchless-Protein | C T G H K Q W V L C V S W A P D G K R L A S G C K A G S I I |
| | S K G H T H W V L S I A W S P D G K K L A S G C K N S Q I F |

| | 190 / 200 / 210 |
|---|---|
| ZAP-consensus-Protein | I W D P S T G K Q I G K P L T G H S K W I T W L C W E P L H |
| Xeno-notchless | I W D P S T G K Q I G K P L T G H S K W I T W L C W E P L H |
| Droso-notchless-Protein | I W D P E T G Q Q K G R P L S G H K K H I N C L A W E P Y H |
| | I W D P S T G K Q I G K P L T G H S K W I T W L C W E P L H |

Fig. 1 cont.

| | 220 · 230 · 240 |
|---|---|
| ZAP-consensus-Protein | L N P E S R Y L A S A S S G R V D R I W D T T A G R C E R L |
| Xeno-notchless | L N P E S R Y L A S A S K D C T I R I W D T V M G Q C Q K I |
| Droso-notchless-Protein | R D P E C R K L A S A S G D G D C R I W D V K L G Q C L M N |
| | L N P E S R Y L A S A S D R I W D T . G Q C . . I |

| | 250 · 260 · 270 |
|---|---|
| ZAP-consensus-Protein | L T G H T Q S V T C L R W G G D G L L Y S A S Q D R T I K V |
| Xeno-notchless | L T S H T Q S V T A V K W G G D G L L Y S S S Q D R T I K A |
| Droso-notchless-Protein | I A G H T N A V T A V R W G G A G L I Y T S S K D R T V K M |
| | L T G H T Q S V T A V R W G G D G L L Y S S S Q D R T I K . |

| | 280 · 290 · 300 |
|---|---|
| ZAP-consensus-Protein | W R A H D G V L C R T L Q G H G H W V N T M A L S T D Y A L |
| Xeno-notchless | W R A Q D G V L C R T L Q G H A H W V N T M A L S T D Y V L |
| Droso-notchless-Protein | W R A A D G I L C R T F S G Q A H W V N N I A L S T D Y V L |
| | W R A . D G V L C R T L Q G H A H W V N T M A L S T D Y V L |

| | 310 · 320 · 330 |
|---|---|
| ZAP-consensus-Protein | R T G A F E P A E A S V N P Q D L Q G S L Q E L K E R A L S |
| Xeno-notchless | R T G A F N P A D A S V N P Q D M S G S L E V L K E K A L K |
| Droso-notchless-Protein | R T G P F H P V K D R S K - S H L S L S T E E L Q E S A L K |
| | R T G A F . P A . A S V N P Q D L S G S L E E L K E . A L K |

Fig. 1 cont.

```
                                              340                 350                 360
ZAP-consensus-Protein     R Y N L V R G Q G P E R L V S G S D D F T L F L W S P A E D
Xeno-notchless            R Y N E V R G Q G P E R L V S G S D D F T L F L W A P A E E
Droso-notchless-Protein   R Y Q A V C P D E V E S L V S C S D D N T L Y E W R N N Q N
                          R Y N   V R G Q G P E R L V S G S D D F T L F L W . P A E .

                                              370                 380                 390
ZAP-consensus-Protein     K K P L T R M T G H Q A L I N Q V L F S P D S R I V A S A S
Xeno-notchless            K K P L Q R M T G H Q A L I N E V L F S P D T R I I A S A S
Droso-notchless-Protein   K - C V E R M T G H Q N V V N D V K Y S P D V K L I A S A S
                          K K P L . R M T G H Q A L I N . V L F S P D . R I I A S A S

                                              400                 410                 420
ZAP-consensus-Protein     F D K S I K L W D G R T G K Y L A S L R G H V A A V Y Q I A
Xeno-notchless            F D K S I K L W D G K T G K F L T S L R G H V S A V Y Q I A
Droso-notchless-Protein   F D K S V R L W R A S D G Q Y M A T F R G H V Q A V Y T V A
                          F D K S I K L W D G . T G K Y L A S L R G H V . A V Y Q I A

                                              430                 440                 450
ZAP-consensus-Protein     W S A D S R L L V S G S S D S T L K V W D V K A Q K L A M D
Xeno-notchless            W S A D S R L L V S G S S D S T L K V W D S K T K K L L I D
Droso-notchless-Protein   W S A D S R L I V S G S K D S T L K V W S V Q T K K L A Q E
                          W S A D S R L L V S G S S D S T L K V W D V K T K K L A . D
```

Fig. 1 cont.

| | 460 | 470 | 480 |
|---|---|---|---|
| ZAP-consensus-Protein | L P G H A D E V Y A | V D W S P D G Q R V | A S G G K D K C L R |
| Xeno-notchless | L P G H A D E V Y S | V D W S P D G Q R V | A S G G K D K C L R |
| Droso-notchless-Protein | L P G H A D E V F G | V D W A P D G S R V | A S G G K D K V I K |
| | L P G H A D E V Y . | V D W S P D G Q R V | A S G G K D K C L R |

| | 490 | 500 | 510 |
|---|---|---|---|
| ZAP-consensus-Protein | I W R R | | |
| Xeno-notchless | I W R K | | |
| Droso-notchless-Protein | L W A Y | | |
| | I W R . | | |

Fig. 1 cont.

# ClustalW Formatted Alignments

```
                                        10                  20                  30
TZap7/A
Droso-notchless-Protein   M Q E T D T E Q E A T P H T I Q A R L V Y T G E E A G P P I
Xeno-notchless                        M K E D V G R L L I Q F K N E N G E G L G T P F
                                       . E       .   .             . G E     G   P

                                        40                  50                  60
TZap7/A
Droso-notchless-Protein   D L P A G L T T Q Q L G L I C N A L L K N E E A T P Y L F F
Xeno-notchless            D V P L D E T P D K L Q L V C N A L L Q E E D P V P L A F F
                          D . P     I T   . L   L . C N A L L .   E .     P       F F

                                        70                  80                  90
TZap7/A
Droso-notchless-Protein   V G E D E I K K S L E D T L D L A S V D T E N V I D I V Y Q
Xeno-notchless            V Q D L E I V T S L D K T L E K Q S V E T E K V I D I I Y Q
                          V   .   E I     S L .   T L .     S V . T E   V I D I . Y Q
```

Fig. 2

100 110 120

TZap7/A
Droso-notchless-Protein P Q A V F K V R P V T R C T S S M P G H A E A V V S L N F S
Xeno-notchless P Q A V E K V R A V T R C T S S L E G H T E A V I S V A F S
P Q A V F K V R V T R C T S S . G H . E A V . S . F S

130 140 150

TZap7/A
Droso-notchless-Protein P D G A H L A S G S G D T T V R L W D L N T E T P H F T C T
Xeno-notchless P T G K Y L A S G S G D T T V R F W D L S T E T P H F T S K
P G L A S G S G D T T V R W D L T E T P H F T .

160 170 180

TZap7/A
Droso-notchless-Protein G H K Q W V L C V S W A P D G K R L A S G C K A G S I I I W
Xeno-notchless G H T H W V L S I A W S P D G K K L A S G C K N S Q I F I W
G H . W V L . . . W . P D G K . L A S G C K I I W

190 200 210

TZap7/A G R - - - - - - - - - - - - - - - - - - -
Droso-notchless-Protein D P E T G Q Q K G R P L S G H K K H I N C L A W E P Y H R D
Xeno-notchless D P S T G K Q I G K P L T G H S K W I T W L C W E P L H L N
D P T G . Q G R P L . G H K I . L . W E P H

Fig. 2 cont.

```
                                         220                 230                 240
TZap7/A                  - - - - - - - - - - - - - - V D R I W D T T A G R C E R I L T
Droso-notchless-Protein  P E C R K L A S A S G D G D C R I W D V K L G Q C L M N I A
Xeno-notchless           P E S R Y L A S A S K D C T I R I W D T V M G Q C Q K I L T
                         P E . R   L A S A S   D       R I W D T   . G Q C . . I L T

                                         250                 260                 270
TZap7/A                  G H T Q S V T C L R W G G D G L L Y S A S Q D R T I K V W R
Droso-notchless-Protein  G H T N A V T A V R W G G A G L I Y T S S K D R T V K M W R
Xeno-notchless           S H T Q S V T A V K W G G D G L L Y S S S Q D R T I K A W R
                         G H T Q S V T A V R W G G D G L L Y S S S Q D R T I K . W R

                                         280                 290                 300
TZap7/A                  A H D G V L C R T L Q G H G H W V N T M A L S T D Y A L R T
Droso-notchless-Protein  A A D G I L C R T F S G Q A H W V N N I A L S T D Y V L R T
Xeno-notchless           A Q D G V L C R T L Q G H A H W V N T M A L S T D Y V L R T
                         A . D G V L C R T L Q G H A H W V N T M A L S T D Y V L R T

                                         310                 320                 330
TZap7/A                  G A F E P A E A S V N P Q D L Q G S L Q E L K E R A L S R Y
Droso-notchless-Protein  G P F H P V K D R S K - S H L S L S T E E L Q E S A L K R Y
Xeno-notchless           G A F N P A D A S V N P Q D M S G S L E V L K E K A L K R Y
                         G A F . P A . A S V N P Q D L S G S L E E L K E . A L K R Y
```

Fig. 2 cont.

```
                                               340                 350                 360
TZap7/A                   N L V R G Q G P E R L V S G S D D F T L F L W S P A E D K K
Droso-notchless-Protein   Q A V C P D E V E S L V S C S D D N T L Y L W R N N Q N K -
Xeno-notchless            N E V R G Q G P E R L V S G S D D F T L F L W A P A E E K K
                          N   V R G Q G P E R L V S G S D D F T L F L W . P A E . K K

                                               370                 380                 390
TZap7/A                   P L T R M T G H Q A L I N Q V L F S P D S R I V A S A S F D
Droso-notchless-Protein   C V E R M T G H Q N V V N D V K Y S P D V K L I A S A S F D
Xeno-notchless            P L Q R M T G H Q A L I N E V L F S P D T R I I A S A S F D
                          P L . R M T G H Q A L I N . V L F S P D . R I I A S A S F D

                                               400                 410                 420
TZap7/A                   K S I K L W D G R T G K Y L A S L R G H V A A V Y Q I A W S
Droso-notchless-Protein   K S V R L W R A S D G Q Y M A T F R G H V Q A V Y T V A W S
Xeno-notchless            K S I K L W D G K T G K F L T S L R G H V S A V Y Q I A W S
                          K S I K L W D G . T G K Y L A S L R G H V . A V Y Q I A W S

                                               430                 440                 450
TZap7/A                   A D S R L L V S G S S D S T L K V W D V K A Q K L A M D L P
Droso-notchless-Protein   A D S R L I V S G S K D S T L K V W S V Q T K K L A Q E L P
Xeno-notchless            A D S R L L V S G S S D S T L K V W D S K T K K L L I D L P
                          A D S R L L V S G S S D S T L K V W D V K T K K L A . D L P
```

Fig. 2 cont.

```
                                     460                 470                 480
TZap7/A                   G H A D E V Y A V D W S P D G Q R V A S G G K D K C L R I W
Droso-notchless-Protein   G H A D E V F G V D W A P D G S R V A S G G K D K V I K L W
Xeno-notchless            G H A D E V Y S V D W S P D G Q R V A S G G K D K C L R I W
                          G H A D E V Y . V D W S P D G Q R V A S G G K D K C L R I W

                                     490                 500                 510
TZap7/A                   R R
Droso-notchless-Protein   A Y
Xeno-notchless            R K
                          R .
```

Fig. 2 cont.

# ClustalW Formatted Alignments

```
                                        10                  20                  30
TZap7/A                                                                       G R V
ß-transducin-Protein
AF056183                M A T A A V A R G W L R A G E E R S G R P A C Q K A N G F P
BRTC-Protein              M D P A E A V L Q E K A L K F M N S S E R E D C N N G E P
                                                                              G

                                        40                  50                  60
TZap7/A                 D R - - - - - - - - - - - I W D T T A G R C E R - - - - - -
ß-transducin-Protein
AF056183                P D K - - - S S G S K K Q K Q Y Q R I R K E K P Q Q - - - -
BRTC-Protein            P R K I I P E K N S L R Q T Y N S C A R L C L N Q E T V C L

                                        70                  80                  90
TZap7/A                 - - - - I L T G H T Q S V T C L R W G G D G L L Y - - - - -
ß-transducin-Protein
AF056183                - - H N F T H R L L A A A L K S H S G N I S C M D F S - - -
BRTC-Protein            A S T A M K T E N C V A K T K L A N G T S S M I V P K Q R K
                                                            G
```

Fig. 3

```
                                       100                 110                 120
TZap7/A                 - S A S Q D R - - - - - - - T I K V W R A H D - - - - - - -
ß-transducin-Protein
AF056183                - S N G K Y L A T C A D D R T I R I W S T K D - - - - - - -
BRTC-Protein            L S A S Y E K E K E L C V K Y F E Q W S E S D Q V E F V E H
                          S                                 W       D

                                       130                 140                 150
TZap7/A                 - - G V L C R T L Q G H G - - - - - - - - - - - - - - - - -
ß-transducin-Protein
AF056183                - F L Q R E H R S M R A N - - - - - - - - - - - - - - - - -
BRTC-Protein            L I S Q M C H Y Q H G H I N S Y L K P M L Q R D F I T A L P

                                       160                 170                 180
TZap7/A                 - - - - - H W - - - - - - - - - - - - - - - - - - - - - - -
ß-transducin-Protein
AF056183                - V E L D H A T L V R F S P - - - - - - - - - - - - - - - -
BRTC-Protein            A R G L D H I A E N I L S Y L D A K S L C A A E L V C K E W
                                  H
```

Fig. 3 cont.

```
                                  190                 200                 210
TZap7/A                   - - - - - - - - - - - - - - - - - V N T M A L S T D Y A L R
ß-transducin-Protein
AF056183                  - - - - - - - - D C R A F I V W L A N G D T L R V F K M T K
BRTC-Protein              Y R V T S D G M L W K K L I E R M V R T D S L W R G L A E R
                                                                      . L             .

                                  220                 230                 240
TZap7/A                   T G A - - - - F - - E P A E A S V N P - - - - - - - - - - -
ß-transducin-Protein
AF056183                  R E D G G Y T F T A T P E D F P K K H K A P V I D I G I A N
BRTC-Protein              R G W G Q Y L F K N K P P D G N A P P N S F Y R A L Y P K I
                                        F         P   .

                                  250                 260                 270
TZap7/A                   - Q D L Q - - - - - - - - - - - - - G S L Q E L K E R A L S
ß-transducin-Protein
AF056183                  T G K F I M T A S S D T T V L I W S L K G Q V L S T I N T N
BRTC-Protein              I Q D I E T I E S - - - N W R C G R H S L Q R I H C R S E T
                                                                      Q   .
```

Fig. 3 cont.

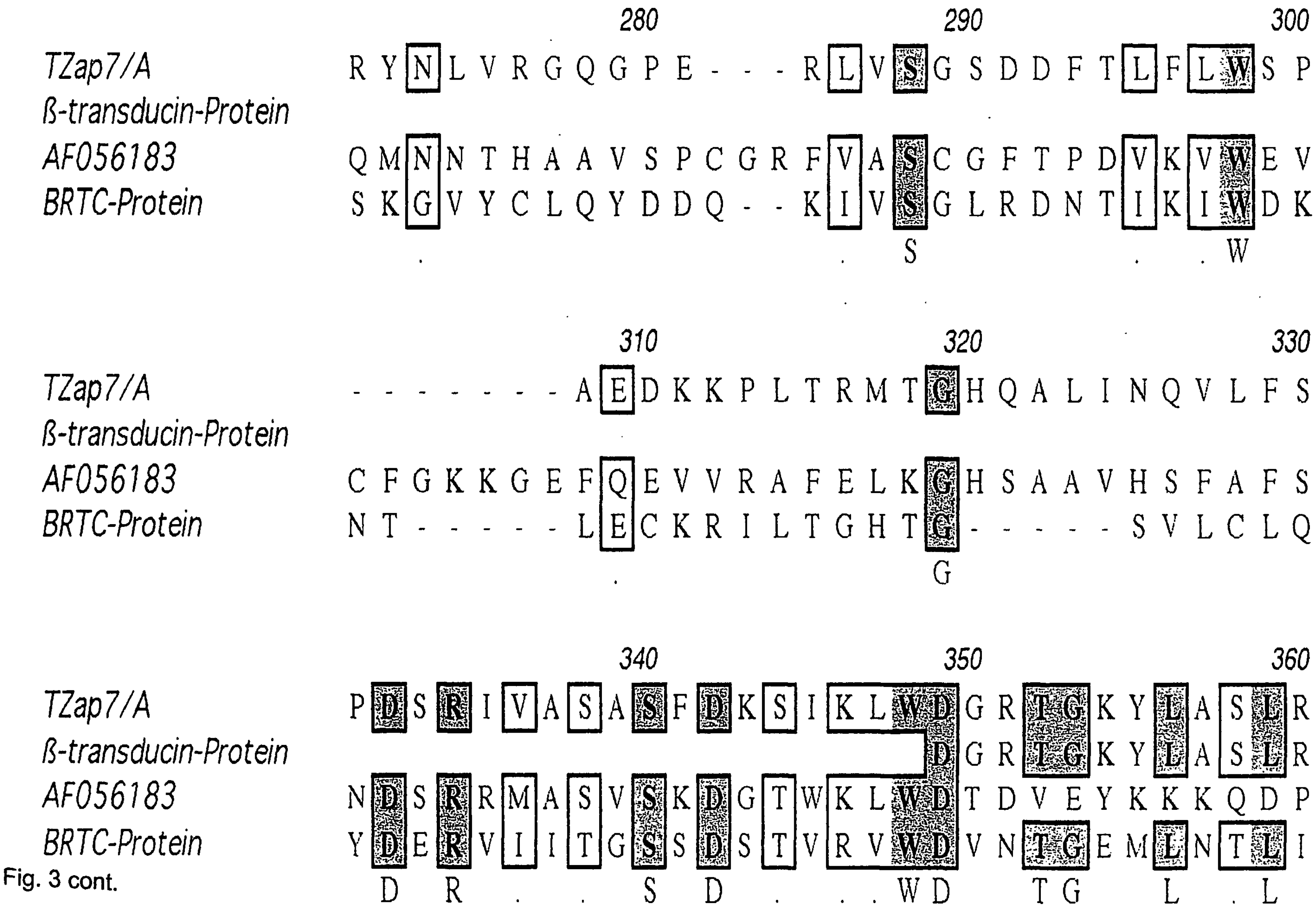
280 290 300
TZap7/A R Y N L V R G Q G P E - - - R L V S G S D D F T L F L W S P
ß-transducin-Protein
AF056183 Q M N N T H A A V S P C G R F V A S C G F T P D V K V W E V
BRTC-Protein S K G V Y C L Q Y D D Q - - K I V S G L R D N T I K I W D K
. . S . . W
310 320 330
TZap7/A - - - - - - - A E D K K P L T R M T G H Q A L I N Q V L F S
ß-transducin-Protein
AF056183 C F G K K G E F Q E V V R A F E L K G H S A A V H S F A F S
BRTC-Protein N T - - - - - L E C K R I L T G H T G - - - - - S V L C L Q
. G
340 350 360
TZap7/A P D S R I V A S A S F D K S I K L W D G R T G K Y L A S L R
ß-transducin-Protein D G R T G K Y L A S L R
AF056183 N D S R R M A S V S K D G T W K L W D T D V E Y K K K Q D P
BRTC-Protein Y D E R V I I T G S S D S T V R V W D V N T G E M L N T L I
D R . . S D . . . W D T G L . L
Fig. 3 cont.

```
                                    370                 380                 390
TZap7/A                 - - - - - - - - - - - - - - - - - - - - - - - - - - - - - -
ß-transducin-Protein    - - - - - - - - - - - - - - - - - - - - - - - - - - - - - -
AF056183                - - - - - - - - - - - - - - - - - - - - - - - - - - - - - -
BRTC-Protein            H H C E A V L H L R F N N G M M V T C S K D R S I A V W D M

                                    400                 410                 420
TZap7/A                 - - - - - - - - - - - - - G H V A A V - - - - - - - - Y Q I
ß-transducin-Protein    - - - - - - - - - - - - - G H V A A V - - - - - - - - Y Q I
AF056183                - - - - - - - - Y L L K T G R F E E A A G - - - A A P C R L
BRTC-Protein            A S P T D I T L R R V L V G H R A A V N V V D F D D K Y I V
                                                  G H   A A V                 Y . .

                                    430                 440                 450
TZap7/A                 A W S A D - - - - - - - - - - - - - - - - - - - - - - - - -
ß-transducin-Protein    A W S A D - - - - - - - - - - - - - - - - - - - - - - - - -
AF056183                A L S P N - - - - - - - - - - - - - - - - - - - - - - - - -
BRTC-Protein            S A S G D R T I K V W N T S T C E F V R T L N G H K R G I A
                        A   S   D
```

Fig. 3 cont.

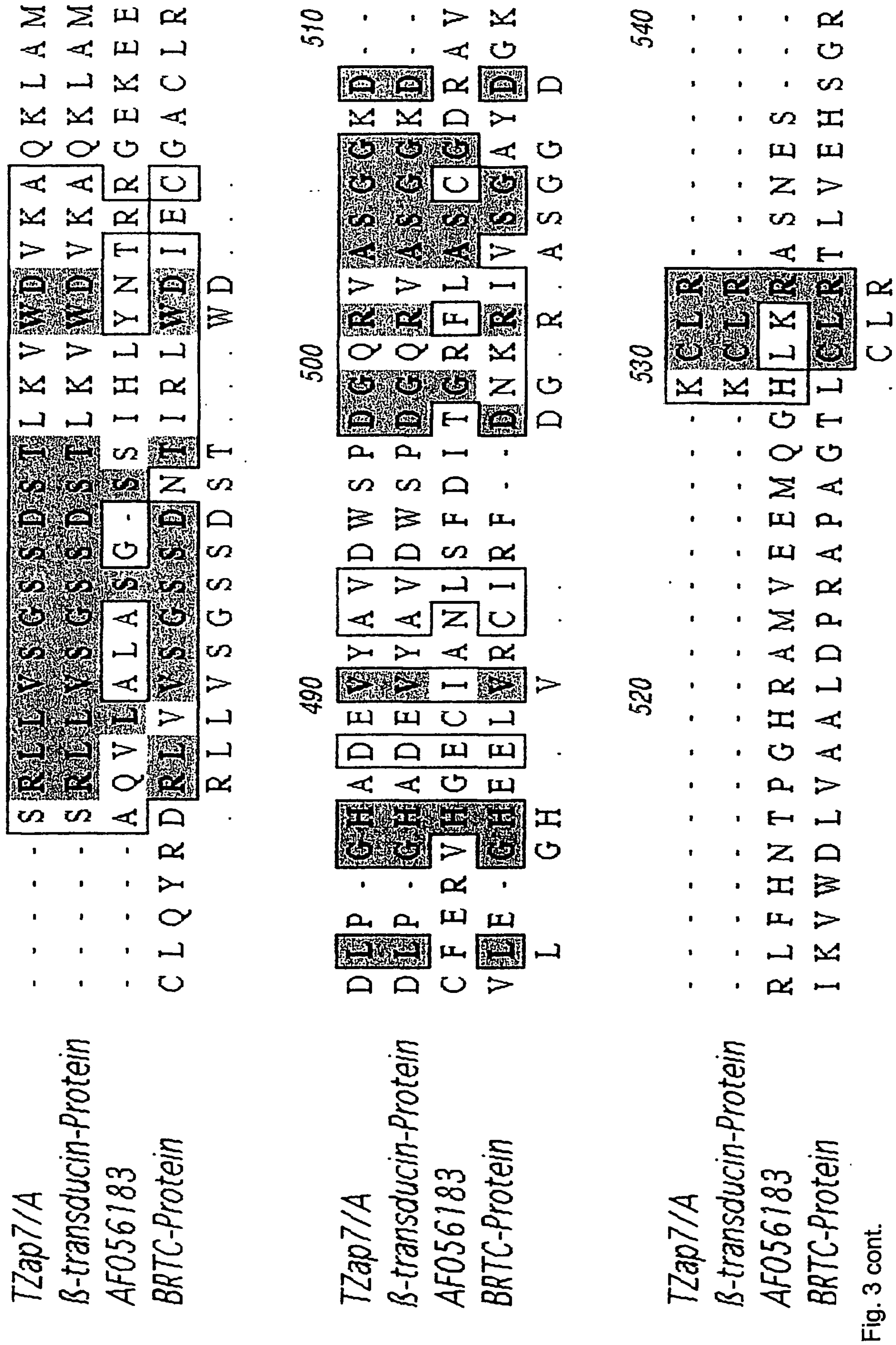
TZap7/A
ß-transducin-Protein
AF056183
BRTC-Protein
460
470
480
490
500
510
520
530
540

Fig. 3 cont.

| | 550 / 560 / 570 |
|---|---|
| TZap7/A | - - - - - - - - - - - - - - - - - - - - - I W R R |
| ß-transducin-Protein | - - - - - - - - - - - - - - - - - - - - - I W R R |
| AF056183 | - - - - - - - - - - - - - - - - - - - - - T R Q R L Q Q Q L |
| BRTC-Protein | V F R L Q F D E F Q I V S S S H D D T I L I W D F L N D P A |
| | I W . R |

| | 580 / 590 / 600 |
|---|---|
| TZap7/A | |
| ß-transducin-Protein | |
| AF056183 | T Q A Q E T L K S L G A L K K |
| BRTC-Protein | A Q A E P P R S P S R T Y T Y I S R |

Fig. 3 cont.

# ClustalW Formatted Alignments

```
                                       10                  20                  30
TZap-7-Protein            M A A A V A D E A V A R D V Q R L L V Q F Q D E G G Q L L G
Xeno-notchless                            M K E D V G R L L I Q F K N E N G E G L G
Droso-notchless-Protein         M Q E T D T E Q E A T P H T I Q A R L V Y T G E E A G
                                  . . . . E D V   R L L . Q F .   E . . G E   L G

                                       40                  50                  60
TZap-7-Protein            S P F D V P V D I T P D R L Q L V C N A L L A Q E D P L P L
Xeno-notchless            T P F D V P L D I T P D K L Q L V C N A L L Q E E D P V P L
Droso-notchless-Protein   P P I D L P A G L T T Q Q L G L I C N A L L K N E E A T P Y
                          . P F D V P . D I T P D . L Q L V C N A L L . . E D P . P L

                                       70                  80                  90
TZap-7-Protein            A F F V H D A E I V S S L G K T L E S Q A V E T E K V L D I
Xeno-notchless            A F F V Q D L E I V T S L D K T L E K Q S V E T E K V I D I
Droso-notchless-Protein   L F F V G E D E I K K S L E D T L D L A S V D T E N V I D I
                          A F F V . D   E I V . S L . K T L E   Q S V E T E K V I D I

                                      100                 110                 120
TZap-7-Protein            I Y Q P Q A I F R V R A V T R C T S S L E G H S E A V I S V
Xeno-notchless            I Y Q P Q A V F K V R A V T R C T S S L E G H T E A V I S V
Droso-notchless-Protein   V Y Q P Q A V F K V R P V T R C T S S M P G H A E A V V S L
                          I Y Q P Q A V F K V R A V T R C T S S L E G H . E A V I S V
```

Fig. 4

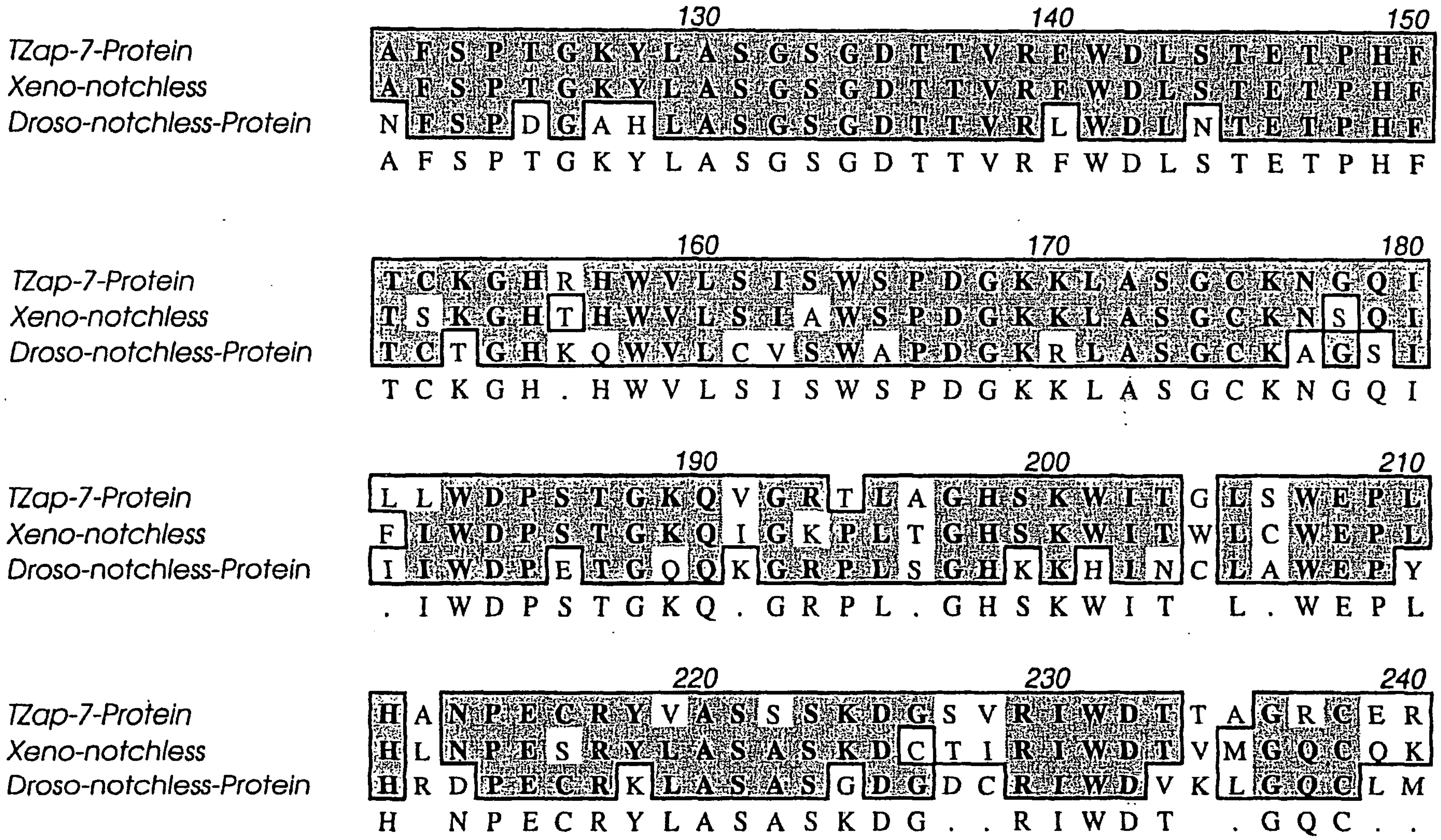
130
140
150
TZap-7-Protein A F S P T G K Y L A S G S G D T T V R F W D L S T E T P H F
Xeno-notchless A F S P T G K Y L A S G S G D T T V R F W D L S T E T P H F
Droso-notchless-Protein N F S P D G A H L A S G S G D T T V R L W D L N T E T P H F
A F S P T G K Y L A S G S G D T T V R F W D L S T E T P H F
160
170
180
TZap-7-Protein T C K G H R H W V L S I S W S P D G K K L A S G C K N G Q I
Xeno-notchless T S K G H T H W V L S I A W S P D G K K L A S G C K N S Q I
Droso-notchless-Protein T C T G H K Q W V L C V S W A P D G K R L A S G C K A G S I
T C K G H . H W V L S I S W S P D G K K L A S G C K N G Q I
190
200
210
TZap-7-Protein L L W D P S T G K Q V G R T L A G H S K W I T G L S W E P L
Xeno-notchless F I W D P S T G K Q I G K P L T G H S K W I T W L C W E P L
Droso-notchless-Protein I I W D P E T G Q Q K G R P L S G H K K H I N C L A W E P Y
. I W D P S T G K Q . G R P L . G H S K W I T L . W E P L
220
230
240
TZap-7-Protein H A N P E C R Y V A S S S K D G S V R I W D T T A G R C E R
Xeno-notchless H L N P E S R Y L A S A S K D C T I R I W D T V M G Q C Q K
Droso-notchless-Protein H R D P E C R K L A S A S G D G D C R I W D V K L G Q C L M
H N P E C R Y L A S A S K D G . . R I W D T . G Q C . .

Fig. 4 cont.

```
                               250                 260                 270
TZap-7-Protein           I L T G H T Q S V T C L R W G G D G L L Y S A S Q D R T I K
Xeno-notchless           I L T S H T Q S V T A V K W G G D G L L Y S S S Q D R T I K
Droso-notchless-Protein  N I A G H T N A V T A V R W G G A G L I Y T S S K D R T V K
                         I L T G H T Q S V T A V R W G G D G L L Y S S S Q D R T I K

                               280                 290                 300
TZap-7-Protein           V W R A H D G V L C R T L Q G H G H W V N T M A L S T D Y A
Xeno-notchless           A W R A Q D G V L C R T L Q G H A H W V N T M A L S T D Y V
Droso-notchless-Protein  M W R A A D G I L C R T F S G Q A H W V N N I A L S T D Y V
                         . W R A . D G V L C R T L Q G H A H W V N T M A L S T D Y V

                               310                 320                 330
TZap-7-Protein           L R T G A F E P A E A S V N P Q D L Q G S L Q E L K E R A L
Xeno-notchless           L R T G A F N P A D A S V N P Q D M S G S L E V L K E K A L
Droso-notchless-Protein  L R T G P F H P V K D R S K - S H L S L S T E E L Q E S A L
                         L R T G A F . P A . A S V N P Q D L S G S L E E L K E . A L

                               340                 350                 360
TZap-7-Protein           S R Y N L V R G Q G P E R L V S G S D D F T L F L W S P A E
Xeno-notchless           K R Y N E V R G Q G P E R L V S G S D D E T L F L W A P A E
Droso-notchless-Protein  K R Y Q A V C P D E V E S L V S C S D D N T L Y L W R N N Q
                         K R Y N   V R G Q G P E R L V S G S D D F T L F L W . P A E
```

Fig. 4 cont.

```
                                          370                   380                   390
TZap-7-Protein             D K K P L T R M T G H Q A L I N Q V X F S P D S R I V A S A
Xeno-notchless             E K K P L Q R M T G H Q A L I N E V L F S P D T R I I A S A
Droso-notchless-Protein    N K - C V E R M T G H Q N V V N D V K Y S P D V K L I A S A
                           . K K P L . R M T G H Q A L I N . V   F S P D . R I I A S A

                                          400                   410                   420
TZap-7-Protein             S F D K S I K L W D G R I G K Y L A S L R G H V A A V Y Q I
Xeno-notchless             S F D K S I K L W D G K T G K F L T S L R G H V S A V Y Q I
Droso-notchless-Protein    S F D K S V R L W R A S D G Q Y M A T F R G H V Q A V Y T V
                           S F D K S I K L W D G . T G K Y L A S L R G H V . A V Y Q I

                                          430                   440                   450
TZap-7-Protein             A W S A D S R L L V S G S S D S T L K V W D V K A Q K L A M
Xeno-notchless             A W S A D S R L L V S G S S D S T L K V W D S K T K K L L I
Droso-notchless-Protein    A W S A D S R L I V S G S K D S T L K V W S V Q T K K L A Q
                           A W S A D S R L L V S G S S D S T L K V W D V K T K K L A .

                                          460                   470                   480
TZap-7-Protein             D L P G H A D E V Y A V D W S P D G Q R V A S G G K D K C L
Xeno-notchless             D L P G H A D E V Y S V D W S P D G Q R V A S G G K D K C L
Droso-notchless-Protein    E L P G H A D E V F G V D W A P D G S R V A S G G K D K V I
                           D L P G H A D E V Y . V D W S P D G Q R V A S G G K D K C L
```

Fig. 4 cont.

| | | 490 | 500 | 510 |
|---|---|---|---|---|
| *TZap-7-Protein* | R I W R R | | | |
| *Xeno-notchless* | R I W R K | | | |
| *Droso-notchless-Protein* | K L W A Y | | | |
| | R I W R . | | | |

Fig. 4 cont.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 0291533 A1
- EP 0321201 A1
- EP 0360257 A2
- WO 8909622 A
- EP 0239400 A1
- WO 9007861 A
- WO 9110741 A
- WO 9402602 A
- WO 9634096 A
- WO 9633735 A
- WO 9306852 A
- WO 9111194 A
- EP 0403506 A
- WO 9306866 A
- WO 9911782 A


### Non-patent literature cited in the description


- **PUI JC ; ALLMAN D ; XU L ; DEROCCO S ; KARNELL FG ; BAKKOUR S ; LEE JY ; KADESCH T ; HARDY RR ; ASTER JC.** Notch1 expression in early lymphopoiesis influences B versus T lineage determination. *Immunity,* 1999, vol. 11, 299-308
- **NEER EJ ; SCHMIDT CJ ; NAMBUDRIPAD R ; SMITH TF.** The ancient regulatory-protein family of WD-repeat proteins. *Nature,* 1994, vol. 371, 297-300
- **NEER EJ ; SMITH TF.** G protein heterodimers: new structures propel new questions. *Cell,* 1996, vol. 84, 175-178
- **SMITH TF ; GAITATZES CG ; SAXENA K ; NEER EJ.** The WD repeat: a common architecture for diverse functions. *TIBS,* 1999, vol. 24, 181-185
- **SAMBROOK et al.** Molecular cloning; A Laboratory Manual. Cold Spring Harbor Laboratory Press, 1989
- **GOSSEN ; BUJARD.** *Proc. Natl. Acad. Sci. USA,* 1992, vol. 89, 5547-5551
- **GOSSEN et al.** *Trends Biotech.,* 1994, vol. 12, 58-62
- **STEINECKE ; RIBOZYMES et al.** Methods in Cell Biology. Academic Press, Inc, 1995, vol. 50, 449-460
- **MELANI.** *Cancer Res.,* 1991, vol. 51, 2897-2901
- **MOUELLIC.** *Proc. Natl. Acad. Sci. USA,* 1990, vol. 87, 4712-4716
- **JOYNER.** Gene Targeting, A Practical Approach. Oxford University Press
- **SAMBROOK.** Molecular Cloning A Laboratory Manual. Cold Spring Harbor Laboratory, 1989
- **AUSUBEL.** Current Protocols in Molecular Biology. Green Publishing Associates and Wiley Interscience, 1989
- **SAMBROOK.** Molecular Cloning: A Laboratory Manual. Cold Spring Harbor Laboratory, 1989
- **SCOPES.** Protein Purification. Springer-Verlag, 1982
- **KÖHLER ; MILSTEIN.** *Nature,* 1975, vol. 256, 495
- **GALFRÉ.** *Meth. Enzymol.,* 1981, vol. 73, 3
- **HARLOW ; LANE.** Antibodies, A Laboratory Manual. CSH Press, 1988
- **SCHIER.** *Human Antibodies Hybridomas,* 1996, vol. 7, 97-105
- **MALMBORG.** *J. Immunol. Methods,* 1995, vol. 183, 7-13
- **BEILSTEIN.** Handbook of Organic Chemistry. Springer edition New York Inc,
- Organic Synthesis. Wiley
- **FASSINA.** *Immunomethods,* 1994, vol. 5, 114-120
- **BERRY.** *Biochem. Soc. Trans.,* 1994, vol. 22, 1033-1036
- **WODAK.** *Ann. N. Y. Acad. Sci.,* 1987, vol. 501, 1-13
- **PABO.** *Biochemistry,* 1986, vol. 25, 5987-5991
- **OSTRESH.** *Methods in Enzymology,* 1996, vol. 267, 220-234
- **DORNER.** *Bioorg. Med. Chem.,* 1996, vol. 4, 709-715
- **ROSE.** *Biochemistry,* 1996, vol. 35, 12933-12944
- **RUTENBER.** *Bioorg. Med. Chem.,* 1996, vol. 4, 1545-1558
- **SCOFIELD.** *Science,* 1996, vol. 274, 2063-2065

- **BERKS.** *TIBTECH,* 1994, vol. 12, 352-364
- **ARTAVANIS-TSAKONAS, S. ; MATSUNO, K. ; FORTINI, M.E.** Notch Signalling. *Science,* 1995, vol. 268, 225-232
- **GREENWALD. I. ; RUBIN, G.M.** Making a difference: the role of cell-cell interactions in establishing separate identities for equivalent cells. *Cell,* 1992, vol. 68, 271-281
- **ELLISEN, L.W. ; BIRD, J. ; WEST, D.C. ; SORENG, A.L. ; REYNOLDS, T.C. ; SMITH, S.D. ; SKLAR, J.** Tan-1, the human homolog of the Drosophila notch gene, is broken by chromosomal translocations in T lymphoblastic neoplasms. *Cell,* 1991, vol. 66, 649-661
- **SWIATEK, P.J. ; LINDSELL, C.E. ; DEL AMO, F.F. ; WEINMASTER, G. ; GRIDLEY, T.** Notch1 is essential for postimplantation development in mice. *Genes & Development,* 1994, vol. 6, 707-719
- **ROBEY, E. ; CHANG, D. ; ITANO, A. ; CADO, D. ; ALEXANDER, H. ; LANS, D. ; WEINMASTER, G. ; SALMON, P.** An activated form of notch influences the choice between CD4 and CD8 T cell lineage. *Cell,* 1996, vol. 87, 483-4.92
- **WHARTON KA ; JOHANSEN KM ; XU T ; ARTAVANIS-TSAKONAS S.** Nucleotide sequence from the neurogenic locus notch implies a gene product that shares homology with proteins containing EGF-like repeats. *Cell,* vol. 43, 567-581
- **PEAR, W.S. ; ASTER, J.C. ; HASSERJIAN, R. ; B., SOFFER ; B., SKLAR ; BALTIMORE, D.** Exclusive development of T cell neoplasms in mice transplanted with bone marrow expressing activated Notch alleles. *Journal of Experimental Medicine,* 1996, vol. 183, 2283-2291
- **ARTAVANIS-TSAKONAS, S. ; RAND, M.D. ; LAKE, R.J.** Notch signaling: cell fate control and signal integration in development. *Science,* 1999, vol. 284, 770-776
- **BAILEY, A.M. ; POSAKONY, J.W.** Suppressor of hairless directly activates transcription of Enhancer of split complex genes in response to notch receptor activity. *Genes & Development,* 1995, vol. 9, 2609-2622
- **JEHN BM ; BIELKE W ; PEAR WS ; OSBORNE BA.** Protective effects of notch-1 on TCR-induced apoptosis. *J Immunol,* 1999, vol. 162, 635-638
- **ROYET, J. ; BOUWMEESTER, T. ; COHEN, S.M.** Notchless encodes a novel WD40-repeat-containing protein that modulates Notch signaling activity. *The EMBO Journal,* 1998, vol. 17, 7351-7360
- **MATSUNO K ; EASTMAN D ; MITSIADES T ; QUINN AM ; CARCANCIU ML ; ORDENTLICH P ; KADESCH T ; ARTAVANIS-TSAKONAS S.** Human deltex is a conserved regulator of Notch signalling. *Nature Genetics,* 1995, vol. 19, 74-78
- **G.R. CRABTREE.** Contingent genetic regulatory events in T lymphocyte activation. *Science,* 1989, vol. 243, 355-361
- **C.H. JUNE.** Signal transduction in T cells. *Curr. Opin. Immunol.,* 1991, vol. 3, 287-293
- **R. H. SCHWARTZ.** Costimulation of T lymphocytes: the role of CD28, CTLA-4, and B7/BB1 in Interleukin-2 production and immunotherapy. *Cell,* 1992, vol. 71, 1065-1068
- **J. BANCHEREAU ; F. BAZAN ; D. BLANCHARD ; F. BRIERE ; J. GALIZZI ; C. VAN KOOTEN ; Y. LIU ; F. ROUSSET ; S. SEELAND.** The CD40 antigen and its ligand. *Annu. Rev. Immunol.,* 1994, vol. 12, 881-922
- **D.J. LENSCHOW ; T. WALUNAS ; J. BLUESTONE.** CD28/B7 system of T cell costimulation. *Annu. Rev. Immunol.,* 1996, vol. 14, 233-258
- **P. LINSLEY ; J. LEDBETTER.** The role of the CD28 receptor during T cell responses to antigen. *Annu. Rev. Immunol.,* 1993, vol. 11, 191-212
- **A. KUPFER ; S.L. SWAIN ; S.J. SINGER.** The specific direct interaction of helper T cells and antigen-presenting B cells. II. Reorientation of the microtubule organizing center and reorganization of the membrane-associated cytoskeleton inside the bound helper T cells. *J. Exp. Med.,* 1987, vol. 165, 1565-1580
- **M.V. PARSEY ; G.K. LEWIS.** Actin polymerization and pseudopod reorganization accompany anti-CD3-induced growth arrest in Jurkat T cells. *J. Immunol.,* 1993, vol. 151, 1881-1893
- **N. SELLIAH ; W.H. BROOKS ; T.L. ROSZMAN.** Proteolytic cleavage of -actinin by calpain in T cells stimulated with anti-CD3 monoclonal antibody. *J. Immunol.,* 1996, vol. 156, 3215-3221
- **R. KOJIMA ; J. RANDALL ; B.M. BRENNER ; S.R. GULLANS.** Osmotic stress protein 94 (Osp94): A new member of the Hsp110/SSE gene subfamily. *J. Biol. Chem.,* 1996, vol. 271, 12327-12332
- **J. SAMBROOK ; E.F. FRITSCH ; T. MANIATIS.** Molecular Cloning: A Laboratory Manual. Cold Spring Harbour, 1989
- **A.P. FEINBERG ; B. VOGELSTEIN.** A technique for radiolabeling DNA restriction endonuclease fragments to high specific activity. *Anal. Biochem.,* 1983, vol. 132, 6-13
- **F. SANGER ; S. NICKLEN ; A.R. COULSON.** DNA sequencing with chain-terminating inhibitors. *Proc. Natl. Acad. Sci. USA,* 1977, vol. 74, 5463-5467
- **M. ASHBURNER ; P. THOMPSON ; J. ROOTE ; P.F. LASKO ; Y. GRAU ; M. EL MESSAL ; S. ROTH ; P. SIMPSON.** The genetics of a small autosomal region of Drosophila melanogaster containing the structural gene for alcohol dehydrogenase. Characterization of the region around the snail and cactus loci. *Genetics,* 1990, vol. 126, 679-694

- **S. ROTH ; F.S. NEUMAN-SILBERBERG ; G. BARCELO ; T. SCHÜPBACH.** Cornichon and the EGF receptor signaling process are necessary for both anterior-posterior and dorsal-ventral pattern formation in Drosophila. *Cell,* 1995, vol. 81, 967-978
- **R. LEHMANN.** Establishment of embryonic Drosophila oogenesis. *Dev. Biol.,* 1995, vol. 6, 25-38
- **F.S. NEUMAN-SILBERBERG ; T. SCHÜPBACH.** The Drosophila dorsoventral patterning gene gurken produces a dorsally localized RNA and encodes a TGF-like protein. *Cell,* 1993, vol. 75, 165-174
- **J.V. PRICE ; R.J. CLIFFORD ; T. SCHÜPBACH.** The maternal ventralizing locus torpedo is allelic to faint little ball, an embryogenic lethal, and encodes the Drosophila EGF receptor homolog. *Cell,* 1998, vol. 56, 1085-1092
- **J. SCHLESSINGER ; B. GEIGER.** Epidermal growth factor induces redistribution of actin and alpha-actinin in human epidermal carcinoma cells. *Exp. Cell. Res.,* 1981, vol. 134, 273-279
- **J. SINGER.** Intercellular communication and cell-cell adhesion. *Science,* 1992, vol. 255, 1671-1677
- **R. PARDI ; L. INVERARDI ; C. RUGARLI ; J.R. BENDER.** Antigen-receptor complex stimulation triggers protein kinase C-dependent CD11a/CD18-cytoskeleton association in T lymphocytes. *J. Cell. Biol.,* 1992, vol. 116, 1211-1220
- **PEARSON.** *Methods in Enzymology,* 1990, vol. 183, 63-98